# EUROPEAN PATENT SPECIFICATION

(11) **EP 2 859 019 B1**
(45) Date of publication and mention of the grant of the patent: **21.02.2018**
(21) Application number: 13742750.6
(22) Date of filing: 07.06.2013
(51) Int. Cl.: A61K 39/00, A61K 45/06, C07K 16/28

(54) **HUMANIZED ANTI-TRKA ANTIBODIES WITH AMINO ACID SUBSTITUTIONS**
HUMANISIERTEN ANTI-TRKA ANTIKÖRPER MIT AMINOSÄURE-SUBSTITUTIONEN
ANTICORPS HUMANISÉS ANTI-TRKA AVEC SUBSTITUTIONS D'ACIDES AMINÉS

(30) Priority: 08.06.2012 US 201261657184 P
(43) Date of publication of application: 15.04.2015
(73) Proprietor: Glenmark Pharmaceuticals S.A., 2300 La Chaux-de-Fonds (CH)
(72) Inventor: BLEIN, Stanislas, CH-2300 La Chaux-de-Fonds (CH); OLLIER, Romain, CH-2300 La Chaux-de-Fonds (CH); SKEGRO, Darko, CH-2300 La Chaux-de-Fonds (CH)
(74) Representative: Thomas, Dean
(86) International application number: PCT/IB2013/054688
(87) International publication number: WO 2013/183032

(56) References cited:
- WO-A1-2009/098238

### SEQUENCE LISTING

<110> Glenmark Pharmaceuticals S.A.
<120> ANTI-TRKA ANTIBODIES WITH ENHANCED INHIBITORY PROPERTIES AND DERIVATIVES THEREOF
<130> PCT
<150> US 61/657,184
   <151> 2012-06-08
<160> 116
<170> PatentIn version 3.3
<210> 1
   <211> 123
   <212> PRT
   <213> Artificial
<220>
   <223> VH1 domain
<400> 1
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 2
   <211> 123
   <212> PRT
   <213> Artificial
<220>
   <223> VH2 domain
<400> 2
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Gly Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 3
   <211> 123
   <212> PRT
   <213> Artificial
<220>
   <223> VH3 domain
<400> 3
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 4
   <211> 123
   <212> PRT
   <213> Artificial
<220>
   <223> VH4 domain
<400> 4
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 5
   <211> 123
   <212> PRT
   <213> Artificial
<220>
   <223> VH5 domain
<400> 5
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 6
   <211> 106
   <212> PRT
   <213> Artificial
<220>
   <223> VL1 domain
<400> 6
   Glu Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ala Pro Arg Leu Leu Ile Tyr
           35                  40                  45
   Thr Thr Ser Asn Leu Ala Ser Gly Ile Pro Ala Arg Phe Ser Gly Ser
       50                  55                  60
   Gly Ser Gly Thr Asp Phe Thr Leu Thr Ile Ser Ser Leu Glu Pro Glu
   65                  70                  75                  80
   Asp Phe Ala Val Tyr Tyr Cys His Gln Trp Ser Ser Tyr Pro Trp Thr
                   85                  90                  95
   Phe Gly Gln Gly Thr Lys Leu Glu Ile Lys
               100                 105
<210> 7
   <211> 106
   <212> PRT
   <213> Artificial
<220>
   <223> VL2 domain
<400> 7
   Glu Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ser Pro Arg Leu Leu Ile Tyr
           35                  40                  45
   Thr Thr Ser Asn Leu Ala Ser Gly Ile Pro Ser Arg Phe Ser Gly Ser
       50                  55                  60
   Gly Ser Gly Thr Asp Phe Thr Leu Thr Ile Ser Ser Leu Glu Pro Glu
   65                  70                  75                  80
   Asp Ala Ala Asp Tyr Tyr Cys His Gln Trp Ser Ser Tyr Pro Trp Thr
                   85                  90                  95
   Phe Gly Gln Gly Thr Lys Leu Glu Ile Lys
               100                 105
<210> 8
   <211> 106
   <212> PRT
   <213> Artificial
<220>
   <223> VL3 domain
<400> 8
   Gln Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
   20 25 30
   His Trp Tyr Gln Gln Lys Pro Gly Gln Ser Pro Arg Leu Leu Ile Tyr
   35 40 45
   Thr Thr Ser Asn Leu Ala Ser Gly Ile Pro Ser Arg Phe Ser Gly Ser
   50 55 60
   Gly Ser Gly Thr Phe Tyr Thr Leu Thr Ile Ser Ser Leu Glu Pro Glu
   65 70 75 80
   Asp Phe Ala Val Tyr Tyr Cys His Gln Trp Ser Ser Tyr Pro Trp Thr
   85 90 95
   Phe Gly Gly Gly Thr Lys Leu Glu Ile Lys
   100 105
<210> 9
   <211> 106
   <212> PRT
   <213> Artificial
<220>
   <223> VL4 domain
<400> 9
   Gln Ile Val Leu Thr Gln Ser Pro Ala Thr Leu Ser Leu Ser Pro Gly
   1 5 10 15
   Glu Arg Ala Thr Leu Ser Cys Ser Ala Ser Ser Ser Val Ser Tyr Met
   20 25 30
   His Trp Tyr Gln Gln Lys Pro Gly Gln Ser Pro Arg Leu Leu Ile Tyr
   35 40 45
   Thr Thr Ser Asn Leu Ala Ser Gly Ile Pro Ser Arg Phe Ser Gly Ser
   50 55 60
   Gly Ser Gly Thr Asp Phe Thr Leu Thr Ile Ser Ser Leu Glu Pro Glu
   65 70 75 80
   Asp Ala Ala Asp Tyr Tyr Cys His Gln Trp Ser Ser Tyr Pro Trp Thr
   85 90 95
   Phe Gly Gly Gly Thr Lys Leu Glu Ile Lys
   100 105
<210> 10
   <211> 106
   <212> PRT
   <213> Artificial
<220>
   <223> VL5 domain
<400> 10
   Gln Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ser Pro Arg Leu Leu Ile Tyr
           35                  40                  45
   Thr Thr Ser Asn Leu Ala Ser Gly Ile Pro Ser Arg Phe Ser Gly Ser
       50                  55                  60
   Gly Ser Gly Thr Asp Tyr Thr Leu Thr Ile Ser Ser Leu Glu Pro Glu
   65                  70                  75                  80
   Asp Ala Ala Asp Tyr Tyr Cys His Gln Trp Ser Ser Tyr Pro Trp Thr
                   85                  90                  95
   Phe Gly Gln Gly Thr Lys Leu Glu Ile Lys
               100                 105
<210> 11
   <211> 106
   <212> PRT
   <213> Artificial
<220>
   <223> VL6 domain
<400> 11
   Glu Ile Val Leu Thr Gln Ser Pro Ala Thr Leu Ser Leu Ser Pro Gly
   1               5                   10                  15
   Glu Glu Ala Thr Leu Ser Cys Ser Ala Ser Ser Ser Val Ser Tyr Met
               20                  25                  30
   His Trp Tyr Gln Gln Lys Pro Gly Gln Ser Pro Arg Leu Leu Ile Tyr
           35                  40                  45
   Thr Thr Ser Asn Leu Ala Ser Gly Ile Pro Ser Arg Phe Ser Gly Ser
       50                  55                  60
   Gly Ser Gly Thr Phe Tyr Thr Leu Thr Ile Ser Ser Leu Glu Pro Glu
   65                  70                  75                  80
   Asp Ala Ala Asp Tyr Tyr Cys His Gln Trp Ser Ser Tyr Pro Trp Thr
                   85                  90                  95
   Phe Gly Gly Gly Thr Lys Leu Glu Ile Lys
               100                 105
<210> 12
   <211> 106
   <212> PRT
   <213> Artificial
<220>
   <223> VL7 domain
<400> 12
   Gln Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ser Pro Arg Leu Leu Ile Tyr
           35                  40                  45
   Thr Thr Ser Asn Leu Ala Ser Gly Ile Pro Ser Arg Phe Ser Gly Ser
       50                  55                  60
   Gly Ser Gly Thr Phe Tyr Thr Leu Thr Ile Ser Ser Leu Glu Pro Glu
   65                  70                  75                  80
   Asp Ala Ala Asp Tyr Tyr Cys His Gln Trp Ser Ser Tyr Pro Trp Thr
                   85                  90                  95
   Phe Gly Gly Gly Thr Lys Leu Glu Ile Lys
               100                 105
<210> 13
   <211> 106
   <212> PRT
   <213> Artificial
<220>
   <223> VL8 domain
<400> 13
   Gln Ile Val Leu Thr Gln Ser Pro Ala Thr Leu Ser Leu Ser Pro Gly
   1               5                   10                  15
   Glu Glu Ala Thr Leu Ser Cys Ser Ala Ser Ser Ser Val Ser Tyr Met
   20 25 30
   His Trp Tyr Gln Gln Lys Ser Gly Thr Ser Pro Arg Leu Leu Ile Tyr
   35 40 45
   Thr Thr Ser Asn Leu Ala Ser Gly Ile Pro Ser Arg Phe Ser Gly Ser
   50 55 60
   Gly Ser Gly Thr Phe Tyr Thr Leu Thr Ile Ser Ser Leu Glu Pro Glu
   65 70 75 80
   Asp Ala Ala Asp Tyr Tyr Cys His Gln Trp Ser Ser Tyr Pro Trp Thr
   85 90 95
   Phe Gly Gly Gly Thr Lys Leu Glu Ile Lys
   100 105
<210> 14
   <211> 10
   <212> PRT
   <213> Artificial
<220>
   <223> CDR1 of the heavy chain variable domain
<400> 14
   Gly Phe Thr Phe Ser Thr Tyr Thr Met Ser
   1 5 10
<210> 15
   <211> 17
   <212> PRT
   <213> Artificial
<220>
   <223> CDR2 of the heavy chain variable domain
<400> 15
   Tyr Ile Ser Lys Gly Gly Gly Ser Thr Tyr Tyr Pro Asp Thr Val Lys
   1 5 10 15
   Gly
<210> 16
   <211> 14
   <212> PRT
   <213> Artificial
<220>
   <223> CDR3 of the heavy chain variable domain
<400> 16
   Gly Ala Met Tyr Gly Asn Asp Phe Phe Tyr Pro Met Asp Tyr
   1               5                   10
<210> 17
   <211> 10
   <212> PRT
   <213> Artificial
<220>
   <223> CDR1 of the light chain variable domain
<400> 17
   Ser Ala Ser Ser Ser Val Ser Tyr Met His
   1               5                   10
<210> 18
   <211> 7
   <212> PRT
   <213> Artificial
<220>
   <223> CDR2 of the light chain variable domain
<400> 18
   Thr Thr Ser Asn Leu Ala Ser
   1               5
<210> 19
   <211> 10
   <212> PRT
   <213> Artificial
<220>
   <223> CDR3 of the light chain variable domain
<400> 19
   His Gln Trp Ser Ser Tyr Pro Trp Thr Phe
   1               5                   10
<210> 20
   <211> 123
   <212> PRT
   <213> Artificial
<220>
   <223> MNAC13 VH domain
<400> 20
   Glu Val Lys Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Ser Thr Tyr
   20 25 30
   Thr Met Ser Trp Ala Arg Gln Thr Pro Glu Lys Arg Leu Glu Trp Val
   35 40 45
   Ala Tyr Ile Ser Lys Gly Gly Gly Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Ser Ser Leu Lys Ser Glu Asp Thr Ala Leu Tyr Tyr Cys
   85 90 95
   Ala Arg Gly Ala Met Tyr Gly Asn Asp Phe Phe Tyr Pro Met Asp Tyr
   100 105 110
   Trp Gly Gln Gly Thr Ser Val Thr Val Ser Ser
   115 120
<210> 21
   <211> 446
   <212> PRT
   <213> Artificial
<220>
   <223> MNAC13 heavy chain
<400> 21
   Glu Val Lys Leu Val Glu Ser Gly Gly Gly Leu Val Gln Pro Gly Gly
   1 5 10 15
   Ser Leu Lys Leu Ser Cys Ala Ala Ser Gly Phe Thr Phe Ser Thr Tyr
   20 25 30
   Thr Met Ser Trp Ala Arg Gln Thr Pro Glu Lys Arg Leu Glu Trp Val
   35 40 45
   Ala Tyr Ile Ser Lys Gly Gly Gly Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Ser Ser Leu Lys Ser Glu Asp Thr Ala Leu Tyr Tyr Cys
   85 90 95
   Ala Arg Gly Ala Met Tyr Gly Asn Asp Phe Phe Tyr Pro Met Asp Tyr
               100                 105                 110
   Trp Gly Gln Gly Thr Ser Val Thr Val Ser Ser Lys Thr Thr Pro Pro
           115                 120                 125
   Ser Val Tyr Pro Leu Ala Pro Gly Ser Ala Ala Gln Thr Asn Ser Met
       130                 135                 140
   Val Thr Leu Gly Cys Leu Val Lys Gly Tyr Phe Pro Glu Pro Val Thr
   145                 150                 155                 160
   Val Thr Trp Asn Ser Gly Ser Leu Ser Ser Gly Val His Thr Phe Pro
                   165                 170                 175
   Ala Val Leu Glu Ser Asp Leu Tyr Thr Leu Ser Ser Ser Val Thr Val
               180                 185                 190
   Pro Ser Ser Pro Arg Pro Ser Glu Thr Val Thr Cys Asn Val Ala His
           195                 200                 205
   Pro Ala Ser Ser Thr Lys Val Asp Lys Lys Ile Val Pro Arg Asp Cys
       210                 215                 220
   Gly Cys Lys Pro Cys Ile Cys Thr Val Pro Glu Val Ser Ser Val Phe
   225                 230                 235                 240
   Ile Phe Pro Pro Lys Pro Lys Asp Val Leu Thr Ile Thr Leu Thr Pro
                   245                 250                 255
   Lys Val Thr Cys Val Val Val Asp Ile Ser Lys Asp Asp Pro Glu Val
               260                 265                 270
   Gln Phe Ser Trp Phe Val Asp Asp Val Glu Val His Thr Ala Gln Thr
           275                 280                 285
   Gln Pro Arg Glu Glu Gln Phe Asn Ser Thr Phe Arg Ser Val Ser Glu
       290                 295                 300
   Leu Pro Ile Met His Gln Asp Trp Leu Asn Gly Lys Glu Phe Lys Cys
   305                 310                 315                 320
   Arg Val Asn Ser Ala Ala Phe Pro Ala Pro Ile Glu Lys Thr Ile Ser
                   325                 330                 335
   Lys Thr Lys Gly Arg Pro Lys Ala Pro Gln Val Tyr Thr Ile Pro Pro
               340                 345                 350
   Pro Lys Glu Gln Met Ala Lys Asp Lys Val Ser Leu Thr Cys Met Ile
           355                 360                 365
   Thr Asp Phe Phe Pro Glu Asp Ile Thr Val Glu Trp Gln Trp Asn Gly
       370                 375                 380
   Gln Pro Ala Glu Asn Tyr Lys Asn Thr Gln Pro Ile Met Asn Thr Asn
   385                 390                 395                 400
   Gly Ser Tyr Phe Val Tyr Ser Lys Leu Asn Val Gln Lys Ser Asn Trp
                   405                 410                 415
   Glu Ala Gly Asn Thr Phe Thr Cys Ser Val Leu His Glu Gly Leu His
               420                 425                 430
   Asn His His Thr Glu Lys Ser Leu Ser His Ser Pro Gly Lys
           435                 440                 445
<210> 22
   <211> 212
   <212> PRT
   <213> Artificial
<220>
   <223> MNAC13 light chain
<400> 22
   Gln Ile Val Leu Thr Gln Ser Pro Ala Ile Met Ser Ala Ser Leu Gly
   1               5                   10                  15
   Glu Glu Ile Thr Leu Thr Cys Ser Ala Ser Ser Ser Val Ser Tyr Met
               20                  25                  30
   His Trp Tyr Gln Gln Lys Ser Gly Thr Ser Pro Lys Leu Leu Ile Tyr
           35                  40                  45
   Thr Thr Ser Asn Leu Ala Ser Gly Val Pro Ser Arg Phe Ser Gly Ser
       50                  55                  60
   Gly Ser Gly Thr Phe Tyr Ser Leu Thr Ile Ser Ser Val Glu Ala Glu
   65                  70                  75                  80
   Asp Ala Ala Asp Tyr Tyr Cys His Gln Trp Ser Ser Tyr Pro Trp Thr
                   85                  90                  95
   Phe Gly Gly Gly Thr Lys Leu Glu Ile Lys Ala Asp Ala Ala Pro Thr
               100                 105                 110
   Val Ser Ile Phe Pro Pro Ser Ser Glu Gln Leu Thr Ser Gly Gly Ala
           115                 120                 125
   Ser Val Val Cys Phe Leu Asn Asn Phe Tyr Pro Lys Asp Ile Asn Val
       130                 135                 140
   Lys Trp Lys Ile Asp Gly Ser Glu Arg Gln Asn Gly Val Leu Asn Ser
   145                 150                 155                 160
   Trp Thr Asp Gln Asp Ser Lys Asp Ser Thr Tyr Ser Met Ser Ser Thr
                   165                 170                 175
   Leu Thr Leu Thr Lys Asp Glu Tyr Glu Arg His Asn Ser Tyr Thr Cys
               180                 185                 190
   Glu Ala Thr His Lys Thr Ser Thr Ser Pro Ile Val Lys Ser Phe Asn
           195                 200                 205
   Arg Asn Glu Cys
       210
<210> 23
   <211> 98
   <212> PRT
   <213> Homo sapiens
<220>
   <221> VH3-23*01 domain
   <222> (1)..(98)
<400> 23
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys
<210> 24
   <211> 628
   <212> PRT
   <213> Artificial
<220>
   <223> human TrkA-Fc fusion protein
<400> 24
   Ala Ala Pro Cys Pro Asp Ala Cys Cys Pro His Gly Ser Ser Gly Leu
   1               5                   10                  15
   Arg Cys Thr Arg Asp Gly Ala Leu Asp Ser Leu His His Leu Pro Gly
               20                  25                  30
   Ala Glu Asn Leu Thr Glu Leu Tyr Ile Glu Asn Gln Gln His Leu Gln
           35                  40                  45
   His Leu Glu Leu Arg Asp Leu Arg Gly Leu Gly Glu Leu Arg Asn Leu
       50                  55                  60
   Thr Ile Val Lys Ser Gly Leu Arg Phe Val Ala Pro Asp Ala Phe His
   65                  70                  75                  80
   Phe Thr Pro Arg Leu Ser Arg Leu Asn Leu Ser Phe Asn Ala Leu Glu
                   85                  90                  95
   Ser Leu Ser Trp Lys Thr Val Gln Gly Leu Ser Leu Gln Glu Leu Val
               100                 105                 110
   Leu Ser Gly Asn Pro Leu His Cys Ser Cys Ala Leu Arg Trp Leu Gln
           115                 120                 125
   Arg Trp Glu Glu Glu Gly Leu Gly Gly Val Pro Glu Gln Lys Leu Gln
       130                 135                 140
   Cys His Gly Gln Gly Pro Leu Ala His Met Pro Asn Ala Ser Cys Gly
   145                 150                 155                 160
   Val Pro Thr Leu Lys Val Gln Val Pro Asn Ala Ser Val Asp Val Gly
                   165                 170                 175
   Asp Asp Val Leu Leu Arg Cys Gln Val Glu Gly Arg Gly Leu Glu Gln
               180                 185                 190
   Ala Gly Trp Ile Leu Thr Glu Leu Glu Gln Ser Ala Thr Val Met Lys
           195                 200                 205
   Ser Gly Gly Leu Pro Ser Leu Gly Leu Thr Leu Ala Asn Val Thr Ser
       210                 215                 220
   Asp Leu Asn Arg Lys Asn Val Thr Cys Trp Ala Glu Asn Asp Val Gly
   225                 230                 235                 240
   Arg Ala Glu Val Ser Val Gln Val Asn Val Ser Phe Pro Ala Ser Val
                   245                 250                 255
   Gln Leu His Thr Ala Val Glu Met His His Trp Cys Ile Pro Phe Ser
               260                 265                 270
   Val Asp Gly Gln Pro Ala Pro Ser Leu Arg Trp Leu Phe Asn Gly Ser
           275                 280                 285
   Val Leu Asn Glu Thr Ser Phe Ile Phe Thr Glu Phe Leu Glu Pro Ala
       290                 295                 300
   Ala Asn Glu Thr Val Arg His Gly Cys Leu Arg Leu Asn Gln Pro Thr
   305                 310                 315                 320
   His Val Asn Asn Gly Asn Tyr Thr Leu Leu Ala Ala Asn Pro Phe Gly
                   325                 330                 335
   Gln Ala Ser Ala Ser Ile Met Ala Ala Phe Met Asp Asn Pro Phe Glu
               340                 345                 350
   Phe Asn Pro Glu Asp Pro Ile Pro Val Ser Phe Ser Pro Val Asp Thr
           355                 360                 365
   Asn Ser Thr Ser Gly Asp Pro Val Glu Lys Lys Asp Glu Thr Pro Phe
       370                 375                 380
   Gly Val Ser Val Ala Val Gly Ala Ala Ala Glu Asn Leu Tyr Phe Gln
   385                 390                 395                 400
   Ser Gly Ser Thr His Thr Cys Pro Pro Cys Pro Ala Pro Glu Leu Leu
                   405                 410                 415
   Gly Gly Pro Ser Val Phe Leu Phe Pro Pro Lys Pro Lys Asp Thr Leu
               420                 425                 430
   Met Ile Ser Arg Thr Pro Glu Val Thr Cys Val Val Val Asp Val Ser
           435                 440                 445
   His Glu Asp Pro Glu Val Lys Phe Asn Trp Tyr Val Asp Gly Val Glu
   450 455 460
   Val His Asn Ala Lys Thr Lys Pro Arg Glu Glu Gln Tyr Asn Ser Thr
   465 470 475 480
   Tyr Arg Val Val Ser Val Leu Thr Val Leu His Gln Asp Trp Leu Asn
   485 490 495
   Gly Lys Glu Tyr Lys Cys Lys Val Ser Asn Lys Ala Leu Pro Ala Pro
   500 505 510
   Ile Glu Lys Thr Ile Ser Lys Ala Lys Gly Gln Pro Arg Glu Pro Gln
   515 520 525
   Val Tyr Thr Leu Pro Pro Ser Arg Asp Glu Leu Thr Lys Asn Gln Val
   530 535 540
   Ser Leu Thr Cys Leu Val Lys Gly Phe Tyr Pro Ser Asp Ile Ala Val
   545 550 555 560
   Glu Trp Glu Ser Asn Gly Gln Pro Glu Asn Asn Tyr Lys Thr Thr Pro
   565 570 575
   Pro Val Leu Asp Ser Asp Gly Ser Phe Phe Leu Tyr Ser Lys Leu Thr
   580 585 590
   Val Asp Lys Ser Arg Trp Gln Gln Gly Asn Val Phe Ser Cys Ser Val
   595 600 605
   Met His Glu Ala Leu His Asn His Tyr Thr Gln Lys Ser Leu Ser Leu
   610 615 620
   Ser Pro Gly Lys
   625
<210> 25
   <211> 391
   <212> PRT
   <213> Homo sapiens
<220>
   <221> human TrkA extracellular region
   <222> (1)..(391)
<400> 25
   Ala Ala Pro Cys Pro Asp Ala Cys Cys Pro His Gly Ser Ser Gly Leu
   1               5                   10                  15
   Arg Cys Thr Arg Asp Gly Ala Leu Asp Ser Leu His His Leu Pro Gly
               20                  25                  30
   Ala Glu Asn Leu Thr Glu Leu Tyr Ile Glu Asn Gln Gln His Leu Gln
           35                  40                  45
   His Leu Glu Leu Arg Asp Leu Arg Gly Leu Gly Glu Leu Arg Asn Leu
       50                  55                  60
   Thr Ile Val Lys Ser Gly Leu Arg Phe Val Ala Pro Asp Ala Phe His
   65                  70                  75                  80
   Phe Thr Pro Arg Leu Ser Arg Leu Asn Leu Ser Phe Asn Ala Leu Glu
                   85                  90                  95
   Ser Leu Ser Trp Lys Thr Val Gln Gly Leu Ser Leu Gln Glu Leu Val
               100                 105                 110
   Leu Ser Gly Asn Pro Leu His Cys Ser Cys Ala Leu Arg Trp Leu Gln
           115                 120                 125
   Arg Trp Glu Glu Glu Gly Leu Gly Gly Val Pro Glu Gln Lys Leu Gln
       130                 135                 140
   Cys His Gly Gln Gly Pro Leu Ala His Met Pro Asn Ala Ser Cys Gly
   145                 150                 155                 160
   Val Pro Thr Leu Lys Val Gln Val Pro Asn Ala Ser Val Asp Val Gly
                   165                 170                 175
   Asp Asp Val Leu Leu Arg Cys Gln Val Glu Gly Arg Gly Leu Glu Gln
               180                 185                 190
   Ala Gly Trp Ile Leu Thr Glu Leu Glu Gln Ser Ala Thr Val Met Lys
           195                 200                 205
   Ser Gly Gly Leu Pro Ser Leu Gly Leu Thr Leu Ala Asn Val Thr Ser
       210                 215                 220
   Asp Leu Asn Arg Lys Asn Val Thr Cys Trp Ala Glu Asn Asp Val Gly
   225                 230                 235                 240
   Arg Ala Glu Val Ser Val Gln Val Asn Val Ser Phe Pro Ala Ser Val
                   245                 250                 255
   Gln Leu His Thr Ala Val Glu Met His His Trp Cys Ile Pro Phe Ser
   260 265 270
   Val Asp Gly Gln Pro Ala Pro Ser Leu Arg Trp Leu Phe Asn Gly Ser
   275 280 285
   Val Leu Asn Glu Thr Ser Phe Ile Phe Thr Glu Phe Leu Glu Pro Ala
   290 295 300
   Ala Asn Glu Thr Val Arg His Gly Cys Leu Arg Leu Asn Gln Pro Thr
   305 310 315 320
   His Val Asn Asn Gly Asn Tyr Thr Leu Leu Ala Ala Asn Pro Phe Gly
   325 330 335
   Gln Ala Ser Ala Ser Ile Met Ala Ala Phe Met Asp Asn Pro Phe Glu
   340 345 350
   Phe Asn Pro Glu Asp Pro Ile Pro Val Ser Phe Ser Pro Val Asp Thr
   355 360 365
   Asn Ser Thr Ser Gly Asp Pro Val Glu Lys Lys Asp Glu Thr Pro Phe
   370 375 380
   Gly Val Ser Val Ala Val Gly
   385 390
<210> 26
   <211> 453
   <212> PRT
   <213> Artificial
<220>
   <223> BXhVH1 heavy chain
<400> 26
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 27
   <211> 453
   <212> PRT
   <213> Artificial
<220>
   <223> BXhVH3 heavy chain
<400> 27
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450
<210> 28
   <211> 453
   <212> PRT
   <213> Artificial
<220>
   <223> BXhVH5 heavy chain
<400> 28
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 29
   <211> 213
   <212> PRT
   <213> Artificial
<220>
   <223> BXhVL1 light chain
<400> 29
   Glu Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ala Pro Arg Leu Leu Ile Tyr
           35                  40                  45
   Thr Thr Ser Asn Leu Ala Ser Gly Ile Pro Ala Arg Phe Ser Gly Ser
       50                  55                  60
   Gly Ser Gly Thr Asp Phe Thr Leu Thr Ile Ser Ser Leu Glu Pro Glu
   65                  70                  75                  80
   Asp Phe Ala Val Tyr Tyr Cys His Gln Trp Ser Ser Tyr Pro Trp Thr
                   85                  90                  95
   Phe Gly Gln Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 30
   <211> 213
   <212> PRT
   <213> Artificial
<220>
   <223> BXhVL3 light chain
<400> 30
   Gln Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ser Pro Arg Leu Leu Ile Tyr
           35                  40                  45
   Thr Thr Ser Asn Leu Ala Ser Gly Ile Pro Ser Arg Phe Ser Gly Ser
       50                  55                  60
   Gly Ser Gly Thr Phe Tyr Thr Leu Thr Ile Ser Ser Leu Glu Pro Glu
   65                  70                  75                  80
   Asp Phe Ala Val Tyr Tyr Cys His Gln Trp Ser Ser Tyr Pro Trp Thr
                   85                  90                  95
   Phe Gly Gly Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 31
   <211> 123
   <212> PRT
   <213> Artificial
<220>
   <223> GBR VH5(K3Q) heavy chain variable domain
<400> 31
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 32
   <211> 123
   <212> PRT
   <213> Artificial
<220>
   <223> GBR VH5(V37A) heavy chain variable domain
<400> 32
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 33
   <211> 123
   <212> PRT
   <213> Artificial
<220>
   <223> GBR VH5(G42E) heavy chain variable domain
<400> 33
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Glu Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 34
   <211> 123
   <212> PRT
   <213> Artificial
<220>
   <223> GBR VH5(V89L) heavy chain variable domain
<400> 34
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Leu Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 35
   <211> 123
   <212> PRT
   <213> Artificial
<220>
   <223> GBR VH5(R94K) heavy chain variable domain
<400> 35
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 36
   <211> 123
   <212> PRT
   <213> Artificial
<220>
   <223> GBR VH5(K3Q,V37A) heavy chain variable domain
<400> 36
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 37
   <211> 123
   <212> PRT
   <213> Artificial
<220>
   <223> GBR VH5(K3Q,T40A) heavy chain variable domain
<400> 37
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 38
   <211> 123
   <212> PRT
   <213> Artificial
<220>
   <223> GBR VH5(P60A,T62S) heavy chain variable domain
<400> 38
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                      80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 39
   <211> 123
   <212> PRT
   <213> Artificial
<220>
   <223> GBR VH5(K3Q,V37A,R44G) heavy chain variable domain
<400> 39
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Thr Met Ser Trp Ala Arg Gln Thr Pro Gly Lys Gly Leu Glu Trp Val
   35 40 45
   Ala Tyr Ile Ser Lys Gly Gly Gly Ser Thr Tyr Tyr Pro Asp Thr Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ala Met Tyr Gly Asn Asp Phe Phe Tyr Pro Met Asp Tyr
   100 105 110
   Trp Gly Gln Gly Thr Thr Val Thr Val Ser Ser
   115 120
<210> 40
   <211> 123
   <212> PRT
   <213> Artificial
<220>
   <223> GBR VH5(K3Q,A49S,Y50A) heavy chain variable domain
<400> 40
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Thr Met Ser Trp Val Arg Gln Thr Pro Gly Lys Arg Leu Glu Trp Val
   35 40 45
   Ser Ala Ile Ser Lys Gly Gly Gly Ser Thr Tyr Tyr Pro Asp Thr Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ala Met Tyr Gly Asn Asp Phe Phe Tyr Pro Met Asp Tyr
   100 105 110
   Trp Gly Gln Gly Thr Thr Val Thr Val Ser Ser
   115 120
<210> 41
   <211> 123
   <212> PRT
   <213> Artificial
<220>
   <223> GBR VH5(K3Q,P60A,T62S) heavy chain variable domain
<400> 41
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Thr Met Ser Trp Val Arg Gln Thr Pro Gly Lys Arg Leu Glu Trp Val
   35 40 45
   Ala Tyr Ile Ser Lys Gly Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ala Met Tyr Gly Asn Asp Phe Phe Tyr Pro Met Asp Tyr
   100 105 110
   Trp Gly Gln Gly Thr Thr Val Thr Val Ser Ser
   115 120
<210> 42
   <211> 123
   <212> PRT
   <213> Artificial
<220>
   <223> GBR VH5(K3Q,T40A,P60A,T62S) heavy chain variable domain
<400> 42
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 43
   <211> 123
   <212> PRT
   <213> Artificial
<220>
   <223> GBR VH5(K3Q,V37A,T40A,P60A,T62S) heavy chain variable domain
<400> 43
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Ala Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 44
   <211> 123
   <212> PRT
   <213> Artificial
<220>
   <223> GBR VH5(K3Q,T40A,R44G,A49S,Y50A) heavy chain variable domain
<400> 44
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Ala Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 45
   <211> 123
   <212> PRT
   <213> Artificial
<220>
   <223> GBR VH5(K3Q,A49S,Y50A,P60A,T62S) heavy chain variable domain
<400> 45
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ser Ala Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 46
   <211> 123
   <212> PRT
   <213> Artificial
<220>
   <223> GBR VH5(K3Q,T40A,R44G,A49S,Y50A,P60A,T62S) heavy chain variable domain
<400> 46
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Ala Ile Ser Lys Gly Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ala Met Tyr Gly Asn Asp Phe Phe Tyr Pro Met Asp Tyr
   100 105 110
   Trp Gly Gln Gly Thr Thr Val Thr Val Ser Ser
   115 120
<210> 47
   <211> 123
   <212> PRT
   <213> Artificial
<220>
   <223> GBR VH5(K3Q,T40A,R44G,A49S,Y50A,P60A,T62S,R94K) heavy chain variable domain
<400> 47
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Thr Met Ser Trp Val Arg Gln Ala Pro Gly Lys Gly Leu Glu Trp Val
   35 40 45
   Ser Ala Ile Ser Lys Gly Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Ala Met Tyr Gly Asn Asp Phe Phe Tyr Pro Met Asp Tyr
   100 105 110
   Trp Gly Gln Gly Thr Thr Val Thr Val Ser Ser
   115 120
<210> 48
   <211> 123
   <212> PRT
   <213> Artificial
<220>
   <223> GBR VH1(V37A) heavy chain variable domain
<400> 48
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Ala Pro Gly Lys Gly Leu Glu Trp Val
           35                  40                  45
   Ser Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 49
   <211> 123
   <212> PRT
   <213> Artificial
<220>
   <223> GBR VH3(V37A) heavy chain variable domain
<400> 49
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser
           115                 120
<210> 50
   <211> 453
   <212> PRT
   <213> Artificial
<220>
   <223> GBR VH5(K3Q) heavy chain IGHG1
<400> 50
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 51
   <211> 453
   <212> PRT
   <213> Artificial
<220>
   <223> GBR VH5(V37A) heavy chain IGHG1
<400> 51
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 52
   <211> 450
   <212> PRT
   <213> Artificial
<220>
   <223> GBR VH5(V37A) heavy chain IGHG4 S228P
<400> 52
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Cys Ser Arg Ser Thr Ser Glu Ser
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Lys Thr Tyr Thr Cys Asn Val
   195 200 205
   Asp His Lys Pro Ser Asn Thr Lys Val Asp Lys Arg Val Glu Ser Lys
   210 215 220
   Tyr Gly Pro Pro Cys Pro Pro Cys Pro Ala Pro Glu Phe Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser Gln Glu
   260 265 270
   Asp Pro Glu Val Gln Phe Asn Trp Tyr Val Asp Gly Val Glu Val His
   275 280 285
   Asn Ala Lys Thr Lys Pro Arg Glu Glu Gln Phe Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Tyr
   340 345 350
   Thr Leu Pro Pro Ser Gln Glu Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
   Gly Lys
   450
<210> 53
   <211> 453
   <212> PRT
   <213> Artificial
<220>
   <223> GBR VH5(G42E) heavy chain IGHG1
<400> 53
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Glu Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 54
   <211> 453
   <212> PRT
   <213> Artificial
<220>
   <223> GBR VH5(V89L) heavy chain IGHG1
<400> 54
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Leu Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 55
   <211> 453
   <212> PRT
   <213> Artificial
<220>
   <223> GBR VH5(R94K) heavy chain IGHG1
<400> 55
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 56
   <211> 453
   <212> PRT
   <213> Artificial
<220>
   <223> GBR VH5(K3Q,V37A) heavy chain IGHG1
<400> 56
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 57
   <211> 450
   <212> PRT
   <213> Artificial
<220>
   <223> GBR VH5(K3Q,V37A) heavy chain IGHG4 S228P
<400> 57
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Cys Ser Arg Ser Thr Ser Glu Ser
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Lys Thr Tyr Thr Cys Asn Val
   195 200 205
   Asp His Lys Pro Ser Asn Thr Lys Val Asp Lys Arg Val Glu Ser Lys
   210 215 220
   Tyr Gly Pro Pro Cys Pro Pro Cys Pro Ala Pro Glu Phe Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser Gln Glu
   260 265 270
   Asp Pro Glu Val Gln Phe Asn Trp Tyr Val Asp Gly Val Glu Val His
   275 280 285
   Asn Ala Lys Thr Lys Pro Arg Glu Glu Gln Phe Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Gly Leu Pro Ser Ser Ile Glu
   325 330 335
   Lys Thr Ile Ser Lys Ala Lys Gly Gln Pro Arg Glu Pro Gln Val Tyr
   340 345 350
   Thr Leu Pro Pro Ser Gln Glu Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Arg Leu Thr Val Asp
   405 410 415
   Lys Ser Arg Trp Gln Glu Gly Asn Val Phe Ser Cys Ser Val Met His
   420 425 430
   Glu Ala Leu His Asn His Tyr Thr Gln Lys Ser Leu Ser Leu Ser Leu
   435 440 445
   Gly Lys
   450
<210> 58
   <211> 453
   <212> PRT
   <213> Artificial
<220>
   <223> GBR VH5(K3Q,T40A) heavy chain IGHG1
<400> 58
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 59
   <211> 453
   <212> PRT
   <213> Artificial
<220>
   <223> GBR VH5(P60A,T62S) heavy chain IGHG1
<400> 59
   Glu Val Lys Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 60
   <211> 453
   <212> PRT
   <213> Artificial
<220>
   <223> GBR VH5(K3Q,V37A,R44G) heavy chain IGHG1
<400> 60
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Gly Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                      80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 61
   <211> 453
   <212> PRT
   <213> Artificial
<220>
   <223> GBR VH5(K3Q,A49S,Y50A) heavy chain IGHG1
<400> 61
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ser Ala Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450
<210> 62
   <211> 453
   <212> PRT
   <213> Artificial
<220>
   <223> GBR VH5(K3Q,P60A,T62S) heavy chain IGHG1
<400> 62
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 63
   <211> 453
   <212> PRT
   <213> Artificial
<220>
   <223> GBR VH5(K3Q,T40A,P60A,T62S) heavy chain IGHG1
<400> 63
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 64
   <211> 453
   <212> PRT
   <213> Artificial
<220>
   <223> GBR VH5(K3Q,V37A,T40A,P60A,T62S) heavy chain IGHG1
<400> 64
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Ala Pro Gly Lys Arg Leu Glu Trp Val
           35                  40                  45
   Ala Tyr Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 65
   <211> 453
   <212> PRT
   <213> Artificial
<220>
   <223> GBR VH5(K3Q,T40A,R44G,A49S,Y50A) heavy chain IGHG1
<400> 65
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Ala Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 66
   <211> 453
   <212> PRT
   <213> Artificial
<220>
   <223> GBR VH5(K3Q,A49S,Y50A,P60A,T62S) heavy chain IGHG1
<400> 66
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Thr Pro Gly Lys Arg Leu Glu Trp Val
           35                  40                  45
   Ser Ala Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 67
   <211> 453
   <212> PRT
   <213> Artificial
<220>
   <223> GBR VH5(K3Q,T40A,R44G,A49S,Y50A,P60A,T62S) heavy chain IGHG1
<400> 67
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Ala Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                      80
   Leu Gln Met Asn Ser Leu Arg Ala Glu Asp Thr Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 68
   <211> 453
   <212> PRT
   <213> Artificial
<220>
   <223> GBR VH5(K3Q,T40A,R44G,A49S,Y50A,P60A,T62S,R94K) heavy chain IGHG1
<400> 68
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Lys Gly Leu Glu Trp Val
           35                  40                  45
   Ser Ala Ile Ser Lys Gly Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Asp Glu Leu Thr Lys Asn Gln
   355 360 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450
<210> 69
   <211> 453
   <212> PRT
   <213> Artificial
<220>
   <223> GBR VH1(V37A) heavy chain IGHG1
<400> 69
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Ala Pro Gly Lys Gly Leu Glu Trp Val
           35                  40                  45
   Ser Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                      80
   Leu Gln Met Asn Ser Leu Arg Ala Glu Asp Thr Ala Val Tyr Tyr Cys
                   85                  90                      95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 70
   <211> 453
   <212> PRT
   <213> Artificial
<220>
   <223> GBR VH3(V37A) heavy chain IGHG1
<400> 70
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Thr Pro Gly Lys Arg Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ala Met Tyr Gly Asn Asp Phe Phe Tyr Pro Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 71
   <211> 123
   <212> PRT
   <213> Artificial
<220>
   <223> VH domain of humanized MNAC13
<400> 71
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Thr Met Ser Trp Ala Arg Gln Ala Pro Gly Lys Gly Leu Glu Trp Val
           35                  40                  45
   Ala Tyr Ile Ser Lys Gly Gly Gly Ser Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Ser Ala Val Tyr Tyr Cys
                   85                  90                  95
   Ala Arg Gly Ala Met Tyr Gly Asn Asp Phe Phe Tyr Pro Met Asp Tyr
               100                 105                 110
   Trp Gly Gln Gly Thr Leu Val Thr Val Ser Ser
           115                 120
<210> 72
   <211> 796
   <212> PRT
   <213> Homo sapiens
<220>
   <221> human TrkA
   <222> (1)..(796)
<400> 72
<210> 73
   <211> 369
   <212> DNA
   <213> Artificial
<220>
   <223> GBR VH5(K3Q) heavy chain variable domain cDNA
<400> 73
   gaagtgcagc tgctggaaag cggcggaggc ctggtgcagc ctggcggaag cctgagactg 60
   agctgtgccg ccagcggctt caccttcagc acctacacca tgagctgggt ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 74
   <211> 369
   <212> DNA
   <213> Artificial
<220>
   <223> GBR VH5(V37A) heavy chain variable domain cDNA
<400> 74
   gaagtgaaac tgctggaaag cggcggaggc ctggtgcagc ctggcggaag cctgagactg 60
   agctgtgccg ccagcggctt caccttcagc acctacacca tgagctgggc ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 75
   <211> 369
   <212> DNA
   <213> Artificial
<220>
   <223> GBR VH5(G42E) heavy chain variable domain cDNA
<400> 75
   gaagtgaaac tgctggaaag cggcggaggc ctggtgcagc ctggcggaag cctgagactg 60
   agctgtgccg ccagcggctt caccttcagc acctacacca tgagctgggt ccgacagacc 120
   cccgaaaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 76
   <211> 369
   <212> DNA
   <213> Artificial
<220>
   <223> GBR VH5(V89L) heavy chain variable domain cDNA
<400> 76
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccctgt actactgtgc cagaggcgct     300
   atgtacggca acgacttctt ctaccctatg gactactggg gccagggcac caccgtgacc     360
   gtgtctagc                                                             369
<210> 77
   <211> 369
   <212> DNA
   <213> Artificial
<220>
   <223> GBR VH5(R94K) heavy chain variable domain cDNA
<400> 77
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc caagggcgct     300
   atgtacggca acgacttctt ctaccctatg gactactggg gccagggcac caccgtgacc     360
   gtgtctagc                                                             369
<210> 78
   <211> 369
   <212> DNA
   <213> Artificial
<220>
   <223> GBR VH5(K3Q,V37A) heavy chain variable domain cDNA
<400> 78
   gaagtgcagc tgctggaaag cggcggaggc ctggtgcagc ctggcggaag cctgagactg 60
   agctgtgccg ccagcggctt caccttcagc acctacacca tgagctgggc ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 79
   <211> 369
   <212> DNA
   <213> Artificial
<220>
   <223> GBR VH5(K3Q,T40A) heavy chain variable domain cDNA
<400> 79
   gaagtgcagc tgctggaaag cggcggaggc ctggtgcagc ctggcggaag cctgagactg 60
   agctgtgccg ccagcggctt caccttcagc acctacacca tgagctgggt ccgacaggcc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 80
   <211> 369
   <212> DNA
   <213> Artificial
<220>
   <223> GBR VH5(P60A,T62S) heavy chain variable domain cDNA
<400> 80
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 81
   <211> 369
   <212> DNA
   <213> Artificial
<220>
   <223> GBR VH5(K3Q,V37A,R44G) heavy chain variable domain cDNA
<400> 81
   gaagtgcagc tgctggaaag cggcggaggc ctggtgcagc ctggcggaag cctgagactg      60
   agctgtgccg ccagcggctt caccttcagc acctacacca tgagctgggc ccgacagacc     120
   cccggcaagg ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 82
   <211> 369
   <212> DNA
   <213> Artificial
<220>
   <223> GBR VH5(K3Q,A49S,Y50A) heavy chain variable domain cDNA
<400> 82
   gaagtgcagc tgctggaaag cggcggaggc ctggtgcagc ctggcggaag cctgagactg      60
   agctgtgccg ccagcggctt caccttcagc acctacacca tgagctgggt ccgacagacc 120
   cccggcaagc ggctggaatg ggtgagcgcc atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 83
   <211> 369
   <212> DNA
   <213> Artificial
<220>
   <223> GBR VH5(K3Q,P60A,T62S) heavy chain variable domain cDNA
<400> 83
   gaagtgcagc tgctggaaag cggcggaggc ctggtgcagc ctggcggaag cctgagactg 60
   agctgtgccg ccagcggctt caccttcagc acctacacca tgagctgggt ccgacagacc 120
   cccggcaagc ggctggaatg ggtggcctac atcagcaagg gcggaggcag cacctactac 180
   gccgacagcg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagc 369
<210> 84
   <211> 369
   <212> DNA
   <213> Artificial
<220>
   <223> GBR VH5(K3Q,T40A,P60A,T62S) heavy chain variable domain cDNA
<400> 84
   gaagtgcagc tgctggaaag cggcggaggc ctggtgcagc ctggcggaag cctgagactg      60
   agctgtgccg ccagcggctt caccttcagc acctacacca tgagctgggt ccgacaggcc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 85
   <211> 369
   <212> DNA
   <213> Artificial
<220>
   <223> GBR VH5(K3Q,V37A,T40A,P60A,T62S) heavy chain variable domain cDNA
<400> 85
   gaagtgcagc tgctggaaag cggcggaggc ctggtgcagc ctggcggaag cctgagactg      60
   agctgtgccg ccagcggctt caccttcagc acctacacca tgagctgggc ccgacaggcc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 86
   <211> 369
   <212> DNA
   <213> Artificial
<220>
   <223> GBR VH5(K3Q,T40A,R44G,A49S,Y50A) heavy chain variable domain cDNA
<400> 86
   gaagtgcagc tgctggaaag cggcggaggc ctggtgcagc ctggcggaag cctgagactg      60
   agctgtgccg ccagcggctt caccttcagc acctacacca tgagctgggt ccgacaggcc     120
   cccggcaagg gcctggaatg ggtgagcgcc atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 87
   <211> 369
   <212> DNA
   <213> Artificial
<220>
   <223> GBR VH5(K3Q,A49S,Y50A,P60A,T62S) heavy chain variable domain cDNA
<400> 87
   gaagtgcagc tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtgagcgcc atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagc                                                             369
<210> 88
   <211> 369
   <212> DNA
   <213> Artificial
<220>
   <223> GBR VH5(K3Q,T40A,R44G,A49S,Y50A,P60A,T62S) heavy chain variable domain cDNA
<400> 88
   gaagtgcagc tgctggaaag cggcggaggc ctggtgcagc ctggcggaag cctgagactg     60
   agctgtgccg ccagcggctt caccttcagc acctacacca tgagctgggt ccgacaggcc    120
   cccggcaagg gcctggaatg ggtgagcgcc atcagcaagg gcggaggcag cacctactac    180
   gccgacagcg tgaagggccg gttcaccatc agccgggaca acagcaagaa caccctgtac    240
   ctgcagatga acagcctgcg ggccgaggac accgccgtgt actactgtgc cagaggcgct    300
   atgtacggca acgacttctt ctaccctatg gactactggg gccagggcac caccgtgacc    360
   gtgtctagc                                                            369
<210> 89
   <211> 369
   <212> DNA
   <213> Artificial
<220>
   <223> GBR VH5(K3Q,T40A,R44G,A49S,Y50A,P60A,T62S,R94K) heavy chain variable domain cDNA
<400> 89
   gaagtgcagc tgctggaaag cggcggaggc ctggtgcagc ctggcggaag cctgagactg     60
   agctgtgccg ccagcggctt caccttcagc acctacacca tgagctgggt ccgacaggcc    120
   cccggcaagg gcctggaatg ggtgagcgcc atcagcaagg gcggaggcag cacctactac    180
   gccgacagcg tgaagggccg gttcaccatc agccgggaca acagcaagaa caccctgtac    240
   ctgcagatga acagcctgcg ggccgaggac accgccgtgt actactgtgc caagggcgct    300
   atgtacggca acgacttctt ctaccctatg gactactggg gccagggcac caccgtgacc    360
   gtgtctagc                                                            369
<210> 90
   <211> 369
   <212> DNA
   <213> Artificial
<220>
   <223> GBR VH1(V37A) heavy chain variable domain cDNA
<400> 90
   gaagtgcagc tgctggaaag cggcggaggg ctggtgcagc caggcggcag cctgaggctg      60
   tcctgcgccg ccagcggctt caccttcagc acctacacca tgagctgggc ccgacaggcc     120
   ccaggcaagg gcctggaatg ggtgtcctac atcagcaagg gcggaggaag cacctactac     180
   cccgacaccg tgaagggcag gttcaccatc agcagggaca acagcaagaa caccctgtac     240
   ctgcagatga acagcctgag ggccgaggac accgccgtgt actactgcgc caagggcgcc     300
   atgtacggca acgacttttt ctaccccatg gactactggg ggcagggcac caccgtgacc     360
   gtgtctagc                                                             369
<210> 91
   <211> 369
   <212> DNA
   <213> Artificial
<220>
   <223> GBR VH3(V37A) heavy chain variable domain cDNA
<400> 91
   gaagtgcagc tgctggaaag cggcggaggg ctggtgcagc caggcggcag cctgaggctg      60
   tcctgcgccg ccagcggctt caccttcagc acctacacca tgagctgggc ccgacagacc     120
   cccggcaaga ggctggaatg ggtggcctac atcagcaagg gcggaggaag cacctactac     180
   cccgacaccg tgaagggcag gttcaccatc agcagggaca acagcaagaa caccctgtac     240
   ctgcagatga acagcctgag ggccgaggac accgccgtgt actactgcgc caagggcgcc     300
   atgtacggca acgacttttt ctaccccatg gactactggg ggcagggcac caccgtgacc     360
   gtgtctagc                                                             369
<210> 92
   <211> 1359
   <212> DNA
   <213> Artificial
<220>
   <223> GBR VH5(K3Q) heavy chain IGHG1 cDNA
<400> 92
   gaagtgcagc tgctggaaag cggcggaggc ctggtgcagc ctggcggaag cctgagactg 60
   agctgtgccg ccagcggctt caccttcagc acctacacca tgagctgggt ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 93
   <211> 1359
   <212> DNA
   <213> Artificial
<220>
   <223> GBR VH5(V37A) heavy chain IGHG1 cDNA
<400> 93
   gaagtgaaac tgctggaaag cggcggaggc ctggtgcagc ctggcggaag cctgagactg      60
   agctgtgccg ccagcggctt caccttcagc acctacacca tgagctgggc ccgacagacc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                            1359
<210> 94
   <211> 1350
   <212> DNA
   <213> Artificial
<220>
   <223> GBR VH5(V37A) heavy chain IGHG4 S228P cDNA
<400> 94
   gaagtgaaac tgctggaaag cggcggaggc ctggtgcagc ctggcggaag cctgagactg     60
   agctgtgccg ccagcggctt caccttcagc acctacacca tgagctgggc ccgacagacc    120
   cccggcaagc ggctggaatg ggtggcctac atcagcaagg gcggaggcag cacctactac    180
   cccgacaccg tgaagggccg gttcaccatc agccgggaca acagcaagaa caccctgtac    240
   ctgcagatga acagcctgcg ggccgaggac accgccgtgt actactgtgc cagaggcgct    300
   atgtacggca acgacttctt ctaccctatg gactactggg gccagggcac caccgtgacc    360
   gtgtctagcg cgtcgaccaa gggccccagc gtgttccccc tggccccctg cagcagaagc    420
   accagcgagt ccacagccgc cctgggctgt ctggtgaagg actacttccc cgagcccgtg    480
   accgtgtcct ggaacagcgg agccctgacc agcggcgtgc acaccttccc cgccgtgctg    540
   cagagcagcg gcctgtacag cctgagcagc gtggtgacag tgcccagcag cagcctgggc    600
   accaagacct acacctgcaa cgtggaccac aagcccagca acaccaaggt ggacaagagg    660
   gtggagagca agtacggccc accctgcccc ccatgcccag cccccgagtt cctgggcgga    720
   ccctccgtgt tcctgttccc ccccaagccc aaggacaccc tgatgatcag caggaccccc    780
   gaggtgacct gcgtggtggt ggacgtgagc caggaggacc cagaggtcca gttcaactgg    840
   tacgtggacg gcgtggaggt gcacaacgcc aagaccaagc ccagagagga gcagtttaac    900
   agcacctaca gggtggtgtc cgtgctgacc gtgctgcacc aggactggct gaacggcaag    960
   gaatacaagt gcaaggtctc caacaagggc ctgcccagct ccatcgagaa aaccatcagc   1020
   aaggccaagg gccagccacg ggagccccag gtgtacaccc tgccaccctc ccaggaggag   1080
   atgaccaaga accaggtgtc cctgacctgc ctggtgaagg gcttctaccc cagcgacatc   1140
   gccgtggagt gggagagcaa cggccagccc gagaacaact acaagaccac ccccccagtg   1200
   ctggacagcg acggcagctt cttcctgtac agcaggctga ccgtggacaa gtccaggtgg   1260
   caggagggca acgtctttag ctgcagcgtg atgcacgagg ccctgcacaa ccactacacc   1320
   cagaagagcc tgagcctgtc cctgggcaag                                    1350
<210> 95
   <211> 1359
   <212> DNA
   <213> Artificial
<220>
   <223> GBR VH5(G42E) heavy chain IGHG1 cDNA
<400> 95
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccgaaaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc   1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 96
   <211> 1359
   <212> DNA
   <213> Artificial
<220>
   <223> GBR VH5(V89L) heavy chain IGHG1 cDNA
<400> 96
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccctgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc     1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 97
   <211> 1359
   <212> DNA
   <213> Artificial
<220>
   <223> GBR VH5(R94K) heavy chain IGHG1 cDNA
<400> 97
   gaagtgaaac tgctggaaag cggcggaggc ctggtgcagc ctggcggaag cctgagactg 60
   agctgtgccg ccagcggctt caccttcagc acctacacca tgagctgggt ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc caagggcgct 300
   atgtacggca acgacttctt ctaccctatg gactactggg gccagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 98
   <211> 1359
   <212> DNA
   <213> Artificial
<220>
   <223> GBR VH5(K3Q,V37A) heavy chain IGHG1 cDNA
<400> 98
   gaagtgcagc tgctggaaag cggcggaggc ctggtgcagc ctggcggaag cctgagactg      60
   agctgtgccg ccagcggctt caccttcagc acctacacca tgagctgggc ccgacagacc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 99
   <211> 1350
   <212> DNA
   <213> Artificial
<220>
   <223> GBR VH5(K3Q,V37A) heavy chain IGHG4 S228P cDNA
<400> 99
   gaagtgcagc tgctggaaag cggcggaggc ctggtgcagc ctggcggaag cctgagactg 60
   agctgtgccg ccagcggctt caccttcagc acctacacca tgagctgggc ccgacagacc 120
   cccggcaagc ggctggaatg ggtggcctac atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagcg cgtcgaccaa gggccccagc gtgttccccc tggccccctg cagcagaagc 420
   accagcgagt ccacagccgc cctgggctgt ctggtgaagg actacttccc cgagcccgtg 480
   accgtgtcct ggaacagcgg agccctgacc agcggcgtgc acaccttccc cgccgtgctg 540
   cagagcagcg gcctgtacag cctgagcagc gtggtgacag tgcccagcag cagcctgggc 600
   accaagacct acacctgcaa cgtggaccac aagcccagca acaccaaggt ggacaagagg 660
   gtggagagca agtacggccc accctgcccc ccatgcccag cccccgagtt cctgggcgga 720
   ccctccgtgt tcctgttccc ccccaagccc aaggacaccc tgatgatcag caggaccccc 780
   gaggtgacct gcgtggtggt ggacgtgagc caggaggacc cagaggtcca gttcaactgg 840
   tacgtggacg gcgtggaggt gcacaacgcc aagaccaagc ccagagagga gcagtttaac 900
   agcacctaca gggtggtgtc cgtgctgacc gtgctgcacc aggactggct gaacggcaag 960
   gaatacaagt gcaaggtctc caacaagggc ctgcccagct ccatcgagaa aaccatcagc 1020
   aaggccaagg gccagccacg ggagccccag gtgtacaccc tgccaccctc ccaggaggag 1080
   atgaccaaga accaggtgtc cctgacctgc ctggtgaagg gcttctaccc cagcgacatc 1140
   gccgtggagt gggagagcaa cggccagccc gagaacaact acaagaccac ccccccagtg 1200
   ctggacagcg acggcagctt cttcctgtac agcaggctga ccgtggacaa gtccaggtgg 1260
   caggagggca acgtctttag ctgcagcgtg atgcacgagg ccctgcacaa ccactacacc 1320
   cagaagagcc tgagcctgtc cctgggcaag 1350
<210> 100
   <211> 1359
   <212> DNA
   <213> Artificial
<220>
   <223> GBR VH5(K3Q,T40A) heavy chain IGHG1 cDNA
<400> 100
   gaagtgcagc tgctggaaag cggcggaggc ctggtgcagc ctggcggaag cctgagactg      60
   agctgtgccg ccagcggctt caccttcagc acctacacca tgagctgggt ccgacaggcc     120
   cccggcaagc ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 101
   <211> 1359
   <212> DNA
   <213> Artificial
<220>
   <223> GBR VH5(P60A,T62S) heavy chain IGHG1 cDNA
<400> 101
   gaagtgaaac tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 102
   <211> 1359
   <212> DNA
   <213> Artificial
<220>
   <223> GBR VH5(K3Q,V37A,R44G) heavy chain IGHG1 cDNA
<400> 102
   gaagtgcagc tgctggaaag cggcggaggc ctggtgcagc ctggcggaag cctgagactg      60
   agctgtgccg ccagcggctt caccttcagc acctacacca tgagctgggc ccgacagacc     120
   cccggcaagg ggctggaatg ggtggcctac atcagcaagg gcggaggcag cacctactac     180
   cccgacaccg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                            1359
<210> 103
   <211> 1359
   <212> DNA
   <213> Artificial
<220>
   <223> GBR VH5(K3Q,A49S,Y50A) heavy chain IGHG1 cDNA
<400> 103
   gaagtgcagc tgctggaaag cggcggaggc ctggtgcagc ctggcggaag cctgagactg 60
   agctgtgccg ccagcggctt caccttcagc acctacacca tgagctgggt ccgacagacc 120
   cccggcaagc ggctggaatg ggtgagcgcc atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 104
   <211> 1359
   <212> DNA
   <213> Artificial
<220>
   <223> GBR VH5(K3Q,P60A,T62S) heavy chain IGHG1 cDNA
<400> 104
   gaagtgcagc tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 105
   <211> 1359
   <212> DNA
   <213> Artificial
<220>
   <223> GBR VH5(K3Q,T40A,P60A,T62S) heavy chain IGHG1 cDNA
<400> 105
   gaagtgcagc tgctggaaag cggcggaggc ctggtgcagc ctggcggaag cctgagactg 60
   agctgtgccg ccagcggctt caccttcagc acctacacca tgagctgggt ccgacaggcc 120
   cccggcaagc ggctggaatg ggtggcctac atcagcaagg gcggaggcag cacctactac 180
   gccgacagcg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 106
   <211> 1359
   <212> DNA
   <213> Artificial
<220>
   <223> GBR VH5(K3Q,V37A,T40A,P60A,T62S) heavy chain IGHG1 cDNA
<400> 106
   gaagtgcagc tgctggaaag cggcggaggc ctggtgcagc ctggcggaag cctgagactg      60
   agctgtgccg ccagcggctt caccttcagc acctacacca tgagctgggc ccgacaggcc     120
   cccggcaagc ggctggaatg ggtggcctac atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 107
   <211> 1359
   <212> DNA
   <213> Artificial
<220>
   <223> GBR VH5(K3Q,T40A,R44G,A49S,Y50A) heavy chain IGHG1 cDNA
<400> 107
   gaagtgcagc tgctggaaag cggcggaggc ctggtgcagc ctggcggaag cctgagactg 60
   agctgtgccg ccagcggctt caccttcagc acctacacca tgagctgggt ccgacaggcc 120
   cccggcaagg gcctggaatg ggtgagcgcc atcagcaagg gcggaggcag cacctactac 180
   cccgacaccg tgaagggccg gttcaccatc agccgggaca acagcaagaa caccctgtac 240
   ctgcagatga acagcctgcg ggccgaggac accgccgtgt actactgtgc cagaggcgct 300
   atgtacggca acgacttctt ctaccctatg gactactggg gccagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 108
   <211> 1359
   <212> DNA
   <213> Artificial
<220>
   <223> GBR VH5(K3Q,A49S,Y50A,P60A,T62S) heavy chain IGHG1 cDNA
<400> 108
   gaagtgcagc tgctggaaag cggcggaggc ctggtgcagc ctggcggaag cctgagactg      60
   agctgtgccg ccagcggctt caccttcagc acctacacca tgagctgggt ccgacagacc     120
   cccggcaagc ggctggaatg ggtgagcgcc atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc  1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 109
   <211> 1359
   <212> DNA
   <213> Artificial
<220>
   <223> GBR VH5(K3Q,T40A,R44G,A49S,Y50A,P60A,T62S) heavy chain IGHG1 cDNA
<400> 109
   gaagtgcagc tgctggaaag cggcggaggc ctggtgcagc ctggcggaag cctgagactg      60
   agctgtgccg ccagcggctt caccttcagc acctacacca tgagctgggt ccgacaggcc     120
   cccggcaagg gcctggaatg ggtgagcgcc atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc cagaggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc   1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 110
   <211> 1359
   <212> DNA
   <213> Artificial
<220>
   <223> GBR VH5(K3Q,T40A,R44G,A49S,Y50A,P60A,T62S,R94K) heavy chain IGHG1 cDNA
<400> 110
   gaagtgcagc tgctggaaag cggcggaggc ctggtgcagc ctggcggaag cctgagactg      60
   agctgtgccg ccagcggctt caccttcagc acctacacca tgagctgggt ccgacaggcc     120
   cccggcaagg gcctggaatg ggtgagcgcc atcagcaagg gcggaggcag cacctactac     180
   gccgacagcg tgaagggccg gttcaccatc agccgggaca acagcaagaa caccctgtac     240
   ctgcagatga acagcctgcg ggccgaggac accgccgtgt actactgtgc caagggcgct     300
   atgtacggca acgacttctt ctaccctatg gactactggg gccagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                           1359
<210> 111
   <211> 1359
   <212> DNA
   <213> Artificial
<220>
   <223> GBR VH1(V37A) heavy chain IGHG1 cDNA
<400> 111
   gaagtgcagc tgctggaaag cggcggaggg ctggtgcagc caggcggcag cctgaggctg      60
   tcctgcgccg ccagcggctt caccttcagc acctacacca tgagctgggc ccgacaggcc     120
   ccaggcaagg gcctggaatg ggtgtcctac atcagcaagg gcggaggaag cacctactac     180
   cccgacaccg tgaagggcag gttcaccatc agcagggaca acagcaagaa caccctgtac     240
   ctgcagatga acagcctgag ggccgaggac accgccgtgt actactgcgc caagggcgcc     300
   atgtacggca acgacttttt ctaccccatg gactactggg ggcagggcac caccgtgacc     360
   gtgtctagcg cgtcgaccaa gggccccagc gtgttcccgc tagcccccag cagcaagagc     420
   accagcggcg gcacagccgc cctgggctgc ctggtgaagg actacttccc cgagcccgtg     480
   accgtgtcct ggaactctgg agccctgacc tccggcgtgc acaccttccc cgccgtgctc     540
   cagagcagcg gcctgtacag cctgagcagc gtggtgacag tgcccagcag cagcctggga     600
   acccagacct acatctgcaa cgtgaaccac aagcccagca acaccaaggt ggacaagaag     660
   gtggagccca agagctgcga caagacccac acctgccccc cctgccctgc ccctgagctg     720
   ctgggcggac cctccgtgtt cctgttcccc cccaagccca aggacaccct gatgatcagc     780
   cggacccccg aggtgacctg cgtggtggtg gacgtgagcc acgaggaccc tgaggtgaag     840
   ttcaattggt acgtggacgg cgtggaggtg cacaacgcca agaccaagcc ccgggaggaa     900
   cagtacaaca gcacctaccg ggtggtgtcc gtgctgaccg tgctgcacca ggactggctg     960
   aacggcaagg aatacaagtg caaggtctcc aacaaggccc tgcctgcccc catcgaaaag    1020
   accatcagca aggccaaggg ccagcccagg gagccccagg tgtacaccct gcccccctcc    1080
   cgggacgagc tgaccaagaa ccaggtgtcc ctgacctgtc tggtgaaggg cttctacccc    1140
   agcgacatcg ccgtggagtg ggagagcaac ggccagcccg agaacaacta caagaccacc    1200
   ccccctgtgc tggacagcga cggcagcttc ttcctgtaca gcaagctgac cgtggacaag    1260
   agccggtggc agcagggcaa cgtgttcagc tgctccgtga tgcacgaggc cctgcacaac    1320
   cactacaccc agaagagcct gagcctgtcc cccggcaag                          1359
<210> 112
   <211> 1359
   <212> DNA
   <213> Artificial
<220>
   <223> GBR900 VH3(V37A) heavy chain IGHG1 cDNA
<400> 112
   gaagtgcagc tgctggaaag cggcggaggg ctggtgcagc caggcggcag cctgaggctg 60
   tcctgcgccg ccagcggctt caccttcagc acctacacca tgagctgggc ccgacagacc 120
   cccggcaaga ggctggaatg ggtggcctac atcagcaagg gcggaggaag cacctactac 180
   cccgacaccg tgaagggcag gttcaccatc agcagggaca acagcaagaa caccctgtac 240
   ctgcagatga acagcctgag ggccgaggac accgccgtgt actactgcgc caagggcgcc 300
   atgtacggca acgacttttt ctaccccatg gactactggg ggcagggcac caccgtgacc 360
   gtgtctagcg cgtcgaccaa gggccccagc gtgttcccgc tagcccccag cagcaagagc 420
   accagcggcg gcacagccgc cctgggctgc ctggtgaagg actacttccc cgagcccgtg 480
   accgtgtcct ggaactctgg agccctgacc tccggcgtgc acaccttccc cgccgtgctc 540
   cagagcagcg gcctgtacag cctgagcagc gtggtgacag tgcccagcag cagcctggga 600
   acccagacct acatctgcaa cgtgaaccac aagcccagca acaccaaggt ggacaagaag 660
   gtggagccca agagctgcga caagacccac acctgccccc cctgccctgc ccctgagctg 720
   ctgggcggac cctccgtgtt cctgttcccc cccaagccca aggacaccct gatgatcagc 780
   cggacccccg aggtgacctg cgtggtggtg gacgtgagcc acgaggaccc tgaggtgaag 840
   ttcaattggt acgtggacgg cgtggaggtg cacaacgcca agaccaagcc ccgggaggaa 900
   cagtacaaca gcacctaccg ggtggtgtcc gtgctgaccg tgctgcacca ggactggctg 960
   aacggcaagg aatacaagtg caaggtctcc aacaaggccc tgcctgcccc catcgaaaag 1020
   accatcagca aggccaaggg ccagcccagg gagccccagg tgtacaccct gcccccctcc 1080
   cgggacgagc tgaccaagaa ccaggtgtcc ctgacctgtc tggtgaaggg cttctacccc 1140
   agcgacatcg ccgtggagtg ggagagcaac ggccagcccg agaacaacta caagaccacc 1200
   ccccctgtgc tggacagcga cggcagcttc ttcctgtaca gcaagctgac cgtggacaag 1260
   agccggtggc agcagggcaa cgtgttcagc tgctccgtga tgcacgaggc cctgcacaac 1320
   cactacaccc agaagagcct gagcctgtcc cccggcaag 1359
<210> 113
   <211> 318
   <212> DNA
   <213> Artificial
<220>
   <223> VL1 variable domain cDNA
<400> 113
   gagatcgtgc tgacccagag ccccgccacc ctgtctctga gccctggcga gagagccacc 60
   ctgagctgta gcgcctccag cagcgtgtcc tacatgcact ggtatcagca gaagcccggc 120
   caggccccca gactgctgat ctacaccacc agcaacctgg ccagcggcat ccccgccaga 180
   ttttctggca gcggcagcgg caccgacttc accctgacca tcagcagcct ggaacccgag 240
   gatttcgccg tgtattattg ccaccagtgg tccagctacc cctggacctt cggccagggg 300
   accaagctgg aaatcaag 318
<210> 114
   <211> 318
   <212> DNA
   <213> Artificial
<220>
   <223> VL3 variable domain cDNA
<400> 114
   cagatcgtgc tgacccagag ccccgccacc ctgagcctga gcccaggcga gagggccacc 60
   ctgtcctgca gcgccagcag cagcgtgagc tacatgcact ggtaccagca gaagcccggc 120
   cagagcccaa gactgctgat ctacaccacc tccaacctgg ccagcggcat ccccagcagg 180
   ttcagcggca gcggctccgg caccttctac accctgacca tcagcagcct ggaacccgag 240
   gacttcgccg tgtactactg ccaccagtgg agcagctacc cctggacctt cggcggcggg 300
   accaagctgg aaatcaag 318
<210> 115
   <211> 639
   <212> DNA
   <213> Artificial
<220>
   <223> BXhVL1 light chain cDNA
<400> 115
   gagatcgtgc tgacccagag ccccgccacc ctgtctctga gccctggcga gagagccacc     60
   ctgagctgta gcgcctccag cagcgtgtcc tacatgcact ggtatcagca gaagcccggc    120
   caggccccca gactgctgat ctacaccacc agcaacctgg ccagcggcat ccccgccaga    180
   ttttctggca gcggcagcgg caccgacttc accctgacca tcagcagcct ggaacccgag    240
   gatttcgccg tgtattattg ccaccagtgg tccagctacc cctggacctt cggccagggg    300
   accaagctgg aaatcaagcg tacggtggcc gctcccagcg tgttcatctt cccccccagc    360
   gacgagcagc tgaagagcgg caccgcctcc gtggtgtgcc tgctgaacaa cttctacccc    420
   cgggaggcca aggtgcagtg gaaggtggac aacgccctcc agagcggcaa cagccaggaa    480
   agcgtcaccg agcaggacag caaggactcc acctacagcc tgagcagcac cctgaccctg    540
   agcaaggccg actacgagaa gcacaaggtg tacgcctgcg aggtgaccca ccagggcctg    600
   tccagccccg tgaccaagag cttcaaccgg ggcgagtgc                           639
<210> 116
   <211> 639
   <212> DNA
   <213> Artificial
<220>
   <223> BXhVL3 light chain cDNA
<400> 116
   cagatcgtgc tgacccagag ccccgccacc ctgagcctga gcccaggcga gagggccacc     60
   ctgtcctgca gcgccagcag cagcgtgagc tacatgcact ggtaccagca gaagcccggc    120
   cagagcccaa gactgctgat ctacaccacc tccaacctgg ccagcggcat ccccagcagg    180
   ttcagcggca gcggctccgg caccttctac accctgacca tcagcagcct ggaacccgag    240
   gacttcgccg tgtactactg ccaccagtgg agcagctacc cctggacctt cggcggcggg    300
   accaagctgg aaatcaagcg tacggtggcc gctcccagcg tgttcatctt cccccccagc    360
   gacgagcagc tgaagagcgg caccgcctcc gtggtgtgcc tgctgaacaa cttctacccc    420
   cgggaggcca aggtgcagtg gaaggtggac aacgccctcc agagcggcaa cagccaggaa    480
   agcgtcaccg agcaggacag caaggactcc acctacagcc tgagcagcac cctgaccctg    540
   agcaaggccg actacgagaa gcacaaggtg tacgcctgcg aggtgaccca ccagggcctg    600
   tccagccccg tgaccaagag cttcaaccgg ggcgagtgc                           639

## Description

### The field of the invention

The present invention relates generally to antibodies directed against TrkA receptor and their uses, including humanized anti-TrkA antibodies and methods of treatment with anti-TrkA antibodies. In one aspect, the present invention relates to humanized anti-TrkA antibodies with enhanced inhibitory properties.

In a further aspect, the present invention relates to humanized anti-TrkA antibodies with enhanced inhibitory properties comprising a heavy chain variable region, a light chain variable region, a human light chain constant region and a variant human IgG4 heavy chain constant region which exhibit altered exchange properties.

### Background of the invention

Neurotrophins are a family of peptide growth factors (Barde YA (1994) J. Neurobiol. 25(11): 1329-33) structurally related to the first member of the family NGF (Levi-Montalcini R (1987) EMBO J. 6(5):1145-54). Neurotrophins modulate neuronal differentiation and survival, as well as synaptic transmission, both of the peripheral neurons and of the central nervous system. Furthermore NGF acts on various non neuronal tissues and cells, such as immune cells. NGF acts through two membrane receptors present in the target cells, the low affinity p75 receptor, and the 140 kDa high affinity transmembrane glycoprotein, TrkA (Kaplan DR et al., (1991) Science 252(5005):554-8; Klein R et al., (1991) Cell 65(1):189-97) having tyrosine kinase activity. TrkA is expressed in neural-crest neurons, in sympathetic neurons as well as in cholinergic neurons of the basal fore-brain and corpus striatum, where it represents the crucial mediator of NGF activities (Holtzman DM et al., (1992) Neuron 9(3):465-78; Verge VM et al., (1992) J. Neurosci. 12(10):4011-22). TrkA is also expressed in some non neuronal tissues and cells, including B lymphocytes (Torcia M et al., (1996) Cell 85(3):345-56).

Nerve growth factor (NGF) was identified originally as a survival factor for sensory and sympathetic neurons in the developing nervous system (Gorin PD & Johnson EM (1979) PNAS USA, 76(10):5382-6). In adults, NGF is not required for survival but it has a crucial role in the generation of pain and hyperalgesia in several acute and chronic pain states. The expression of NGF is high in injured and inflamed tissues, and activation of trkA on nociceptive neurons triggers and potentiates pain signalling by multiple mechanisms. Inflammation-related pain can be significantly reduced by neutralizing NGF bioactivity in animal models (Woolf CJ et al., (1994) Neuroscience 62(2):327-31; McMahon SB et al., (1995) Nat. Med. 1(8):774-80; Koltzenburg M et al., (1999) Eur. J. Neurosci. 11(5):1698-704), implying that an enhanced level of this neurotrophin is necessary to generate the full hyperalgesic response. Remarkably, the inhibition of other neurotrophins does not result in antagonizing the induced hyperalgesia, suggesting that this effect is specific to NGF (McMahon SB et al., (1995) Nat. Med. 1(8):774-80); in addition, NGF inhibition results in analgesia in different neuropathy-related pain protocols (Koltzenburg M et al., (1999) Eur. J. Neurosci. 11(5): 1698-704; Ro LS et al., (1999) Pain 79(2-3):265-74; Theodosiou M et al., (1999) Pain, 81(3):245-55; Christensen MD & Hulsebosch CE (1997) Exp. Neurol. 147(2):463-75). There is a large, unmet medical need in the treatment of pain. The dominant classes of analgesic drugs, the nonsteroidal anti-inflammatory drugs (NSAIDs) and the opiates are limited by their efficacy and tolerability (Hefti FF et al., (2006) Trends Pharmacol. Sci. 27(2): 85-91). Less than 30% of patients with chronic pain obtain adequate relief with current therapies, and there are many adverse effects, particularly with long-term administration (Kalso E et al., (2004) Pain 112(3):372-80). The recognition that NGF has a central role in pain mechanisms in adults provides an opportunity to develop a completely novel class of pain therapeutics.

Targeting TrkA instead of NGF may represent a better therapeutic choice as this receptor does not interfere with NGF functions mediated by the p75 receptor, the latter having broad function in neuronal development. Antibodies that specifically bind to TrkA have been previously described in WO2009/098238.

### Summary of the invention

The present disclosure relates generally to humanized anti-TrkA antibodies, methods for their preparation and use.

In one aspect, the present disclosure provides a humanized anti-TrkA antibody or fragment thereof comprising:
a) a heavy chain variable domain comprising a sequence selected from the group consisting of SEQ ID NOs: 1-5, and
b) a light chain variable domain comprising a sequence selected from the group consisting of SEQ ID NOs: 6-13,
wherein CDR2 of the heavy chain variable domain comprises at least one amino acid substitution and/or wherein the non-CDR region of the heavy chain variable domain comprises an amino acid substitution at an amino acid position selected from the group consisting of 37, 42 and 89, wherein the amino acid position of each group member is indicated utilizing the numbering system set forth in Kabat.

In further aspects, the present disclosure provides an isolated nucleic acid encoding a humanized anti-TrkA antibody or fragment thereof, a vector comprising the isolated nucleic acid and a host cell comprising the isolated nucleic acid or the vector. Also provided by the present disclosure is a method of producing a humanized anti-TrkA antibody or fragment thereof.

In further aspects, the present disclosure provides a composition comprising a humanized anti-TrkA antibody or fragment thereof and an immunoconjugate comprising a humanized anti-TrkA antibody or fragment thereof linked to a therapeutic agent.
In further aspects, the present disclosure provides a humanized anti-TrkA antibody or fragment thereof, a composition or an immunoconjugate for use in medicine, for use in the treatment of pain, for use in the treatment of chronic pain, for use in the treatment of acute pain, for use in the treatment of pain associated with one or more of the following: pancreatitis, kidney stones, endometriosis, IBD, Crohn's disease, post surgical adhesions, gall bladder stones, headaches, dysmenorrhea, musculoskeletal pain, sprains, visceral pain, ovarian cysts, prostatitis, cystitis, interstitial cystitis, post-operative pain, migraine, trigeminal neuralgia, pain from bums and/or wounds, pain associated with trauma, neuropathic pain, pain associated with musculoskeletal diseases, rheumatoid arthritis, osteoarthritis, ankylosing spondilitis, periarticular pathologies, oncological pain, pain from bone metastases, HIV infection, for use in the treatment of cancer, a neuronal disorder, Alzheimer's disease, diabetes mellitus, diabetic nephropathy, a viral disorder, an HIV mediated disorder, leprosy, or an inflammatory disorder and for use in diagnosis or prognosis.

In another aspect, in an *in vivo* model of chronic inflammatory hyperalgesia, administration of a humanized anti-TrkA antibody resulted in a significant and sustained reversal of chronic inflammatory hyperalgesia at a dose of 0.01mg/kg. In another aspect, in an *in vivo* model of chronic osteoarthritic hyperalgesia, administration of a humanized anti-TrkA antibody resulted in a significant and sustained reversal of chronic osteoarthritic hyperalgesia at a dose of 0.01mg/kg. In a further aspect, in the *in vivo* chronic constriction injury model of neuropathic pain, administration of a humanized anti-TrkA antibody resulted in a significant reversal of neuropathic pain at a dose of 1.0mg/kg.

In a further aspect, the present disclosure provides an article of manufacture comprising a humanized anti-TrkA antibody or fragment thereof, a composition or an immunoconjugate.

In a further aspect, the present disclosure provides a kit comprising a humanized anti-TrkA antibody or fragment thereof, a composition or an immunoconjugate.

### Brief description of the figures

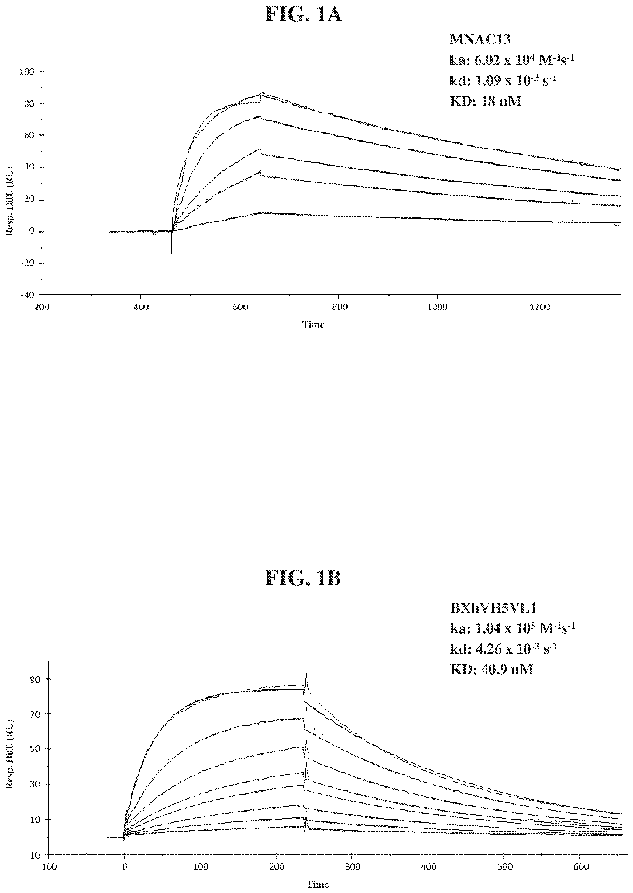

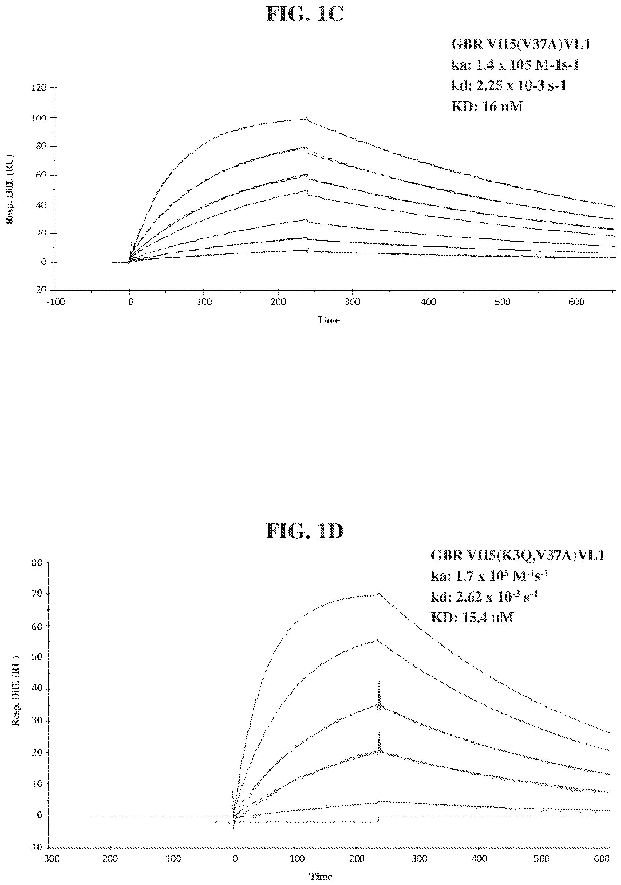

FIG. 1: Surface Plasmon resonance measurements of anti-TrkA antibodies. Data are expressed as number of response (abbreviated RU; Y axis) vs. time (X axis). (FIG. 1A) MNAC13 antibody. (FIG. 1B) BXhVH5VL1 antibody. (FIG. 1C) GBR VH5(V37A)VL1 antibody. (FIG. 1D) GBR VH5(K3Q,V37A)VL1 antibody.

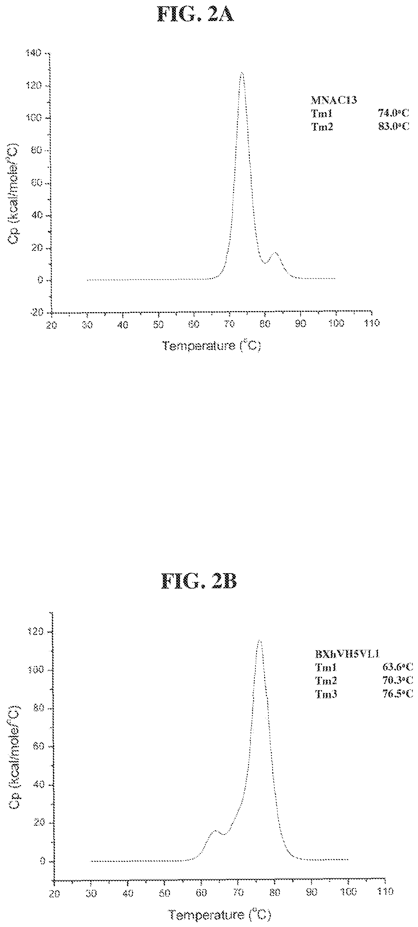

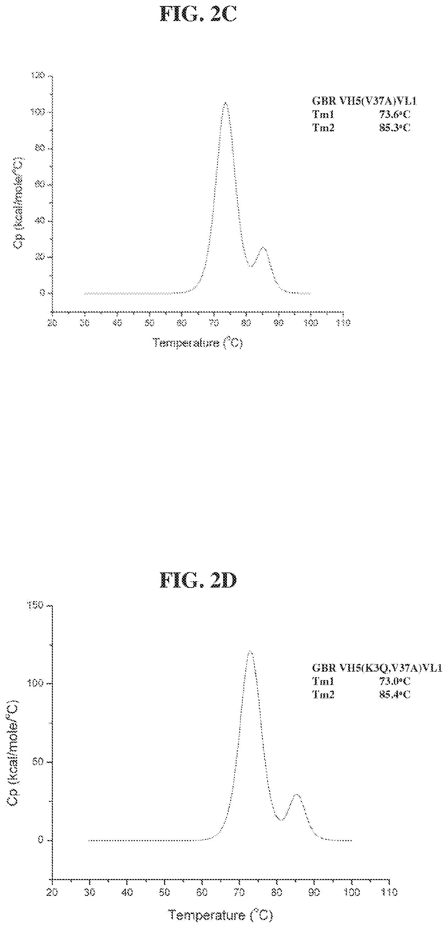

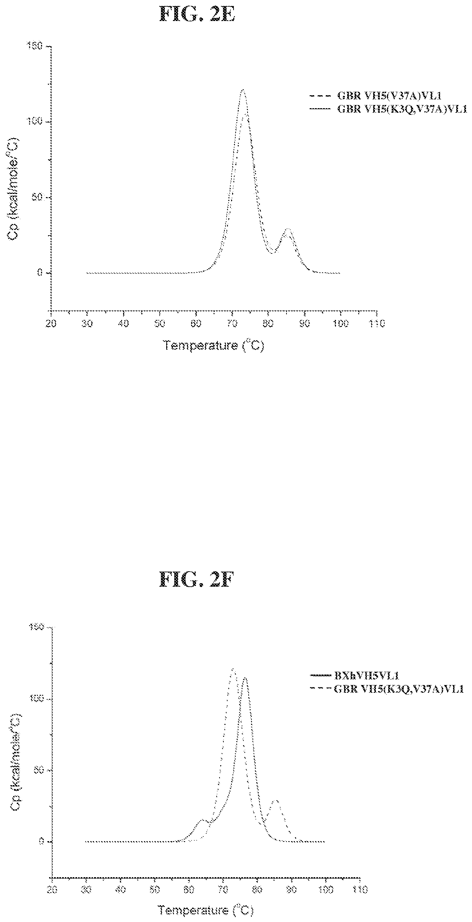

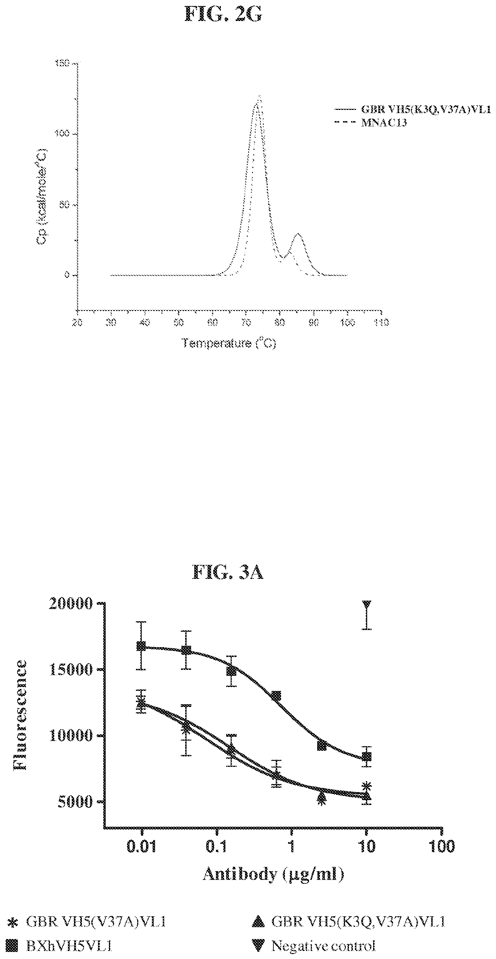

FIG. 2: Thermostability measurements of anti-TrkA antibodies using differential scanning calorimetry. Data are expressed as excess molar heat capacity (abbreviated Cp [kcal/mol/°C]; Y axis) vs. temperature (X axis). (FIG. 2A) MNAC13 antibody (FAB fragment Tm is Tm1 at 74°C). (FIG. 2B) BXhVH5VL1 antibody (FAB fragment Tm is Tm3 at 76.5°C). (FIG. 2C) GBR VH5(V37A)VL1 antibody (FAB fragment Tm is Tm1 at 73.6°C). (FIG. 2D) GBR VH5(K3Q,V37A)VL1 antibody (FAB fragment Tm is Tm1 at 73°C). (FIG. 2E) overlay of FIG. 2C and FIG. 2D. (FIG. 2F) overlay of FIG. 2B and FIG. 2D. (FIG. 2G) overlay of FIG. 2A and FIG. 2D.

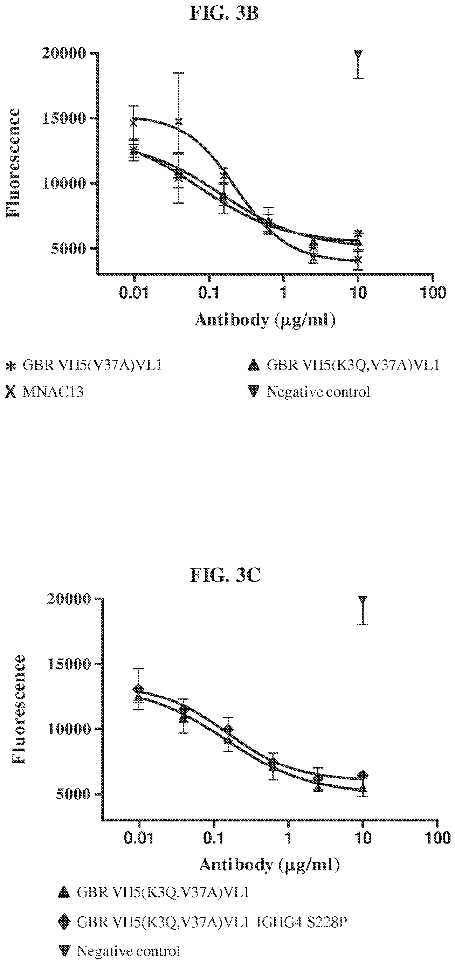

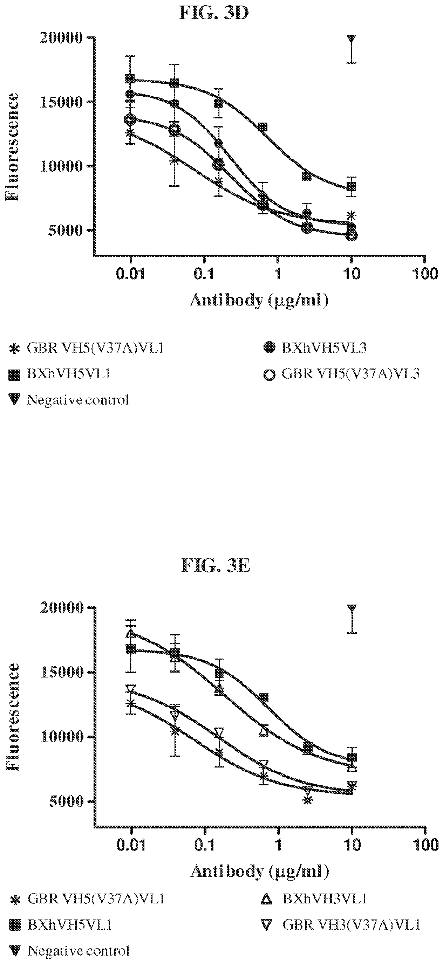

FIG. 3: Functional bioactivity of anti-TrkA antibodies. Effect of humanized anti-TrkA antibodies on the NGF-induced TF-1 cell proliferation; data are expressed as % of proliferative response (Y axis) vs. antibody concentration (µg/ml; X axis). (FIG. 3A) GBR VH5(V37A)VL1, GBR VH5(K3Q,V37A)VL1 vs. BXhVH5VL1. (FIG. 3B) GBR VH5(V37A)VL1, GBR VH5(K3Q,V37A)VL1 vs. MNAC13. (FIG. 3C) GBR VH5(K3Q,V37A)VL1 vs. GBRVH5(K3Q,V37A)VL1 IGHG4 S228P. (FIG. 3D) BXhVH5VL1, BXhVH5VL3, GBR VH5(V37A)VL1 vs. GBR VH5(V37A)VL3. (FIG. 3E) BXhVH5VL1, BXhVH3VL1, GBRVH5(V37A)VL1 vs. GBR VH3(V37A)VL1.

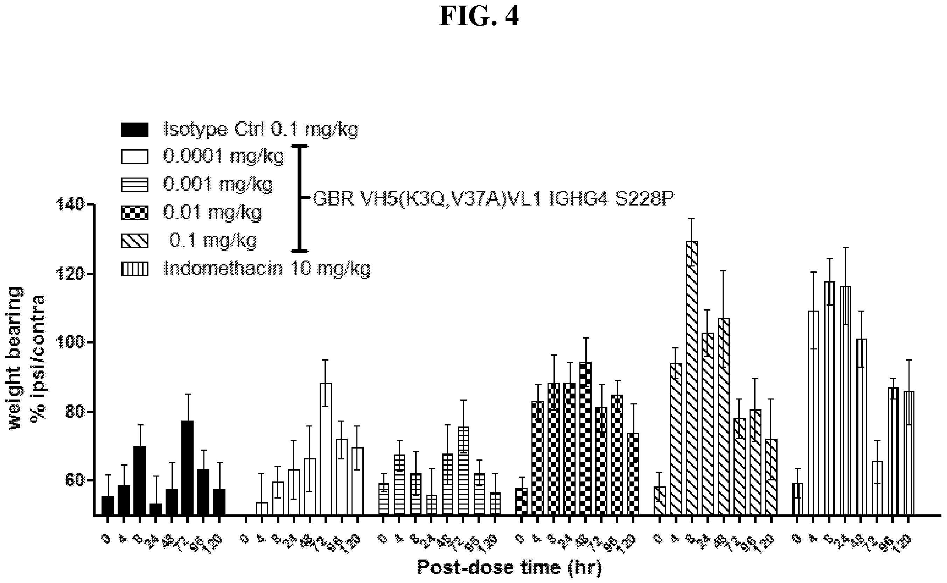

FIG. 4: Humanized anti-TrkA antibody reverses acute inflammatory paw pain. Acute inflammatory hyperalgesia of the paw was induced by intraplantar injection of CFA into one of the hind limb paws of AMB1 mice and measured as the % ratio between weight bearing on the ipsilateral (injected) and contralateral (non-injected) paw (% ipsi/contra, mean ± s.e.m.). Weight bearing readings were taken 23hrs post-CFA injection (0 hr) before treatment initiation at 24hr post-CFA with a single i.p. injection of 0.0001 (white bars), 0.001 (horizontally-hatched bars), 0.01 (chequered bars) and 0.1 mg/kg (diagonally hatched bars) anti-TrkA antibody or 0.1 mg/kg isotype control antibody (black bars) followed by weight bearing measurements at 4, 8, 24, 48, 72, 96 and 120 hrs post-dose. As a positive control, mice were treated with 10mg/kg indomethacin p.o. (vertically-hatched bars).

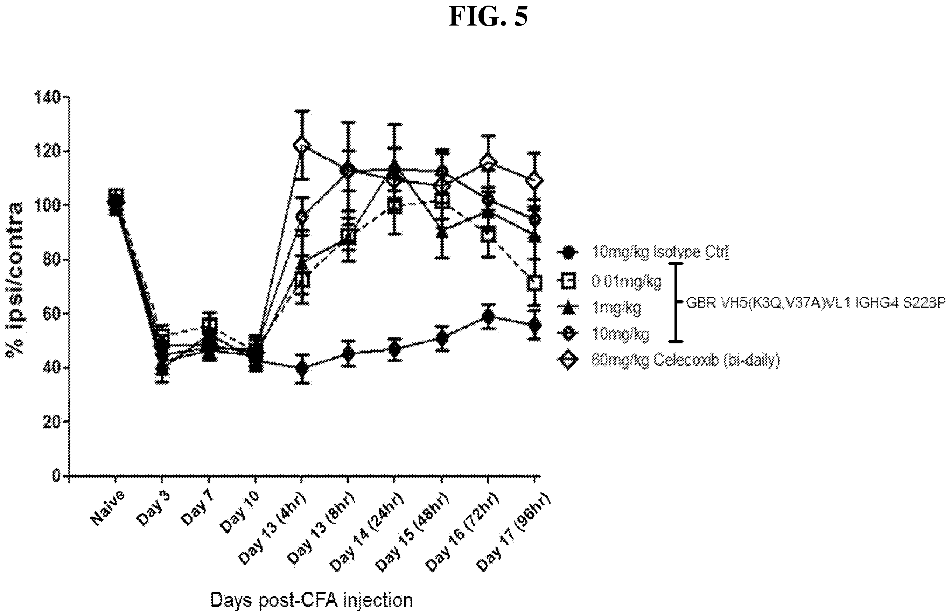

FIG. 5: Humanized anti-TrkA antibody reverses chronic inflammatory joint pain. Chronic inflammatory hyperalgesia of the joint was induced by intra-articular injection of CFA into one of the hind limb knee joints of AMB1 mice and measured as the % ratio between weight bearing on the ipsilateral (injected) and contralateral (non-injected) limb (% ipsi/contra, mean ± s.e.m.). Weight bearing readings were taken immediately prior to CFA injection (naive) and on day 3, 7 and 10 post-CFA before treatment initiation on day 13 post-CFA with a single i.p. injection of 0.01 (open square, dashed line), 1 (closed triangle) and 10mg/kg (open circle) anti-TrkA antibody or 10 mg/kg isotype control antibody (closed circle) followed by weight bearing measurements at 4, 8, 24, 48, 72 and 96 hrs post-dose (in parentheses). As a positive control, mice were treated with 60mg/kg celecoxib twice daily (open diamond) from day 13 onwards and weight bearing was measured at 1 and 8 hrs post-dosing on day 13 post-CFA and then at 1 hr post-dosing on days 14-17 post-CFA.

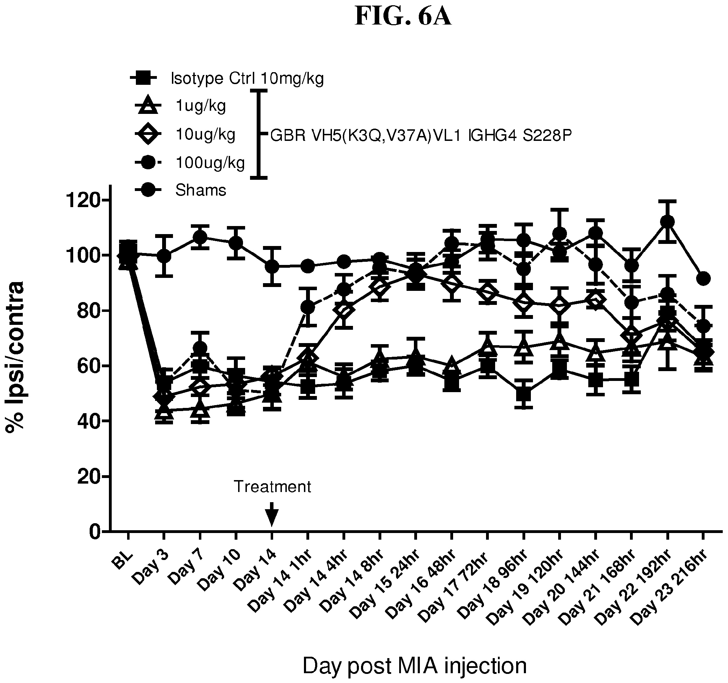

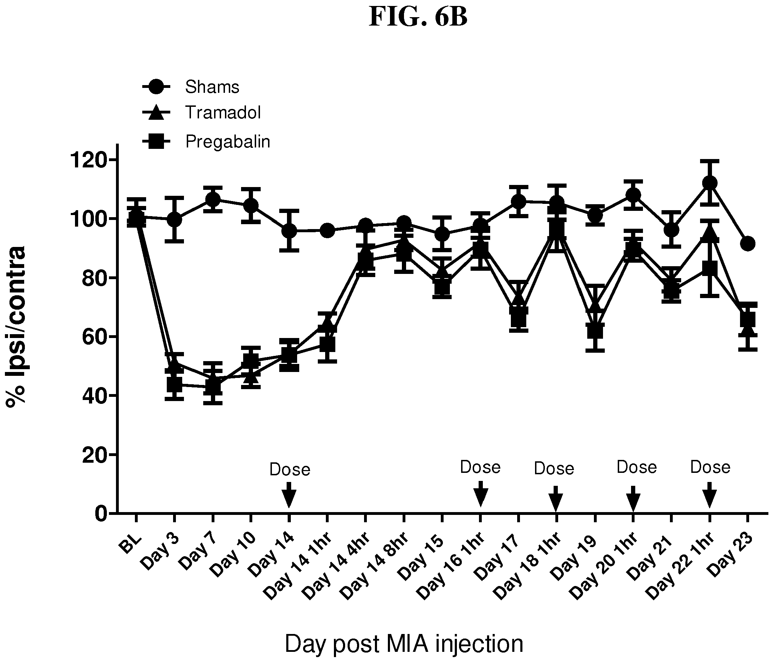

FIG. 6: Humanized anti-TrkA antibody reverses chronic osteoarthritic pain. Chronic osteoarthritic hyperalgesia was induced by intra-articular injection of MIA into one of the hind limb knee joints of AMB1 mice and measured as the % ratio between weight bearing on the ipsilateral (injected) and contralateral (non-injected) limb (% ipsi/contra, mean ± s.e.m.). Baseline (BL) weight bearing readings were taken immediately prior to MIA injection and on day 3, 7 and 10 days post-MIA before treatment initiation on day 14 post-MIA with a single i.p. injection of 1 (open triangle), 10 (open diamond) and 100µg/kg (closed circle, dashed line) anti-TrkA antibody or 10 mg/kg isotype control antibody (closed square) (FIG. 6A). As a comparator control, animals were treated on day 14 post-MIA with tramadol at 10mg/kg or pregabalin at 30mg/kg p.o. followed by tramadol at 30mg/kg or pregabalin at 100mg/kg every other day on days 16-22 post-MIA (FIG. 6B). All animals were assessed using weight bearing at 4, 8, and 24 hours post-dosing on day 14 post-MIA followed by every 24 hours for antibody treated groups and 1 and 24 hours post-dose for the tramadol and pregabalin treated groups.

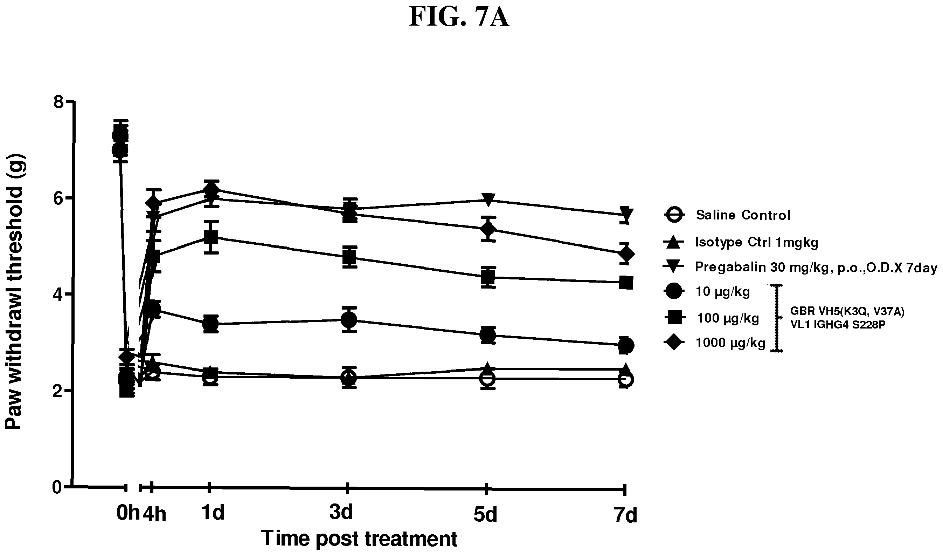

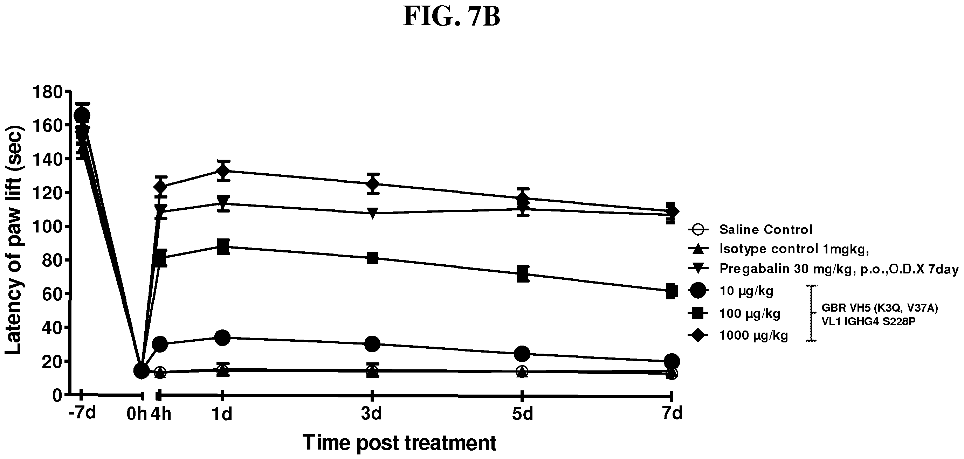

FIG. 7: Humanized anti-TrkA antibody reverses neuropathic pain. Chronic neuropathic hyperalgesia and allodynia were induced by chronic constriction injury of the sciatic nerve of AMB1 mice and measured as the threshold force required for paw withdrawal (g) and the latency time for paw withdrawal from a cold plate (sec), respectively. Readings were taken prior to surgery and 7 days after surgery on the day before treatment initiation (0h). Animals were then treated with a single i.p. injection of either 1000µg/kg isotype control antibody (closed triangle) or 10 (closed circle), 100 (closed square) and 1000µg/kg (closed diamond) anti-TrkA antibody. Pregabalin (closed inverted triangle) at 30 mg/kg/10ml, p.o. or saline (open circle) were administered once daily over the seven day post-dose period. Post-dose readouts were recorded at 4h, 24h and then every other day until the 7th day post-dose. Post-dose readouts were recorded 1h after pregabalin or saline dosing on all days.

### Detailed description of the invention

The present disclosure relates to humanized anti-TrkA antibodies or fragment thereof, methods for their preparation and use.

The term "TrkA", "human TrkA", "TrkA receptor" or "human TrkA receptor" are used herein equivalently and mean "human TrkA" if not otherwise specifically indicated. Human TrkA as used herein include variants, isoforms, and species homologs of human TrkA. Accordingly, antibodies of this disclosure may, in certain cases, cross-react with TrkA from species other than human. In certain embodiments, the antibodies may be completely specific for one or more human TrkA proteins and may not exhibit species or other types of non-human cross-reactivity.

TrkA is also known as high affinity nerve growth factor receptor or neurotrophic tyrosine kinase receptor type 1or TRK1-transforming tyrosine kinase protein or Tropomyosin-related kinase A or Tyrosine kinase receptor or Tyrosine kinase receptor A or Trk-A or gp140trk or p140-TrkA or MTC or TRK. TrkA is a receptor tyrosine kinase involved in the development and the maturation of the central and peripheral nervous systems through regulation of proliferation, differentiation and survival of sympathetic and nervous neurons. TrkA is the high affinity receptor for NGF which is its primary ligand; it can also bind and be activated by NTF3/neurotrophin-3.

The complete amino acid sequence of the four known human TrkA isoforms are found under the UniProt/Swiss-Prot accession number P04629 (Consortium TU, (2012) Nucleic Acids Res. 40(D1):D71-D5). The four isoforms are produced by alternative splicing: isoform TrkA-I is found in most non-neuronal tissues (UniProt/Swiss-Prot accession number P04629-2), while isoform TrkA-II is primarily expressed in neuronal cells (UniProt/Swiss-Prot accession number P04629-1), and isoform TrkA-III is specifically expressed by pluripotent neural stem and neural crest progenitors (UniProt/Swiss-Prot accession number P04629-4). A fourth isoform which differs from isoform TrkA-II at residues 1-71 and lacks residues 393 to 398 is known as isoform 3 (UniProt/Swiss-Prot accession number P04629-3). TrkA-II isoform is the major known isoform of TrkA. Isoform TrkA-I has enhanced responsiveness to NTF3 neurotrophin whereas isoform TrkA-III is constitutively active and does not bind NGF. In a preferred embodiment, the TrkA isoform as used herein is the TrkA-II isoform with SEQ ID NO: 72 and an extracellular region thereof comprising the sequence of SEQ ID NO: 25.

The term "anti-TrkA antibody or fragment thereof" or "humanised anti-TrkA antibody or fragment thereof " as used herein includes antibodies or a fragment thereof that bind to human TrkA e.g. human TrkA in isolated form, specifically antibodies or fragment thereof that bind to the TrkA-II isoform (SEQ ID NO: 72), more specifically antibodies or fragment thereof that binds to a monovalent form of the human TrkA extracellular region (SEQ ID NO: 25) of the TrkA-II isoform, with an affinity (KD) of 500 nM or less, preferably 350 nM or less, more preferably 150 nM or less, even more preferably 100 nM or less, most preferred 50 nM or less, in particular 30 nM or less. Usually the humanized anti-TrkA antibody or fragment thereof is capable of inhibiting the functional activation of TrkA and/or is capable of blocking or reducing one or more biological activities that would otherwise be induced by the binding of NGF to TrkA.

As used herein, an "anti-NGF antibody" refers to an antibody which is able to bind to NGF, preferably human NGF. Usually the anti-NGF antibody is capable of inhibiting the functional activation of TrkA and/or is capable of blocking or reducing one or more biological activities of TrkA. The binding affinity of an anti-NGF antibody to NGF (such as hNGF) can be 500 nM or less, preferably 100 nM or less. Usually the anti-NGF antibody should exhibit any one or more of the following characteristics: (a) bind to NGF and inhibit NGF biological activity and/or downstream pathways mediated by NGF signaling function; (b) block or decrease NGF receptor activation (including TrkA receptor dimerization and/or autophosphorylation); (c) increase clearance of NGF; (d) inhibit (reduce) NGF synthesis, production or release. Anti-NGF antibodies are known in the art, see, e.g., PCT Publication Nos. WO 01/78698, WO 01/64247, US Patent Nos. 5,844,092, 5,877,016 and 6,153,189; Hongo et al., (2000) Hybridoma, 19:215-227; GenBank Accession Nos. U39608, U39609, L17078 or L17077.

The term "humanized anti-TrkA antibody or fragment thereof capable of inhibiting the functional activation of TrkA" as used herein refers to humanized anti-TrkA antibodies that exhibit any one or more of the following characteristics: (a) bind to TrkA and inhibit TrkA biological activities and/or downstream pathways mediated by the binding of NGF or NTF3/neurotrophin-3 signaling function; (b) prevent, ameliorate, or treat any aspect of pain; (c) block or decrease TrkA activation, or dimerization and/or autophosphorylation; (d) increase TrkA clearance; (e) inhibit or reduce TrkA synthesis and/or cell surface expression.

The term "humanized anti-TrkA antibody or fragment thereof capable of blocking or reducing one or more biological activities of TrkA" as used herein refers to humanized anti-TrkA antibodies which directly or indirectly reduce, inhibit, neutralize, or abolish TrkA biological activities.

The term "biological activities of TrkA" or "TrkA biological activities" as used herein refers without limitation to any one or more of the following: the ability to bind NGF or other neurotrophins; the ability to homo-dimerize, or hetero-dimerize and/or autophosphorylate; the ability to activate an NGF induced signalling pathway; the ability to promote cell differentiation, proliferation, survival, growth, migration and other changes in cell physiology, including (in the case of neurons, including peripheral and central neuron) change in neuronal morphology, synaptogenesis, synaptic function, neurotransmitter and/or neuropeptide release and regeneration following damage; and the ability to mediate pain and cancer pain associated with bone metastasis.

The term "IC50" as used herein describes the half maximal inhibitory concentration (IC50) which is a measure of the effectiveness of a compound in inhibiting biological function, e.g. inhibition of humanized anti-TrkA antibodies on the proliferation of NGF-induced TF-1 cells.

The term "antibody" as referred to herein includes full-length antibodies and any antigen binding fragment or single chains thereof. Antibodies and specifically naturally occurring antibodies are glycoproteins which exist as one or more copies of a Y-shaped unit, composed of four polypeptide chains. Each "Y" shape contains two identical copies of a heavy (H) chain, and two identical copies of a light (L) chain, named as such by their relative molecular weights. Each light chain pairs with a heavy chain, and each heavy chain pairs with another heavy chain. Covalent interchain disulfide bonds and non covalent interactions link the chains together. Antibodies and specifically naturally occurring antibodies contain variable regions, which are the two copies of the antigen binding site. Papain, a proteolytic enzyme splits the "Y" shape into three separate molecules, two so called "Fab" fragments (Fab = fragment antigen binding), and one so called "Fc" fragment or "Fc region" (Fc = fragment crystallizable). A Fab fragment consists of the entire light chain and part of the heavy chain. The heavy chain contains one variable domain (heavy chain variable domain or VH) and either three or four constant domains (CH1, CH2, CH3 and CH4, depending on the antibody class or isotype). The region between the CH1 and CH2 domains is called the hinge region and permits flexibility between the two Fab arms of the Y-shaped antibody molecule, allowing them to open and close to accommodate binding to two antigenic determinants separated by a fixed distance. The heavy chains of IgA, IgD and IgG each have four domains, i.e. one variable domain (VH) and three constant domains (CH1-3). IgE and IgM have one variable and four constant domains (CH1-4) on the heavy chain. The constant regions of the antibodies may mediate the binding to host tissues or factors, including various cells of the immune system (e.g., effector cells) and the first component (C1q) of the complement system classical pathway. Each light chain is usually linked to a heavy chain by one covalent disulfide bond. Each light chain contains one variable domain (light chain variable domain or VL) and one light chain constant domain. The light chain constant domain is a kappa light chain constant domain designated herein as IGKC or is a lambda light chain constant domain designated herein as IGLC. IGKC is used herein equivalently to Cκ or CK and has the same meaning. IGLC is used herein equivalently to Cλ or CL and has the same meaning. The term "an IGLC domain" as used herein refers to all lambda light chain constant domains e.g. to all lambda light chain constant domains selected from the group consisting of IGLC1, IGLC2, IGLC3, IGLC6 and IGLC7. The VH and VL regions can be further subdivided into regions of hypervariability, termed complementarity determining regions (CDR), interspersed with regions that are more conserved, termed framework regions (FR or FW or "non-CDR regions"). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

Antibodies are grouped into classes, also referred to as isotypes, as determined genetically by the constant region. Human constant light chains are classified as kappa (CK) and lambda (Cλ) light chains. Heavy chains are classified as mu (µ), delta (δ), gamma (γ), alpha (α), or epsilon (ε), and define the antibody's isotype as IgM, IgD, IgG, IgA, and IgE, respectively. Thus, "isotype" as used herein is meant any of the classes and/or subclasses of immunoglobulins defined by the chemical and antigenic characteristics of their constant regions. The known human immunoglobulin isotypes are IgG1 (IGHG1), IgG2 (IGHG2), IgG3 (IGHG3), IgG4 (IGHG4), IgA1 (IGHA1), IgA2 (IGHA2), IgM (IGHM), IgD (IGHD), and IgE (IGHE). The so-called human immunoglobulin pseudo-gamma IGHGP gene represents an additional human immunoglobulin heavy constant region gene which has been sequenced but does not encode a protein due to an altered switch region (Bensmana M et al., Nucleic Acids Res. 16(7):3108). In spite of having an altered switch region, the human immunoglobulin pseudo-gamma IGHGP gene has open reading frames for all heavy constant domains (CH1-CH3) and hinge. All open reading frames for its heavy constant domains encode protein domains which align well with all human immunoglobulin constant domains with the predicted structural features. This additional pseudo-gamma isotype is referred herein as IgGP or IGHGP. Other pseudo immunoglobulin genes have been reported such as the human immunoglobulin heavy constant domain epsilon P1 and P2 pseudo-genes (IGHEP1 and IGHEP2). The IgG class is the most commonly used for therapeutic purposes. In humans this class comprises subclasses IgG1, IgG2, IgG3 and IgG4. In mice this class comprises subclasses IgG1, IgG2a, IgG2b, IgG2c and IgG3.

The term "chimeric antibody" or "chimeric anti-TrkA antibody" as used herein includes antibodies in which the variable region sequences are derived from one species and the constant region sequences are derived from another species, such as an antibody in which the variable region sequences are derived from a mouse antibody and the constant region sequences are derived from a human antibody.

The term "humanized antibody" or "humanised anti-TrkA antibody" as used herein includes antibodies in which CDR sequences derived from the germline of another mammalian species, such as a mouse, have been grafted onto human framework sequences. Additional framework region modifications may be made within the human framework sequences as well as within the CDR sequences derived from the germline of another mammalian species.

The term "Fab" or "Fab region" as used herein includes the polypeptides that comprise the VH, CH1, VL and CL immunoglobulin domains. Fab may refer to this region in isolation or this region in the context of a full length antibody or antibody fragment.

The term "Fc" or "Fc region" as used herein includes the polypeptide comprising the constant region of an antibody excluding the first constant region immunoglobulin domain. Thus Fc refers to the last two constant region immunoglobulin domains of IgA, IgD and IgG, and the last three constant region immunoglobulin domains of IgE and IgM, and the flexible hinge N-terminal to these domains. For IgA and IgM, Fc may include the J chain. For IgG, Fc comprises immunoglobulin domains Cγ2 and Cγ3 and the hinge between Cγ1 and Cγ2. Although the boundaries of the Fc region may vary, the human IgG heavy chain Fc region is usually defined to comprise residues C226 or P230 to its carboxyl-terminus, wherein the numbering is according to the EU numbering system (Edelman GM et al., (1969) PNAS USA 63(1): 78-85). For human IgG1 the Fc region is herein defined to comprise residue P232 to its carboxyl-terminus, wherein the numbering is according to the EU numbering system. Fc may refer to this region in isolation or this region in the context of an Fc polypeptide, for example an antibody.

The term "hinge" or "hinge region" or "antibody hinge region" herein includes the flexible polypeptide comprising the amino acids between the first and second constant domains of an antibody. The "hinge region" as referred to herein is a sequence region of 6-62 amino acids in length, only present in IgA, IgD and IgG, which encompasses the cysteine residues that bridge the two heavy chains. Structurally, the IgG CH1 domain ends at EU position 220, and the IgG CH2 domain begins at residue EU position 237. Thus for IgG the antibody hinge is herein defined to include positions 221 (D221 in IgG1) to 231 (A231 in IgG1), wherein the numbering is according to the EU numbering system.

The terms "parent antibody", "parent immunoglobulin", "parental antibody" or "parental immunoglobulin", which are used equivalently herein include an unmodified antibody that is subsequently modified to generate a variant. Said parent antibody may be a naturally occurring antibody or a variant or engineered version of a naturally occurring antibody. Parent antibody may refer to the antibody itself, compositions that comprise the parent antibody or the amino acid sequence that encodes it. By "parental murine antibody" or "corresponding parental murine antibody" as used herein is meant an antibody or immunoglobulin that binds human TrkA and is modified to generate a variant, specifically the murine antibody MNAC13 as disclosed in WO00/73344.

The term "variant antibody" or "antibody variant" as used herein includes an antibody sequence that differs from that of a parent antibody sequence by virtue of at least one amino acid modification compared to the parent. The variant antibody sequence herein will preferably possess at least about 80%, most preferably at least about 90%, more preferably at least about 95% amino acid sequence identity with a parent antibody sequence. Antibody variant may refer to the antibody itself, compositions comprising the antibody variant or the amino acid sequence that encodes it.

The term "amino acid modification" herein includes an amino acid substitution, insertion, and/or deletion in a polypeptide sequence. By "amino acid substitution" or "substitution" herein is meant the replacement of an amino acid at a particular position in a parent polypeptide sequence with another amino acid. For example, the substitution R94K refers to a variant polypeptide, in this case a heavy chain variable framework region variant, in which the arginine at position 94 is replaced with a lysine. For the preceding example, 94K indicates the substitution of position 94 with a lysine. For the purposes herein, multiple substitutions are typically separated by a slash. For example, R94K/L78V refers to a double variant comprising the substitutions R94K and L78V. By "amino acid insertion" or "insertion" as used herein is meant the addition of an amino acid at a particular position in a parent polypeptide sequence. For example, insert -94 designates an insertion at position 94. By "amino acid deletion" or "deletion" as used herein is meant the removal of an amino acid at a particular position in a parent polypeptide sequence. For example, R94- designates the deletion of arginine at position 94.

As used herein, the term "conservative modifications" or "conservative sequence modifications" is intended to refer to amino acid modifications that do not significantly affect or alter the binding characteristics of the antibody containing the amino acid sequence. Such conservative modifications include amino acid substitutions, insertions and deletions. Modifications can be introduced into an antibody of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions or within the framework regions of an antibody of the invention can be replaced with other amino acid residues from the same side chain family and the altered antibody (variant antibody) can be tested for retained function.

For all human immunoglobulin heavy chain constant domains numbering is according to the "EU numbering system" (Edelman GM *et al*., *ibid.).* For the human kappa immunoglobulin light chain constant domain (IGKC), numbering is according to the "EU numbering system" (Edelman GM *et al*., *ibid.).* For the human lambda immunoglobulin light chain constant domains (IGLC1, IGLC2, IGLC3, IGLC6, and IGLC7), numbering is according to the "Kabat numbering system" (Kabat EA et al., (1991) Sequences of proteins of immunological interest. 5th Edition - US Department of Health and Human Services, NIH publication n° 91-3242) as described by Dariavach P et al., (1987) PNAS USA 84(24):9074-8 and Frangione B et al., (1985) PNAS USA 82(10):3415-9.

The term "variable domain" refers to the domains that mediates antigen-binding and defines specificity of a particular antibody for a particular antigen. In naturally occurring antibodies, the antigen-binding site consists of two variable domains that define specificity: one located in the heavy chain, referred herein as heavy chain variable domain (VH) and the other located in the light chain, referred herein as light chain variable domain (VL). In some cases, specificity may exclusively reside in only one of the two domains as in single-domain antibodies from heavy-chain antibodies found in camelids. The V regions are usually about 110 amino acids long, and consist of relatively invariant stretches of amino acid sequence called framework regions (FRs or "non-CDR regions) of 15-30 amino acids separated by shorter regions of extreme variability called "hypervariable regions" that are 7-17 amino acids long. The variable domains of native heavy and light chains comprise four FRs, largely adopting a beta-sheet configuration, connected by three hypervariable regions, which form loops. The hypervariable regions in each chain are held together in close proximity by FRs and, with the hypervariable regions from the other chain, contribute to the formation of the antigen binding site of antibodies (see Kabat EA *et al*., *ibid.).* The term "hypervariable region" as used herein refers to the amino acid residues of an antibody which are responsible for antigen binding. The hypervariable region generally comprises amino acid residues from a "complementary determining region" or "CDR", the latter being of highest sequence variability and/or involved in antigen recognition. For all variable domains numbering is according to Kabat (Kabat EA *et al*., *ibid.).*

A number of CDR definitions are in use and are encompassed herein. The Kabat definition is based on sequence variability and is the most commonly used (Kabat EA *et al*., *ibid.).* Chothia refers instead to the location of the structural loops (Chothia & Lesk J. (1987) Mol. Biol. 196:901-917). The AbM definition is a compromise between the Kabat and the Chothia definitions and is used by Oxford Molecular's AbM antibody modelling software (Martin ACR et al., (1989) PNAS USA 86:9268-9272; Martin ACR et al., (1991) Methods Enzymol. 203:121-153; Pedersen JT et al., (1992) Immunomethods 1:126-136; Rees AR et al., (1996) In Sternberg M.J.E. (ed.), Protein Structure Prediction. Oxford University Press, Oxford, 141-172). The contact definition has been recently introduced (MacCallum RM et al., (1996) J. Mol. Biol. 262:732-745) and is based on an analysis of the available complex structures available in the Protein Databank. The definition of the CDR by IMGT®, the international ImMunoGeneTics information system® (http://www.imgt.org) is based on the IMGT numbering for all immunoglobulin and T cell receptor V-REGIONs of all species (IMGT®, the international ImMunoGeneTics information system®; Lefranc MP et al., (1999) Nucleic Acids Res. 27(1):209-12; Ruiz M et al., (2000) Nucleic Acids Res. 28(1):219-21; Lefranc MP (2001) Nucleic Acids Res. 29(1):207-9; Lefranc MP (2003) Nucleic Acids Res. 31(1):307-10; Lefranc MP et al., (2005) Dev. Comp. Immunol. 29(3):185-203; Kaas Q et al., (2007) Briefings in Functional Genomics & Proteomics, 6(4):253-64).

All Complementarity Determining Regions (CDRs) as referred to in the present invention, are defined preferably as follows (numbering according to Kabat EA *et al*., *ibid.):* LCDR1: 24-34; LCDR2: 50-56; LCDR3: 89-98; HCDR1: 26-35; HCDR2: 50-65; HCDR3: 95-102. The "non-CDR regions" of the variable domain are known as framework regions (FR). The "non-CDR regions" of the VL region as used herein comprise the amino acid sequences: 1-23 (FR 1), 35-49 (FR2), 57-88 (FR3), and 99-107 (FR4). The "non-CDR regions" of the VH region as used herein comprise the amino acid sequences: 1-25 (FR1), 36-49 (FR2), 66-94 (FR3), and 103-113 (FR4).

The term "full length antibody" as used herein includes the structure that constitutes the natural biological form of an antibody, including variable and constant regions. For example, in most mammals, including humans and mice, the full length antibody of the IgG class is a tetramer and consists of two identical pairs of two immunoglobulin chains, each pair having one light and one heavy chain, each light chain comprising immunoglobulin domains VL and CL and each heavy chain comprising immunoglobulin domains VH, CH2 (Cγ1), CH2 (Cγ2), and CH3 (Cγ3). In some mammals, for example in camels and llamas, IgG antibodies may consist of only two heavy chains, each heavy chain comprising a variable domain attached to the Fc region.

Antibody fragments as used herein refer to antigen-binding fragments include, but are not limited to, (i) the Fab fragment consisting of VL, VH, CL and CH1 domains, including Fab' and Fab'-SH, (ii) the Fd fragment consisting of the VH and CH1 domains, (iii) the Fv fragment consisting of the VL and VH domains of a single antibody; (iv) the dAb fragment (Ward et al. (1989) Nature 341: 544-546) which consists of a single variable domain, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird et al. (1988) Science 242: 423-426; Huston et al. (1988) PNAS USA 85: 5879-5883), (vii) bispecific single chain Fv dimers (PCT/US92/09965), (viii) "diabodies" or "triabodies", multivalent or multispecific fragments constructed by gene fusion (Tomlinson I et al., (2000) Methods Enzymol. 326:461-479; WO94/13804; Holliger et al., (1993) PNAS USA 90:6444-6448) and (ix) scFv genetically fused to the same or a different antibody (Coloma & Morrison (1997) Nature Biotech. 15:159-163).

The term "effector function" as used herein includes a biochemical event that results from the interaction of an antibody Fc region with an Fc receptor or ligand. Effector functions include FcγR-mediated effector functions such as ADCC (antibody dependent cell-mediated cytotoxicity) and ADCP (antibody dependent cell-mediated phagocytosis), and complement-mediated effector functions such as CDC (complement dependent cytotoxicity). An effector function of an antibody may be altered by altering, i.e. enhancing or reducing, preferably enhancing, the affinity of the antibody for an effector molecule such as an Fc receptor or a complement component. Binding affinity will generally be varied by modifying the effector molecule binding site and in this case it is appropriate to locate the site of interest and modify at least part of the site in a suitable way. It is also envisaged that an alteration in the binding site on the antibody for the effector molecule need not alter significantly the overall binding affinity but may alter the geometry of the interaction rendering the effector mechanism ineffective as in non-productive binding. It is further envisaged that an effector function may also be altered by modifying a site not directly involved in effector molecule binding, but otherwise involved in performance of the effector function. By altering an effector function of an antibody it may be possible to control various aspects of the immune response, eg enhancing or suppressing various reactions of the immune system, with possible beneficial effects in diagnosis and therapy.

As used herein, the term "subject" includes any human or non-human animal. The term "non-human animal" includes all vertebrates, e.g., mammals and non-mammals, such as primates, sheep, dogs, cats, horses, cows, chickens, amphibians, reptiles, etc. Preferably the subject is human.

### Antibodies of the invention

In a first aspect the present invention provides a humanized anti-TrkA antibody or fragment thereof comprising:
a) a heavy chain variable domain comprising a sequence selected from the group consisting of SEQ ID NOs: 1-5, and
b) a light chain variable domain comprising a sequence selected from the group consisting of SEQ ID NOs: 6-13,
wherein CDR2 of the heavy chain variable domain comprises at least one amino acid substitution and/or wherein the non-CDR region of the heavy chain variable domain comprises an amino acid substitution at an amino acid position selected from the group consisting of 37, 42 and 89, wherein the amino acid position of each group member is indicated utilizing the numbering system set forth in Kabat.

In some embodiments, the CDR2 of the heavy chain variable domain comprises at least one conservative amino acid substitution.

In some embodiments, the non-CDR region of the heavy chain variable domain comprises a conservative amino acid substitution at an amino acid position selected from the group consisting of 37, 42 and 89.

In some embodiments, the CDR2 of the heavy chain variable domain comprises the sequence of SEQ ID NO: 15.

In some embodiments, the heavy chain variable domain comprises a sequence selected from the group consisting of SEQ ID NOs: 1, 3 and 5, and the light chain variable domain comprises a sequence selected from the group consisting of SEQ ID NOs: 6 and 8.

In some embodiments, the humanized anti-TrkA antibody or fragment thereof comprises a combination of a heavy chain variable domain and a light chain variable domain comprising the sequences selected from the group consisting of SEQ ID NO: 1 and SEQ ID NO: 6, SEQ ID NO: 3 and SEQ ID NO: 6, SEQ ID NO: 3 and SEQ ID NO: 8, SEQ ID NO: 5 and SEQ ID NO: 6, and SEQ ID NO: 5 and SEQ ID NO: 8; preferably the sequences of SEQ ID NO: 5 and SEQ ID NO: 6 or the sequences of SEQ ID NO: 5 and SEQ ID NO: 8; more preferably the sequences of SEQ ID NO: 5 and SEQ ID NO: 6.

In some embodiments, the heavy chain variable domain of the humanized anti-TrkA antibody or fragment thereof provided by the present disclosure does not comprise the sequence of SEQ ID NO: 71.

In some embodiments, the heavy chain variable domain of the humanized anti-TrkA antibody or fragment thereof provided by the present disclosure lacks a serine at position 87, wherein the amino acid position of each group member is indicated utilizing the numbering system set forth in Kabat.

In some embodiments, the heavy chain variable domain of the humanized anti-TrkA antibody or fragment thereof provided by the present disclosure comprises a threonine at position 87, wherein the amino acid position of each group member is indicated utilizing the numbering system set forth in Kabat.

In some embodiments, the amino acid substitution of the CDR2 of the heavy chain variable domain comprises an amino acid substitution at an amino acid position selected from the group consisting of 50, 60 and 62, preferably selected from the group consisting of 60 and 62, wherein the amino acid position of each group member is indicated utilizing the numbering system set forth in Kabat.

In some embodiments, the amino acid substitution of the CDR2 of the heavy chain variable domain does not comprise an amino acid substitution selected from the group consisting of Y50A, P60A and T62S, wherein the amino acid position of each group member is indicated utilizing the numbering system set forth in Kabat.

In some embodiments, if the amino acid substitution of the humanized anti-TrkA antibody or fragment in the non-CDR region of the heavy chain variable domain is A49S, the amino acid substitution of the humanized anti-TrkA antibody or fragment in the CDR2 of the heavy chain variable domain is not Y50A.

In some embodiments, the amino acid substitution of the humanized anti-TrkA antibody or fragment in the non-CDR region of the heavy chain variable domain is not A49S and/or the amino acid substitution of the humanized anti-TrkA antibody or fragment in the CDR2 of the heavy chain variable domain is not Y50A.

In some embodiments, the amino acid substitution of the non-CDR region of the heavy chain variable domain of the antibody or the fragment thereof comprises an amino acid substitution selected from the group consisting of V37A, G42E and V89L, preferably V37A, wherein the amino acid position is indicated utilizing the numbering system set forth in Kabat.

In some embodiments, the antibody comprises a heavy chain variable domain comprising the sequence of SEQ ID NO: 3, wherein the amino acid substitution of the non-CDR region of the heavy chain variable domain comprises an amino acid substitution selected from the group consisting of V37A, T40A, G42E, R44G, A49S and V89L, preferably selected from the group consisting of V37A, T40A, G42E, R44G and V89L wherein the amino acid position of each group member is indicated utilizing the numbering system set forth in Kabat.

In some embodiments, the antibody comprises a combination of a heavy chain variable domain and a light chain variable domain comprising the sequences of SEQ ID NO: 3 and SEQ ID NO: 6 or comprising the sequences of SEQ ID NO: 3 and SEQ ID NO: 8, wherein the amino acid substitution of the non-CDR region of the heavy chain variable domain comprises an amino acid substitution selected from the group consisting of V37A, T40A, G42E, R44G, A49S and V89L, preferably selected from the group consisting of V37A, T40A, G42E, R44G and V89L, wherein the amino acid position of each group member is indicated utilizing the numbering system set forth in Kabat.

In some embodiments, the amino acid substitution of the non-CDR region of the heavy chain variable domain comprises an amino acid substitution selected from the group consisting of K3Q, V37A, G42E, A49S, V89L and R94K, preferably selected from the group consisting of K3Q, V37A, G42E, V89L and R94K, more preferably comprises the amino acid substitutions K3Q and V37A and most preferably comprises the amino acid substitution V37A, wherein the amino acid position of each group member is indicated utilizing the numbering system set forth in Kabat. Equally most preferred are embodiments wherein the amino acid substitution of the non-CDR region of the heavy chain variable domain comprises an amino acid substitution selected from the group consisting of V37A and K3Q, V37A and wherein the amino acid position of each group member is indicated utilizing the numbering system set forth in Kabat.

In some embodiments, the antibody comprises a heavy chain variable domain comprising the sequence of SEQ ID NO: 5, wherein the amino acid substitution of the non-CDR region of the heavy chain variable domain comprises an amino acid substitution selected from the group consisting of K3Q, V37A, G42E, A49S, V89L and R94K, preferably selected from the group consisting of K3Q, V37A, G42E, V89L and R94K, more preferably comprises the amino acid substitutions K3Q and V37A and most preferably comprises the amino acid substitution V37A, wherein the amino acid position of each group member is indicated utilizing the numbering system set forth in Kabat. Equally most preferred are embodiments wherein the antibody comprises a heavy chain variable domain comprising the sequence of SEQ ID NO: 5, wherein the amino acid substitution of the non-CDR region of the heavy chain variable domain comprises an amino acid substitution selected from the group consisting of V37A and K3Q, V37A and wherein the amino acid position of each group member is indicated utilizing the numbering system set forth in Kabat.

In a further aspect the present invention provides a humanized anti-TrkA antibody or fragment thereof comprising:
a) a heavy chain variable domain comprising a sequence selected from the group consisting of SEQ ID NOs: 31-49, and
b) a light chain variable domain comprising a sequence selected from the group consisting of SEQ ID NOs: 6-13.

In some embodiments the humanized anti-TrkA antibody or fragment thereof comprises:
a) a heavy chain variable domain comprising a sequence selected from the group consisting of SEQ ID NOs: 32, 36, 39, 43, 48 and 49, and
b) a light chain variable domain comprising a sequence selected from the group consisting of SEQ ID NOs: 6-13 or selected from the group consisting of SEQ ID NOs: 6 and 8.

In some embodiments the humanized anti-TrkA antibody or fragment thereof comprises:
a) a heavy chain variable domain comprising a sequence selected from the group consisting of SEQ ID NOs: 32, 36, 48 and 49 and
b) a light chain variable domain comprising a sequence of SEQ ID NO: 6 or SEQ ID NO: 8.

In some embodiments the humanized anti-TrkA antibody or fragment thereof comprises
a) a heavy chain variable domain comprising a sequence selected from the group consisting of SEQ ID NOs: 32 and 36, and
b) a light chain variable domain comprising the sequence of SEQ ID NO: 6.

In some embodiments the humanized anti-TrkA antibody or fragment thereof comprises
a) a heavy chain variable domain comprising the sequence of SEQ ID NOs: 36, and
b) a light chain variable domain comprising the sequence of SEQ ID NO: 6.

In some embodiments, the humanized anti-TrkA antibody comprises a combination of a heavy chain variable domain and a light chain variable domain selected from the group comprising the sequences of SEQ ID NO: 32 and SEQ ID NO: 6, SEQ ID NO: 32 and SEQ ID NO: 8, SEQ ID NO: 36 and SEQ ID NO: 6, SEQ ID NO: 48 and SEQ ID NO: 6, SEQ ID NO: 49 and SEQ ID NO: 6, and SEQ ID NO: 49 and SEQ ID NO: 8, preferably selected from the group comprising the sequences of SEQ ID NO: 32 and SEQ ID NO: 6, SEQ ID NO: 32 and SEQ ID NO: 8, SEQ ID NO: 36 and SEQ ID NO: 6, SEQ ID NO: 49 and SEQ ID NO: 6, and SEQ ID NO: 49 and SEQ ID NO: 8, most preferably selected from the combination of sequences of SEQ ID NO: 36 and SEQ ID NO: 6.

In some embodiments the humanized anti-TrkA antibody or fragment thereof further comprises heavy and/or light constant regions, preferably heavy and/or light constant regions and a hinge region. Preferably the heavy constant regions are of human origin and are e.g. of human IgG1 (IGHG1), IgG2 (IGHG2), IgG3 (IGHG3), IgG4 (IGHG4), IgA1 (IGHA1), IgA2 (IGHA2), IgM (IGHM), IgD (IGHD), or IgE (IGHE) isotype. More preferably the heavy constant regions are of human IGHG1 isotype or are of human IGHG4 isotype. Preferably the light constant regions are of human origin and are human kappa (CK) or human lambda (Cλ) light constant domains, preferably a human kappa light constant domain.

In some embodiments the humanized anti-TrkA antibody or fragment thereof further comprises heavy and/or light constant regions and a hinge region, wherein the heavy constant region and the hinge region are of human IGHG1 isotype or are of human IGHG4 isotype.

In some embodiments the humanized anti-TrkA antibody or fragment thereof further comprises heavy and/or light constant regions and a hinge region, wherein the heavy constant region and the hinge region are of human IGHG4 isotype and wherein the hinge region comprises amino acid substitution S228P, wherein the amino acid position is indicated utilizing the EU numbering system.

In a further aspect the present invention provides a humanized anti-TrkA antibody or fragment thereof comprising:
a) a heavy chain comprising a sequence selected from the group consisting of SEQ ID NOs: 50 to 70, and
b) a light chain comprising a sequence selected from the group consisting of SEQ ID NOs: 29 and 30.

In some embodiments the humanized anti-TrkA antibody or fragment thereof comprises
a) a heavy chain comprising a sequence selected from the group consisting of SEQ ID NOs: 51, 52, 56, 57, 60, 64, 69 and 70, and
b) a light chain comprising a sequence selected from the group consisting of SEQ ID NOs: 29 and 30, preferably SEQ ID NO: 29.

In some embodiments the humanized anti-TrkA antibody or fragment thereof comprises
a) a heavy chain comprising a sequence selected from the group consisting of SEQ ID NOs: 51, 52, 56, 57, 69 and 70, and
b) a light chain comprising a sequence selected from the group consisting of SEQ ID NOs: 29 and 30, preferably SEQ ID NO: 29.

In some embodiments the humanized anti-TrkA antibody or fragment thereof comprises
a) a heavy chain comprising a sequence selected from the group consisting of SEQ ID NOs: 51, 52, 56, 57, and 70, and
b) a light chain comprising a sequence selected from the group consisting of SEQ ID NOs: 29 and 30, preferably SEQ ID NO: 29.

In some embodiments the humanized anti-TrkA antibody or fragment thereof comprises
a) a heavy chain comprising a sequence selected from the group consisting of SEQ ID NOs: 51, 52, 56, and 57, and
b) a light chain comprising a sequence selected from the group consisting of SEQ ID NOs: 29 and 30, preferably SEQ ID NO: 29.

In a preferred embodiment, the humanized anti-TrkA antibody or fragment thereof comprises
a) a heavy chain comprising the sequence of SEQ ID NO: 57, and
b) a light chain comprising the sequence of SEQ ID NO: 29.

In some embodiments the humanized anti-TrkA antibody or fragment thereof is a full length antibody.

In some embodiments the humanized anti-TrkA antibody or fragment thereof is an antibody fragment selected from the group consisting of Fab, Fab', Fab'-SH, Fd, Fv, dAb, F(ab')2, scFv, bispecific single chain Fv dimers, diabodies, triabodies and scFv genetically fused to the same or a different antibody; preferably a scFv, or a Fab; more preferably a scFv dimer or a diabody or a F(ab')₂.

In some embodiments the humanized anti-TrkA antibody or fragment thereof comprises a variant Fc region which comprises at least one amino acid modification relative to the Fc region of the parent antibody, whereas the antibody comprising the variant Fc region exhibits altered effector function compared to the parent antibody.

Amino acid modification within the Fc region typically alter one or more functional properties of the antibody, such as serum half-life, complement fixation, effector function related to Fc receptor or ligand binding, and/or antigen-dependent cellular cytotoxicity. Modifications within the Fc region as outlined below are according to the EU numbering of residues in the Fc region. In one embodiment, the hinge region of CH1 is modified such that the number of cysteine residues in the hinge region is altered, e.g., increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody. In another embodiment, the Fc hinge region of an antibody is mutated to decrease the biological half life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al. In another embodiment, the antibody is modified to increase its biological half life. Various approaches are possible. For example, one or more of the following mutations can be introduced: T252L, T254S, T256F, as described in U.S. Patent No. 6,277,375 to Ward. Alternatively, to increase the biological half life, the antibody can be altered within the CH1 or CL region to contain a salvage receptor binding epitope taken from two loops of a CH2 domain of an Fc region of an IgG, as described in U.S. Patent Nos. 5,869,046 and 6,121,022 by Presta *et al.* In a further embodiment, an Fc region is altered by replacing at least one amino acid residue with a different amino acid residue to alter the effector function(s) of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen- binding ability of the parent antibody. The effector ligand to which affinity is altered can be, for example, an Fc receptor or the C1 component of complement. This approach is described in further detail in U.S. Patent Nos. 5,624,821 and 5,648,260, both by Winter *et al.* In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Patent Nos. 6,194,551 by Idusogie et al. In another example, one or more amino acid residues within amino acid positions 231 to 238 in the N-terminal region of the CH2 domain are altered to thereby alter the ability of the antibody to fix complement. This approach is described further in PCT Publication WO 94/29351 by Bodmer et al. In yet another example, the Fc region is modified to increase the ability of the antibody to mediate antibody dependent cellular cytotoxicity (ADCC) and/or to increase the affinity of the antibody for an Fcγ receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Furthermore, an antibody of the invention may be chemically modified (e.g., one or more chemical moieties can be attached to the antibody) or be modified to alter its glycosylation.

### Properties of the antibodies of the invention

In some embodiments the humanized anti-TrkA antibody or fragment thereof is capable of inhibiting the functional activation of TrkA.

In some embodiments the humanized anti-TrkA antibody is capable of blocking or reducing one or more biological activities of TrkA.

In some embodiments the humanized anti-TrkA antibody or fragment thereof binds to human TrkA with an affinity (K_{D}) of 500 nM or less, preferably 350nM or less, more preferably 150 nM or less, even more preferably 100 nM or less, most preferably 50 nM or less, in particular 30 nM or less e.g. measured by Surface Plasmon Resonance (SPR) using a BIAcore 2000 instrument (GE Healthcare Europe GmbH, Glattbrugg, Switzerland) or equivalent instrument known in the art by capturing the antibody on the instrument sensor chip with a recombinant monovalent human TrkA extracellular domain (SEQ ID NO: 25) used as analyte. "Monovalent" as used herein in relation to affinity measurements using TrkA receptor refers to a human TrkA receptor domain, like the human TrkA extracellular domain which is not artificially dimerized or multimerized as it would be e.g. if the domain would be amino-terminally fused to an immunoglobulin Fc portion, or which is not naturally dimerized as it would be e.g. if the domain would be associated with its natural ligand NGF.

Standard assays to evaluate the binding ability of the antibodies toward e.g. human TrkA are known in the art, including for example, ELISAs, BIAcore, Western blots, RIAs and flow cytometry analysis. Suitable assays are described in detail in the Examples. The binding kinetics (e.g. binding affinity like K_{D}) of the antibodies also can be assessed by standard assays known in the art, such as by Scatchard or Biacore® system analysis. The relative binding affinity Kᵢ can be assessed by standard competition assays known in the art. Engineered anti-TrkA antibodies can be assayed for their ability to inhibit the functional activation of TrkA in TF-1 cell proliferation assays. The half maximal inhibitory concentration (IC50) which is a measure of the effectiveness of a compound in inhibiting biological function can be used to select preferred antibodies.

In some embodiments the humanized anti-TrkA antibody or fragment thereof has at least an equivalent or lower IC50 in a TF-1 cell proliferation assay than the corresponding parental murine antibody e.g. measured by the ability of the antibody to block cell surface TrkA/beta-NGF mediated cell proliferation using the factor dependent human erythroleukemic cell line TF-1 (Kitamura T et al., (1989) J. Cellular Physiology 140(2):323-34). Preferably the humanized anti-TrkA antibody or fragment thereof has an IC50 in a TF-1 cell proliferation assay of 1 µg/ml or less, more preferably of 0.75 µg/ml, even more preferably of 0.5 µg/ml or less, most preferably of 0.3 µg/ml or less, in particular of 0.1 µg/ml or less.

In some embodiments the humanized anti-TrkA antibody or fragment thereof has a FAB fragment thermostability temperature greater than 65°C, preferably greater than 70°C. For analysis of FAB fragment thermostability differential scanning calorimetry measurements are used, whereas a mid-point melting temperature of the FAB fragment in context of a full-length IgG is identified. These kind of calorimetric measurements are known to the skilled person and can be carried out according to e.g. Garber & Demarest (2007) BBRC 355:751-7. Surprisingly, it has been found that the humanized antibody of the present invention has a FAB fragment thermostability temperature equivalent to the FAB fragment thermostability temperature of the parental murine antibody while having equivalent affinity as measured by SPR and improved potency as measured by TF-1 cell proliferation assay. Thus the present disclosure also provides a humanized anti-TrkA antibody which has a FAB fragment thermostability temperature which is equivalent to the FAB fragment thermostability temperature of the parental murine antibody with an equivalent affinity for human TrkA and improved inhibitory properties.

"Equivalent to the FAB fragment thermostability temperature of the parental murine antibody" as used herein in this context means that the humanized anti-TrkA antibody or fragment thereof has a FAB fragment thermostability temperature which is within a range of ± 20%, preferably within a range of ± 15%, more preferably within a range of ± 10%, even more preferably within a range of ± 5% of the FAB fragment thermostability temperature of the parental murine antibody. Preferably, the humanized antibody of the present invention has a FAB fragment thermostability temperature not more than 15% lower than the FAB fragment thermostability temperature of the parental murine antibody.

"Equivalent affinity for human TrkA" as used herein in this context means that the humanized anti-TrkA antibody or fragment thereof has an affinity which is within a range of ± 20%, preferably within a range of ± 15%, more preferably within a range of ± 10% of the parental murine antibody. Preferably, the humanized antibody of the present invention has a K_{D} which is at least 5%, preferably at least 10% lower than the K_{D} of the parental murine antibody.

The present invention also provides a humanized anti-TrkA antibody or fragment thereof which can be used to treat pain.

The effect of a humanized anti-TrkA antibody was tested in mice suffering from acute inflammatory hyperalgesia induced by intraplantar injection of Complete Freunds adjuvant (CFA) into the hind paw (see Example 2). Administration of the humanized anti-TrkA antibody at a dose of 0.01mg/kg or above produced a significant reversal of hyperalgesia, which was similar to that observed with the NSAID indomethacin.

The effect of a humanized anti-TrkA antibody was tested in mice suffering from chronic inflammatory hyperalgesia induced by intra-articular injection of CFA into the knee joint (see Example 3). Administration of the humanized anti-TrkA antibody at a single dose of 0.01 mg/kg or above produced a significant reversal of hyperalgesia, which was comparable to that observed with multiple dosing of the COX-2 selective NSAID celecoxib.

The effect of a humanized anti-TrkA antibody was tested in mice suffering from chronic osteoarthritic hyperalgesia induced by intra-articular injection of monosodium iodoacetate (MIA) into the knee joint (see Example 4). Administration of the humanized anti-TrkA antibody at a single dose of 0.01 mg/kg or above produced a significant reversal of hyperalgesia, which was comparable to that observed with multiple dosing of the opiate tramadol and pregabalin.

The effect of a humanized anti-TrkA antibody was tested in mice suffering from neuropathic pain induced by chronic constriction of the sciatic nerve (CCI model; see Example 5). Administration of the humanized anti-TrkA antibody at a single dose of 0.01 mg/kg or above produced a significant reversal of mechanical hyperalgesia and cold allodynia, which at the highest dose tested, 1 mg/kg, was comparable to that observed with multiple dosing of pregabalin.

Therefore, a preferred embodiment of the present invention provides a humanized anti-TrkA antibody for the treatment of a patient suffering from acute inflammatory pain, chronic inflammatory pain, osteoarthritic pain and/or neuropathic pain.

### Nucleic acids, Vectors and Host Cells

The present disclosure also provides isolated nucleic acids encoding the anti-TrkA antibodies and fragments thereof, vectors, and host cells comprising the nucleic acid or the vector. The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art, see e.g. Ausubel F et al., ed. (1987) Current Protocols in Molecular Biology, Greene Publishing and Wiley Interscience, New York. A nucleic acid of the invention can be, for example, DNA or RNA and may or may not contain intronic sequences. In a preferred embodiment, the nucleic acid is a cDNA molecule.

Nucleic acids of the invention can be obtained using standard molecular biology techniques e.g. cDNAs encoding the light and heavy chains of the antibody or encoding VH and VL segments can be obtained by standard PCR amplification or cDNA cloning techniques. For antibodies obtained from an immunoglobulin gene library (e.g., using phage display techniques), one or more nucleic acids encoding the antibody can be recovered from the library. The methods of introducing exogenous nucleic acid into host cells are well known in the art and will vary with the host cell used. Techniques include but are not limited to dextran- mediated transfection, calcium phosphate precipitation, calcium chloride treatment, polyethylenimine mediated transfection, polybrene mediated transfection, protoplast fusion, electroporation, viral or phage infection, encapsulation of the polynucleotide(s) in liposomes, and direct microinjection of the DNA into nuclei. In the case of mammalian cells, transfection may be either transient or stable.

In some embodiments the isolated nucleic acids encoding the anti-TrkA antibodies and fragments thereof comprise a nucleic acid sequence selected from the group consisting of SEQ ID NOs 73-116, usually nucleic acids molecules encoding the light chain variable region or the light chain selected from the group consisting of SEQ ID NOs: 113, 114, 115 and 116 and/or the heavy chain variable region or the heavy chain selected from the group consisting of SEQ ID NOs: 73-112.

Preferred nucleic acids molecules of the invention are those encoding the light chain variable region selected from the group consisting of SEQ ID NOs: 113 and 114 and/or the heavy chain variable region selected from the group consisting of SEQ ID NOs: 74, 78, 81, 85, 90 and 91. More preferred are nucleic acids molecules encoding the heavy chain variable region selected from the group consisting of SEQ ID NOs: 74 and 78 and/or encoding the light chain variable region selected from the group consisting of SEQ ID NOs: 113 and 114. Most preferred are nucleic acids molecules encoding the heavy chain variable region comprising the nucleic acid sequence of SEQ ID NO: 74 or 78 and/or encoding the light chain variable region comprising the nucleic acid sequence of SEQ ID NO: 113.

Further preferred nucleic acids molecules of the invention are those encoding the light chain selected from the group consisting of SEQ ID NO: 115 and 116 and/or the heavy chain selected from the group consisting of SEQ ID NO: 93, 94, 98, 99, 102, 106, 111 and 112. More preferred are nucleic acids molecules encoding the heavy chain comprising the nucleic acid sequence of SEQ ID NO: 93, 94, 98, and 99 and/or encoding the light chain selected from the group consisting of of SEQ ID NO: 115 and 116. Most preferred are nucleic acids molecules encoding the heavy chain comprising the nucleic acid sequence of SEQ ID NO: 93, 94, 98, and 99 and/or encoding the light chain comprising the nucleic acid sequence of SEQ ID NO: 115.

Once DNA fragments encoding VH and VL segments are obtained, these DNA fragments can be further manipulated by standard recombinant DNA techniques, for example to convert the variable region genes to full-length antibody chain genes, or to fragment genes corresponding to the fragments described above like e.g. Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame. The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, EA *et al.*, *ibid.)* and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG4 constant region, preferably a human IGHG4 constant region wherein the hinge region comprises amino acid substitution S228P. For a Fab fragment heavy chain gene, the VH encoding DNA can be operatively linked to another DNA molecule encoding only the heavy chain CH1 constant region. The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat EA *et al*., *ibid.)* and DNA fragments encompassing these regions can be obtained by standard PCR amplification. In preferred embodiments, the light chain constant region can be a kappa or lambda constant region, preferably a kappa constant region. To create a scFv gene, the VH- and VL-encoding DNA fragments are operatively linked to another fragment encoding a flexible linker, e.g., encoding the amino acid sequence (Gly4 -Ser)3, such that the VH and VL sequences can be expressed as a contiguous single-chain protein, with the VL and VH regions joined by the flexible linker (see e.g., Bird *et al*., *ibid.; Huston et al*., *ibid.;* McCafferty et al., (1990) Nature 348: 552-554). Various techniques have been developed for the production of antibody fragments of antibodies. Traditionally, these fragments were derived via proteolytic digestion of intact antibodies (see e.g. Morimoto et al., (1992) J. Biochem. Biophysical Methods, 24: 107-117 and Brennan et al., (1985) Science, 229:81). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., (1992) Bio/Technology, 10:163-167). According to another approach, F(ab')2 fragments can be isolated directly from recombinant host cell culture. Other techniques for the production of antibody fragments will be apparent to the skilled practitioner. In other embodiments, the antibody of choice is a single-chain Fv fragment (scFv), see e.g. WO93/16185; US Patent Nos. 5,571,894 and 5,587,458. The antibody fragment may also be a "linear antibody", e.g., as described in US Patent No. 5,641,870, for example.

The nucleic acids that encode the antibodies of the present invention may be incorporated into a vector, preferably an expression vector in order to express the protein. A variety of expression vectors may be utilized for protein expression. Expression vectors may comprise self-replicating extra- chromosomal vectors or vectors which integrate into a host genome. Expression vectors are constructed to be compatible with the host cell type. Thus vectors, preferably expression vectors, which find use in the present invention include but are not limited to those which enable protein expression in mammalian cells, bacteria, insect cells, yeast, and in *in vitro* systems. As is known in the art, a variety of expression vectors are available, commercially or otherwise, that may find use in the present invention for expressing antibodies.

Expression vectors typically comprise a protein operably linked with control or regulatory sequences, selectable markers, any fusion partners, and/or additional elements. By "operably linked" herein is meant that the nucleic acid is placed into a functional relationship with another nucleic acid sequence. The term "regulatory sequence" is intended to include promoters, enhancers and other expression control elements (e.g., polyadenylation signals) that control the transcription or translation of the antibody chain genes. Such regulatory sequences are described, for example, in Goeddel (Gene Expression Technology, Methods in Enzymology 185, Academic Press, San Diego, CA (1990)). Generally, these expression vectors include transcriptional and translational regulatory nucleic acid operably linked to the nucleic acid encoding the antibody, and are typically appropriate to the host cell used to express the protein. In general, the transcriptional and translational regulatory sequences may include promoter sequences, ribosomal binding sites, transcriptional start and stop sequences, translational start and stop sequences, and enhancer or activator sequences. As is also known in the art, expression vectors typically contain a selection gene or marker to allow the selection of transformed host cells containing the expression vector. Selection genes are well known in the art and will vary with the host cell used. For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr- host cells with methotrexate selection/amplification) and the neo gene (for G418 selection).

Suitable host cells for cloning or expressing the DNA in the vectors herein are prokaryote, yeast, or higher eukaryote cells. Suitable prokaryotes for this purpose include eubacteria, including gram-negative or gram-positive organisms, for example, *Enterobacteriaceae* such as *Escherichia,* e.g., *E. coli, Enterobacter, Klebsiella, Proteus, Salmonella,* e.g., *Salmonella typhimurium, Serratia,* e.g., *Serratia marcescans* and *Shigella,* as well as *Bacilli* such as *B. subtilis* and *B. licheniformis, Pseudomonas* such as *P. aeruginosa* and *Streptomyces.* Suitable *E. coli* cloning hosts include *E. coli* 294 (ATCC 31,446), *E. coli* B, *E. coli* X1776 (ATCC 31,537), and *E. coli* W3110 (ATCC 27,325).

In addition to prokaryotes, eukaryotic microbes such as filamentous fungi or yeast are suitable cloning or expression hosts. *Saccharomyces cerevisiae* or common baker's yeast is the most commonly used among lower eukaryotic host microorganisms. Host cells for expressing the recombinant antibodies of the invention are preferably mammalian host cells which include Chinese Hamster Ovary (CHO cells) (including dhfr⁻ CHO cells, described in Urlaub & Chasin (1980) PNAS USA 77:4216-4220, used with a DHFR selectable marker, e.g., as described in Kaufman & Sharp (1982) J. Mol. Biol. 159:601-621), NSO myeloma cells, COS cells , SP2 cells and HEK293-EBNA1 cells (ATCC® catalogue number: CRL-10852). In particular, for use with NSO myeloma cells, another preferred expression system is the GS gene expression system disclosed in WO87/04462, WO89/01036 and EP0338841.

### Construction and Production of Antibodies

Antibodies generated against the TrkA polypeptide may be obtained by immunisation of an animal i.e. by administering the polypeptides to an animal, preferably a non-human animal, using well-known and routine protocols, see for example Handbook of Experimental Immunology, Weir DM (ed.), Vol 4, Blackwell Scientific Publishers, Oxford, England, (1986). Many warm-blooded animals, such as rabbits, mice, rats, sheep, cows, camels or pigs may be immunized. However, mice, rabbits, pigs and rats in particular mice are generally most suitable. Antibodies can be produced as well by recombinant DNA techniques known to the skilled person. In additional antibodies can be produced by enzymatic or chemical cleavage of naturally occurring antibodies. Humanized antibodies of the present invention may be constructed by transferring one or more CDRs or portions thereof from VH and/or VL regions from a non-human animal (e.g., mouse) to one or more framework regions from human VH and/or VL regions. Preferably the humanized antibodies of the present invention are constructed by transferring one or more CDRs or portions thereof from VH and/or VL regions from murine MNAC 13 antibody as disclosed in WO00/73344 to one or more framework regions from human VH and/or VL regions. Optionally, human framework residues thus present in the VH and/or VL regions may be replaced by corresponding non-human (e.g., mouse) residues when needed or desired for decreasing immunogenicity of the antibody and/or maintaining binding affinity. Optionally, non-human amino acid residues present in the CDRs may be replaced with human residues. Chimeric or humanized antibodies of the present invention can be prepared based on the sequence of a non-human monoclonal antibody prepared as described above. DNA encoding the heavy and light chain immunoglobulins can be obtained from the non- human hybridoma of interest and engineered to contain non-murine (e.g., human) immunoglobulin sequences using standard molecular biology techniques. For example, to create a chimeric antibody, murine variable regions can be linked to human constant regions using methods known in the art (see e.g., US Patent No. 4,816,567 to Cabilly et al). To create a humanized antibody, murine CDR regions can be inserted into a human framework using methods known in the art (see e.g., US Patent No. 5,225,539 to Winter and US Patent Nos. 5,530,101; 5,585,089; 5,693,762 and 6,180,370 to Queen et al).

Humanized antibodies of the present invention may be constructed wherein the human acceptor molecule for the heavy chain variable region is selected based on homology considerations between potential acceptor molecule variable regions and the heavy chain variable region of the murine antibody. Germline candidate human acceptor molecules are preferred to reduce potential immunogenicity. Germline databases are made up of antibody sequences that read through the end of the heavy chain FW3 region and partially into the CDR3 sequence. For selection of a FW4 region, databases of mature antibody sequences which have been derived from the selected germline molecule can be searched or antibody sequences which have been derived from the selected germline molecule from a human donor can be used. Human acceptor molecules are preferably selected from the same heavy chain class as the murine donor molecule, and of the same canonical structural class of the variable region of the murine donor molecule. Secondary considerations for selection of the human acceptor molecule for the heavy chain variable region include homology in CDR length between the murine donor molecule and the human acceptor molecule. Human acceptor antibody molecules are preferably selected by homology search to the V-BASE database, although other databases such as the Kabat and the public NCBI databases may be used as well.

Humanized antibodies of the present invention may be constructed wherein the human acceptor molecule for the light chain variable region is selected based on homology considerations between potential acceptor molecule variable regions and with the light chain variable region of the murine antibody. Germline candidate human acceptor molecules are preferred to reduce potential immunogenicity. Germline databases are made up of antibody sequences that read through the end of the heavy chain FW3 region and partially into the CDR3 sequence. For selection of a FW4 region, databases of mature antibody sequences which have been derived from the selected germline molecule can be searched or antibody sequences which have been derived from the selected germline molecule from a human donor can be used. Human acceptor molecules are preferably selected from the same light chain class as the murine donor molecule and of the same canonical structural class of the variable region of the murine donor molecule. Secondary considerations for selection of the human acceptor molecule for the light chain variable region include homology in CDR length between the murine donor molecule and the human acceptor molecule. Human acceptor antibody molecules are preferably selected by homology searches to the V-BASE database, and other databases such as the Kabat and the public NCBI databases may be used as well.

When the antibody is produced as recombinant antibody by e.g. introducing genes into mammalian host cells, the antibodies are produced by culturing the host cells for a period of time sufficient to allow for expression of the antibody in the host cells or, more preferably, for secretion of the antibody into the culture medium in which the host cells are grown. Host cells useful for producing antibodies that bind to human TrkA may be cultured in a variety of media. Commercially available media such as Ham's F10 (Sigma-Aldrich Chemie GmbH, Buchs, Switzerland), Minimal Essential Medium (MEM; Sigma-Aldrich Chemie GmbH), RPMI-1640 (Sigma-Aldrich Chemie GmbH, Basel, Switzerland), EX-CELL 293, HEK293-serum-free medium (Sigma, Buchs, Switzerland) and Dulbecco's Modified Eagle's Medium ((DMEM; Sigma-Aldrich Chemie GmbH) are suitable for culturing the host cells. Antibodies can be recovered from the culture medium using standard protein purification methods.

Antibodies may be operably linked to a fusion partner to enable targeting of the expressed protein, purification, screening, display and the like. Fusion partners may be linked to the antibody sequence via a linker sequences. The linker sequence will generally comprise a small number of amino acids, typically less than ten, although longer linkers may also be used. Typically, linker sequences are selected to be flexible and resistant to degradation. As will be appreciated by those skilled in the art, any of a wide variety of sequences may be used as linkers. For example, a common linker sequence comprises the amino acid sequence GGGGS. A fusion partner may be a targeting or signal sequence that directs antibody and any associated fusion partners to a desired cellular location or to the extracellular media. As is known in the art, certain signalling sequences may target a protein to be either secreted into the growth media, or into the periplasmic space, located between the inner and outer membrane of the cell. A fusion partner may also be a sequence that encodes a peptide or protein that enables purification and/or screening. Such fusion partners include but are not limited to polyhistidine tags (His-tags) (for example H6 and H10 or other tags for use with Immobilized Metal Affinity Chromatography (IMAC) systems (e.g. Ni²⁺affinity columns)), GST fusions, MBP fusions, Strep-tag, the BSP biotinylation target sequence of the bacterial enzyme BirA and epitope tags which are targeted by antibodies (for example c-myc tags, flag-tags and the like). As will be appreciated by those skilled in the art, such tags may be useful for purification, for screening or for both.

### Characterization and Purification of Anti-TrkA antibodies

Screening for antibodies can be performed using assays to measure binding to human TrkA and/or assays to measure the ability to block the binding of TrkA to its ligand NGF. An example of a binding assay is an ELISA. In addition, Surface Plasmon Resonance (SPR) analysis as e.g. used in the examples can be applied to measure the association and dissociation rate constants for the binding kinetics of the antibodies. An example of a blocking assay is a flow cytometry based assay measuring the blocking of NGF binding to TrkA. As an assay for evaluating the functional activity of anti-TrkA antibodies e.g. a TF-1 cell proliferation assay can be used, wherein the ability of antibodies to block cell surface TrkA/beta-NGF mediated cell proliferation is assayed using the factor dependent human erythroleukemic cell line TF-1 (Kitamura T et al., (1989) J. Cellular Physiology 140(2):323-34).

Antibodies of the present invention may be isolated or purified in a variety of ways known to those skilled in the art. Standard purification methods include chromatographic techniques, including ion exchange, hydrophobic interaction, affinity, sizing or gel filtration and reversed-phase, carried out at atmospheric pressure or at high pressure using systems such as FPLC and HPLC. Purification methods also include electrophoretic, immunological, precipitation, dialysis and chromatofocusing techniques. Ultrafiltration and diafiltration techniques, in conjunction with protein concentration, are also useful. To purify TrkA antibodies, selected host cells can be grown in e.g. spinner-flasks for monoclonal antibody purification. Supernatants can be filtered and concentrated before affinity chromatography with protein A-sepharose (Pharmacia, Piscataway, NJ). Eluted antibodies can be checked by gel electrophoresis and high performance liquid chromatography to ensure purity. A preferred antibody of the present invention is thus an isolated and/or purified antibody that binds to human TrkA.

### Immunoconjugates

In another aspect, the present invention provides anTrkA antibody or a fragment thereof that binds to human TrkA, linked to a therapeutic agent, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g., methotrexate, 6- mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)) and anti-mitotic agents (e.g., vincristine and vinblastine). Other examples of therapeutic cytotoxins that can be linked to an antibody of the invention include duocarmycins, calicheamicins, maytansines and auristatins and derivatives thereof. An example of a calicheamicin antibody conjugate is commercially available (Mylotarg(R); American Home Products). Cytotoxins can be linked to antibodies of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D). For further discussion of types of cytotoxins, linkers and methods for conjugating therapeutic agents to antibodies, see also Saito G et al., (2003) Adv. Drug Deliv. Rev. 55:199-215; Trail PA et al., (2003) Cancer Immunol. Immunother. 52:328-337; Payne G (2003) Cancer Cell 3:207-212; Allen TM (2002) Nat. Rev. Cancer 2:750-763; Pastan I & Kreitman RJ (2002) Curr. Opin. Investig. Drugs 3:1089-1091; Senter PD & Springer CJ (2001) Adv. Drug Deliv. Rev. 53:247-264. Antibodies of the present invention also can be linked to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine-131, indium-111, yttrium-90 and lutetium-177. Methods for preparing radioimmunconjugates are established in the art. Examples of radioimmunoconjugates are commercially available, including Zevalin® (EDEC Pharmaceuticals) and Bexxar® (Corixa Pharmaceuticals) and similar methods can be used to prepare radioimmunoconjugates using the antibodies of the invention. The antibody immunoconjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-γ; or biological response modifiers such as, for example, lymphokines, interleukin-1 (IL-1), interleukin-2 (IL-2), interleukin-6 (IL-6), granulocyte macrophage colony stimulating factor (GM-CSF), granulocyte colony stimulating factor (G-CSF), or other growth factors. Techniques for linking such therapeutic agents to antibodies are well known, see, e.g., Arnon et al., "Monoclonal Antibodies for Immunotargeting of Drugs in Cancer Therapy", in Monoclonal Antibodies and Cancer Therapy, Reisfeld et al., (eds.), pp. 243- 56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985) and Thorpe et al., Immunol. Rev. 62:119-58 (1982).

In another aspect, the present invention provides an anti-TrkA antibody or a fragment thereof that binds to human TrkA, administered together with a therapeutic agent, such as a cytotoxin, a drug (e.g., an immunosuppressant) or a radiotoxin.

### Pharmaceutical Compositions

In another aspect, the present invention provides a composition, e.g., a pharmaceutical composition, comprising the antibody or fragment thereof of the present invention and a pharmaceutically acceptable carrier. Such compositions may include one or a combination of (e.g., two or more different) antibodies, and/or immunoconjugates of the invention and/or a therapeutic agent, such as a cytotoxin, a drug (e.g., an immunosuppressant) or a radiotoxin as described *supra.* For example, a pharmaceutical composition of the invention can comprise a combination of antibodies (or immunoconjugates) that bind to different epitopes on the target antigen or that have complementary activities. Pharmaceutical compositions of the invention also can be administered in combination therapy, i.e., combined with other agents as outlined further below.

As used herein, "pharmaceutically acceptable carrier" includes any and all solvents, dispersion media, coatings, antibacterial and antifungal agents, isotonic and absorption delaying agents and the like that are physiologically compatible. Preferably, the carrier is suitable for intravenous, intramuscular, subcutaneous, parenteral, spinal or epidermal administration (e.g. by injection or infusion). Depending on the route of administration, the active compound, i.e. antibody or immunoconjugate may be coated in a material to protect the compound from the action of acids and other natural conditions that may inactivate the compound. Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

In another aspect, the present invention provides a composition comprising the humanized anti-TrkA antibody or fragment thereof of the present invention and another pharmaceutically active agent. Preferably the pharmaceutically active agent is one or more of:
a) an analgesic agent, b) another anti-TrkA antibody, c) NGF, d) an anti-cancer agent,
e) an anti-NGF antibody as outlined further below.

In another aspect, the present invention provides a composition comprising an immunoconjugate comprising the antibody or fragment thereof that binds to human TrkA linked to a therapeutic agent and a pharmaceutically acceptable carrier. Immunoconjugates and therapeutic agents which can be used are as described *supra.*

A pharmaceutical composition of the invention may also include a pharmaceutically acceptable antioxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil- soluble antioxidants, such as ascorbyl palmitate, butylated hydroxyanisole (BHA), butylated hydroxytoluene (BHT), lecithin, propyl gallate, alpha-tocopherol and the like; and (3) metal chelating agents, such as citric acid, ethylenediamine tetraacetic-acid (EDTA), sorbitol, tartaric acid, phosphoric acid and the like. Examples of suitable aqueous and non-aqueous carriers that may be employed in the pharmaceutical compositions of the invention include water, ethanol, polyols (such as glycerol, propylene glycol, polyethylene glycol and the like) and suitable mixtures thereof, vegetable oils, such as olive oil and injectable organic esters, such as ethyl oleate. Proper fluidity can be maintained, for example, by the use of coating materials, such as lecithin, by the maintenance of the required particle size in the case of dispersions and by the use of surfactants. These compositions may also contain adjuvants such as preservatives, wetting agents, emulsifying agents and dispersing agents. Prevention of presence of microorganisms may be ensured both by sterilization procedures, *supra* and by the inclusion of various antibacterial and antifungal agents, for example, paraben, chlorobutanol, phenol sorbic acid and the like. It may also be desirable to include isotonic agents, such as sugars, sodium chloride and the like into the compositions. In addition, prolonged absorption of the injectable pharmaceutical form may be brought about by the inclusion of agents which delay absorption such as aluminum monostearate and gelatin.

### Therapeutic and other uses

Antibodies of the present invention can be used in medicine to treat various disorders/ conditions, as set out in various categories below.

The invention thus provides a method of treatment of the below mentioned conditions which comprises administering to a subject, suitably a mammalian subject, especially a human subject in need thereof, a therapeutically effective amount of an antibody or derivative as described herein such that the condition is thereby treated.

The invention also provides use of an antibody or derivative as described herein in the manufacture of a medicament for the treatment of the below mentioned conditions.

Here the term "treatment" includes therapeutic treatment of an existing disorder/condition. It also includes prophylactic treatment. It further includes the amelioration of one or more adverse symptoms, even if a patient is not cured of a given disorder/condition. For example, pain may be alleviated or reduced.

A preferred medical use is in the treatment of pain. According to International Association for the Study of Pain ("IASP") pain is generally defined as "An unpleasant sensory and emotional experience associated with actual or potential tissue damage, or described in terms of such damage or both". The essential element in all forms of pain is the activation of specialized high-threshold receptors and nerve fibers to warn the organism of potential tissue damage. The involvement of inflammatory cells and processes is a common element in many pain states. The term "acute pain" means immediate, generally high threshold, pain brought about by injury such as a cut, crush, burn, or by chemical stimulation. The term "chronic pain," as used herein, means pain other than acute pain, both of inflammatory and neuropathic origin. It is understood that chronic pain often is of relatively long duration, for example, months or years and can be continuous or intermittent. Antibodies of the present invention can be used to treat chronic pain or acute pain. The treatment of chronic pain is preferred.

The pain may for example be or may be associated with any of the following: inflammatory pain, post-surgical pain, post-operative pain (including dental pain), neuropathic pain, peripheral neuropathy, diabetic neuropathy, diabetic nephropathy, fracture pain, gout joint pain, post-herpetic neuralgia, cancer pain, osteoarthritis or rheumatoid arthritis pain, sciatica, pains associated with sickle cell crises, headaches (e.g., migraines, tension headache, cluster headache), dysmenorrhea, endometriosis, uterine fibroids, musculoskeletal pain, chronic low back pain, fibromyalgia, sprains, visceral pain, ovarian cysts, prostatitis, chronic pelvic pain syndrome, cystitis, interstitial cystitis, painful bladder syndrome and/or bladder pain syndrome, pain associated with chronic abacterial prostatitis, incisional pain, migraine, trigeminal neuralgia, pain from bums and/or wounds, pain associated with trauma, pain associated with musculoskeletal diseases, ankylosing spondilitis, periarticular pathologies, pain from bone metastases, pain from HIV, erythromelalgia or pain caused by pancreatitis or kidney stones, malignant melanoma, Sjogren's syndrome, asthma, (e.g., uncontrolled asthma with severe airway hyper-responsiveness), intractable cough, demyelinating diseases, chronic alcoholism, stroke, thalamic pain syndrome, pain from toxins, pain from chemotherapy, inflammatory bowel disorders, irritable bowel syndrome, inflammatory eye disorders, inflammatory or unstable bladder disorders, psoriasis, skin complaints with inflammatory components, sunburn, carditis, dermatitis, myositis, neuritis, collagen vascular diseases, chronic inflammatory conditions, inflammatory pain and associated hyperalgesia and allodynia, neuropathic pain and associated hyperalgesia or allodynia, diabetic neuropathy pain, causalgia, sympathetically maintained pain, deafferentation syndromes, epithelial tissue damage or dysfunction, disturbances of visceral motility at respiratory, genitourinary, gastrointestinal or vascular regions, allergic skin reactions, pruritis, vitiligo, general gastrointestinal disorders, colitis, gastric ulceration, duodenal ulcers, vasomotor or allergic rhinitis, bronchial disorders, dyspepsia, gastroesophageal reflux, pancreatitis, visceralgia and fibrous dysplasia of bone (FD).

The pain may for example be or may be associated with any of the following: pancreatitis, kidney stones, endometriosis, IBD, Crohn's disease, post surgical adhesions, gall bladder stones, headaches, dysmenorrhea, musculoskeletal pain, sprains, visceral pain, ovarian cysts, prostatitis, cystitis, interstitial cystitis, post-operative pain, migraine, trigeminal neuralgia, pain from bums and/or wounds, pain associated with trauma, neuropathic pain, pain associated with musculoskeletal diseases, rheumatoid arthritis, osteoarthritis, ankylosing spondilitis, periarticular pathologies, oncological pain, pain from bone metastases, HIV infection.

Various models are known for assessing pain and can be used in screening antibodies/derivatives thereof. For example, the nociception hot plate test can be used, as disclosed in WO 00/73344, for example. The experiment can be carried out according to McMahon et al., 1995 (Nature Med. 1:774-780), using the antibody/derivative as immunoadhesin. The antibody/derivative is infused subcutaneously into hind paw of an adult rat for a period of three weeks or by an osmotic mini-pump. The nociception sensitivity is evaluated at intervals using a hot plate test (Eddy & Leimbach (1953) J. Phar. Exp. Ther. 107: 385-393), which mimics hyperalgesia situations following inflammation or partial damage to the nerve. The nociceptive stimulus induces in such a case a response (paw licking and/or jumping) which presumes an integrated coordination higher than simple reflex. According to the test, the animal is put in a pen having a plate heated to the desired temperature as base, usually 56°C. The latency of any of two responses (paw licking and jumping) is measured in control animals (treated with non relevant antibody) and in those treated with the anti-TrkA antibody/derivative.

As an alternative to the hot plate test, the nociceptive response to formalin can be assessed. This test is disclosed by Porro & Cavazzuti, 1993 (Prog. Neurobiol, 41:565-607) and was used in WO06/137106. It involves assessing the reduction in pain response by analyzing any subsequent reduction paw licking when a given candidate is administered prior to testing. Saline is typically used as a negative control.

An assessment of hyperalgesia can be determined using a weight bearing method. Mice normally distribute their body weight equally between their two limbs. Following a painful stimulus to the limb, for example by the local injection of Complete Freunds adjuvant (CFA) or monosodium iodoacetate (MIA) to the hind paw (intraplantar) or knee joint (intra-articular), the weight is redistributed to reduce that placed on the injected limb and increase that placed on the non-injected limb. Weight bearing is measured using an incapacitance tester with the hind paws placed on separate sensors and the average force exerted by both hind limbs recorded. Weight bearing can be used to assess acute inflammatory hyperalgesia resulting from intraplantar injection of CFA, chronic inflammatory hyperalgesia resulting from intra-articular injection of CFA or chronic osteoarthritic hyperalgesia resulting from intra-articular injection of MIA, as detailed in Examples 2, 3 and 4, respectively.

An assessment of mechanical hyperalgesia can be performed by a number of methods, for example, Von Frey filaments or a Randall-Selitto analgesiometer. Paw withdraw thresholds are measured in response to increasing pressure stimuli applied to the plantar hind paw surface by von Frey filaments or to the dorsal hind paw surface by a wedge-shaped probe of a Randall-Selitto analgesiometer. The latency of hind paw withdraw is measured in control animals and in those treated with the anti-TrkA antibody/derivative. These methods can be used to assess mechanical hyperalgesia resulting from the Chronic Constriction Injury (CCI) animal model as detailed in Example 5.

The CCI model is also a well known animal model. It involves chronic constriction of the sciatic nerve and is used to induce chronic pain of a neuropathic nature in rodents, such as mice or rats. This model is described by Bennett & Xie, 1998 in Pain 33:87- 107. It was used in WO 06/131592, for example. A major feature of many neuropathic pain states is that normally innocuous cool stimuli begin to produce pain. An increased response to a non-painful stimulus is termed allodynia. The extent of the neuropathic pain state induced can therefore be measured using a cold plate test for allodynia, as detailed in Example 5. The animal is put in a pen having a plate cooled to the desired temperature, which can be in the range of -5°C to 15°C. The latency of hind paw withdraw is measured in control animals and in those treated with the anti-TrkA antibody/derivative. Typically the latency for withdrawal becomes different from the baseline at surface temperatures of 5°C and below.

The antibodies can be used in the treatment of cancer, a neuronal disorder, Alzheimer's disease, diabetes mellitus, diabetic nephropathy, a viral disorder, an HIV mediated disorder, leprosy or an inflammatory disorder. In addition, the antibodies are also useful in treating other diseases that may be associated with increased levels of NGF including, for example, lupus erythematosus, shingles, postherpetic neuralgia, and hyperalgesia.

Various cancers express TrkA. The interaction of TrkA with NGF may be involved in tumour development (e.g. of prostate and pancreatic cancers). Indeed in certain forms of cancer, an excess of NGF can facilitate the growth and infiltration of nerve fibres. By blocking the action of NGF it is possible to significantly reduce the formation of neuromas. Furthermore, as an alternative to simply providing a blocking effect, the antibodies can be coupled to a cytotoxic agent and can be used to target cancer cells expressing TrkA. It is not however necessary to couple the antibodies to toxins. ADCC (antibody-dependent cell-mediated cytotoxicity) arises due to an immune response in which antibodies , by coating target cells, can make them vulnerable to attack by the system (e.g. by T cells, by complement activation, etc.) Preferred cancers to be treated are prostate cancer, thyroid cancer, lung cancer, prolactinoma, melanoma or bone cancer pain including cancer pain associated with bone metastasis. A preferred cancer to be treated is bone cancer pain including cancer pain associated with bone metastasis.

The antibodies can also be used in the treatment of various neuronal disorders which comprise neurodegenerative disorders e.g. the antibodies can be used to reduce the formation of neuromas. They can also be used in the treatment of Alzheimer's disease or in neuroregenerative therapies. Antibodies of the present invention may be useful in such treatments to reduce undesired agonist effects of NGF (see also the "Combination therapy" section below). Furthermore, the antibodies can be used to treat neuropathic pain, as discussed above. This may be associated with a lesion or a dysfunction of the nervous system. NGF has potential use in the treatment of diabetes and leprosy but has undesired agonist properties including an increase in pain sensitivity which could be avoided by using the antibodies of the present invention.

A still further application is in the treatment of inflammatory disorders. NGF is released by mast cells, fibroblasts and other cell types in the peripheral sites where inflammatory processes occur. In particular, mast cells appear to play a fundamental role. They produce NGF and at the same time express functional TrkA receptors at their surface. The NGF/TrkA system appears to mediate mastocyte activation through an autocrine positive feedback mechanism which allows local amplification of the algogenic inflammatory signal. Examples of inflammatory disorders that may be treated include inflammatory forms of the urinary tract and of the pelvic region, osteoarthritis, multiple sclerosis, colitis, inflammatory bowel disease, bladder cystitis, eczema, contact dermititis, arthritis, including chronic arthritis and rheumatoid arthritis, Crohn's disease, psoriasis and asthma.

Antibodies of the present invention may be useful in such treatments as mentioned above to reduce undesired agonist effects of NGF.

Antibodies or derivatives thereof of the present invention may be used together with one or more other active agents, e.g. pharmaceutically active agents in combination therapy. They may be used for simultaneous, sequential or concerted administration in medicine. For example, the antibody or derivative may be combined with an analgesic agent such as an analgesic opioid or a non-opioid analgesic. It is disclosed in WO06/137106 that small amounts of molecules able to block TrkA biological activity can potentiate the analgesic effects of opioids. Such analgesic opioids include one or more compounds selected from the following: morphine, codeine, dihydrocodeine diacetylmorphine, hydrocodone, hydromorphone, levorphanol, oxymorphone, alfentanil, buprenorphine, butorphanol, fentanyl, sufentanyl, meperidine, methadone, nabumetone , propoxyphene, pentazocine; and their pharmaceutically acceptable derivatives thereof (e.g. pharmaceutically acceptable salts thereof). Suitable non-opioid analgesics include non-steroidal inflammatory drugs (NSAIDs) as well as other analgesics such as acetaminophen. Commonly available NSAIDs to treat acute inflammatory pain include asprin, ibuprofen, indomethacin, naproxen and ketoprofen and to treat chronic inflammatory pain include celecoxib and meloxicam.

A further combination is that of one or more antibodies of the present invention together with one or more other antibodies. A preferred combination is with one or more other anti-TrkA and/or an anti-NGF antibody. Such combinations may provide increased efficacy in treating one or more of the disorders discussed herein, relative to treatment with a single antibody. For example combinations of two or more antibodies found to be amongst the most effective in assay procedures used herein may be used.

A further combination is that of the antibody of the present invention with NGF. As discussed above, the use of NGF in the treatment of various disorders, including Alzheimer's disease, diabetes mellitus, leprosy, etc., had been proposed, but an increase in pain sensitivity had been noted arising from agonist properties towards peripheral targets. Again, by using an antibody or derivative of the present invention, pain sensitivity can be reduced, thereby making NGF-based therapies more attractive.

A further combination is that of the antibody of the present invention with an anti-cancer agent such as e.g. an alkylating agent, an antimetabolite, a topoisomerase II inhibitor, a topoisomerase I inhibitor, an antimitotic drug or a platinum derivative.

The antibodies of the present invention can be administered by any appropriate route. This includes (but is not limited to) intraperitoneal, intramuscular, intravenous, subcutaneous, intratracheal, oral, enteral, parenteral, intranasal or dermal administration.

The antibodies can typically be administered for local application by injection (intraperitoneal or intracranial-typically in a cerebral ventricle-or intrapericardiac or intrabursal) of liquid formulations or by ingestion of solid formulations (in the form of pills, tablets, capsules) or of liquid formulations (in the form of emulsions and solutions). Compositions for parenteral administration commonly comprise a solution of immunoglobulin dissolved in a compatible, preferably aqueous solution. The concentration of the antibody/derivative in these formulations can vary from less than 0.005% to 15-20% w/v. It is selected mainly according to the volumes of the liquid, viscosity, etc, and according to the particular administration mode desired. Alternatively, the antibodies can be prepared for administration in solid form. The antibodies can be combined with different inert or excipient substances, which can include ligands such as microcrystalline cellulose, gelatin or Arabic rubber; recipients such lactose or starch; agents such as alginic acid, Primogel or corn starch; lubricants such as magnesium stearate, colloidal silicon dioxide; sweeteners such as saccharose or saccharin; or flavours, such as mint and methyl salicylate. Other pharmaceutical administration systems include hydrogel, hydroxymethylcellulose, liposomes, microcapsules, microemulsions, microspheres, etc.

Local injections directly at a site affected by a disorder /close thereto are a preferred mode of administration if a disorder is localised. The anti-TrkA antibodies are suitably administered systemically. Systemic administration can be performed by injection, e.g. continuous intravenous infusion, bolus intravenous infusion, subcutaneous or intramuscular injection.

Alternatively, other forms of administration (e.g. oral, mucosal, via inhalation, sublingually, etc.) may also be used. If desired, however, delivery of the antibody/ derivative can be performed by local administration (e.g. intra-articular injection or subcutaneous, intramuscular injection) in the vicinity of affected tissues.

The anti-TrkA antibody/derivative will suitably be formulated in a pharmaceutical composition appropriate for the intended route of administration. Solutions for injection will suitably contain the antibody/derivative dissolved or dispersed in an aqueous medium (e.g. water for injection) as appropriate containing appropriate buffers and molarity modifiers (e.g. phosphate, salt and/or dextrose).

The treatment regime (i.e. dose, timing and repetition), can be represented by single or repeated administrations (e.g. injections) of the product by the chosen administration route. The interval of dose administration can be subject to modifications depending on the extent and duration of the clinical response, as well as the particular individual and the individual clinical history.

Suitably the anti-TrkA antibody/derivative has a long duration of action. In particular the clinical effect of the antibody extends following administration may be as long as 21 days as determined from animal studies. Furthermore, anti-TrkA antibodies may manifest clinical benefit for a longer period than that in which its presence can be detected in a relevant biological matrix such as serum or plasma following its administration.

In light of the intended long duration of action (i.e. an effect suitably lasting at least one week, or preferably at least two weeks e.g. at least three weeks or at least four weeks), suitably the antibody/derivative may be administered to subjects at a frequency of not more than once per week e.g. not more than once per two weeks or once per three weeks or once per four weeks.

A suitable daily dose of the anti-TrkA antibody/derivative will typically range from 0.1 mg/kg to 10 mg/kg body weight. A suitable dose of the anti-TrkA antibody for the treatment of acute and chronic inflammatory pain is at least 0.01mg/kg (see Examples 2 and 3). A suitable dose of the anti-TrkA antibody for the treatment of osteoarthritic pain is at least 0.01mg/kg (see Example 4). A suitable dose of the anti-TrkA antibody for the treatment of neuropathic pain is at least 0.01 mg/kg, preferably 0.1 mg/kg and most preferably 1 mg/kg (see Example 5). These doses relate to an *in vivo* condition in mice only. The present invention relates the use of a humanized anti-TrkA antibody in the treatment of acute inflammatory pain, wherein hyperalgesia is effectively reversed to the same extent as that observed with administration of a NSAID. The present invention also relates to the use of a humanized anti-TrkA antibody in the treatment of chronic inflammatory pain, wherein hyperalgesia is effectively reversed to the same extent as that observed with administration of a NSAID. The present invention also relates to the use of a humanized anti-TrkA antibody in the treatment of osteoarthritic pain, wherein hyperalgesia is effectively reversed to the same extent as that observed with administration of pregabalin and an opiate. The present invention also relates to the use of a humanized anti-TrkA antibody in the treatment of neuropathic pain, wherein hyperalgesia is effectively reversed to the same extent as that observed with administration of pregabalin. Turning now to administration specifically in respect of tumours, administration may be through direct and localized injection into a tumour or a tissue near the tumour site. For systemic administration, doses vary from 0.05 mg/kg per day to 500 mg/kg per day, although dosages in the lower region of the range are preferred because they are easier to administer. Dosages can be calibrated for example to guarantee a particular level in the plasma of the antibody/derivative (in the range of about 5-30 mg/ml, preferably between 10-15 mg/ml) and maintain this level for a given period of time until the clinical results are achieved.

Effective methods for measuring or assessing the stage of pancreatic or prostatic tumours are based on the measurement of the prostate specific antigen (PSA) in blood, on the measurement of the survival time for pancreas tumours, on the measurement of the slowing or inhibition of diffusion for metastases in the case of both tumour types.

For direct injection at the level of a tumour site, dosage depends on different factors including the type, stage and volume of the tumour, along with many other variables.

Depending on tumour volume, typical therapeutic doses may vary from 0.01 mg/ml and 10 mg/ml injections which can be administered with the necessary frequency.

Whatever the nature of the therapy, humanised antibodies may be eliminated much more slowly and require lower dosages to maintain an effective level in the plasma than non-humanised antibodies. Moreover, with high affinity antibodies, administration may be less frequent and less sizable than with antibodies having lower affinity.

The therapeutically effective dosage of each antibody/ derivative can be determined during the treatment by a skilled medical practitioner. If necessary, dosages can be reduced (e.g. to reduce side effects) or increased (to increase therapeutic effects).

Prior to administration, preparations of antibodies of the invention can be stored by being frozen or lyophilized. They may then be reconstituted immediately before use in a suitable buffer. Given that lyophilisation and reconstitution can result in a loss in activity, antibody administration levels can be calibrated to compensate for this fact. (For conventional immunoglobulins, IgM antibodies tend to have a greater loss of activity than IgG antibodies.)

A shelf life may also be assigned so that antibodies are not used after a certain period of storage.

An antibody or derivative thereof of the present invention can be used in the diagnosis or prognosis of any of the diseases/ conditions discussed above in relation to medical uses. For example it may be used to facilitate detection of TrkA positive tumour markers, as a precocious marker of the insurgence of Alzheimer's disease, etc.

It may also be used in the diagnosis of CIPA ("congenital insensitivity to pain with anhydrosis"). This is a hereditary, recessive, autosomal syndrome characterised by recurrent episodic fever, anhydrosis, the absence of reaction to nociceptive stimuli, mental retardation and a tendency to self-mutilation. It results from mutations in the TrkA gene. Indeed an antibody or derivative of the present invention may be used in the diagnosis or prognosis of a wide range of conditions involving aberrant expression of TrkA (compared to expression of TrkA in a healthy individual or a healthy tissue sample) or an aberrant activity involving TrkA. The present invention therefore includes within its scope a method comprising obtaining a biological sample obtained from a patient and contacting the sample with an antibody or derivative of the present invention. If desired, the antibody/derivative may be immobilised. The method may then include assaying the binding of the antibody / derivative to said sample in a quantitative or qualitative manner. If desired, this may be done with reference and/or to a positive control (indicating a healthy state) or a negative control (indicating the presence/likelihood of a disorder). For diagnostic purposes, the antibodies can be both marked with a detectable marker or can be unmarked. (The term "marker" is used herein to include labels or any other detectable moiety/moiety that can trigger a detectable change.)

### Article of manufacture and kit

In another embodiment of the disclosure, an article of manufacture comprising the antibody or fragment thereof, the composition or the immunoconjugate of the invention for the treatment of one or more of the above mentioned diseases/conditions. The article of manufacture may comprise a container and a label or package insert on or associated with the container. Suitable containers include, for example, bottles, vials or syringes. The containers may be formed from a variety of materials such as glass or plastic. The container holds a composition that may be effective for treating the condition and may have a sterile access port (e.g., the container may be an intravenous solution bag or a vial having a stopper pierceable by a hypodermic injection needle). At least one active agent in the composition may be the antibody described herein. The label or package insert may indicate that the composition may be used for treating the condition of choice.

Moreover, the article of manufacture may comprise (a) a first container with a composition contained therein, wherein the composition comprises the antibody herein, and (b) a second container with a composition contained therein, wherein the composition comprises a therapeutic agent other than the antibody. The article of manufacture in this embodiment of the disclosure may further comprise a package insert indicating that the first and second compositions can be used in combination. Such therapeutic agent may be any of the adjunct therapies described in the preceding section (e.g., a thrombolytic agent, an antiplatelet agent, a chemotherapeutic agent, an anti-angiogenic agent, an anti-hormonal compound, a cardioprotectant, and/or a regulator of immune function in a mammal, including a cytokine). Alternatively, or additionally, the article of manufacture may further comprise a second (or third) container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Also within the scope of the present invention are kits comprising the antibody, the compositions or the immunoconjugates of the invention and instructions for use. The kit can further contain one or more additional reagents, such as an immunosuppressive reagent, a cytotoxic agent or a radiotoxic agent, or one or more additional antibodies of the invention (e.g., an antibody having a complementary activity which binds to an epitope in the TrkA antigen distinct from the first antibody).

Without further description, it is believed that one of ordinary skill in the art may, using the preceding description and the following illustrative examples, make and utilize the agents of the present disclosure and practice the claimed methods. The following working examples are provided to facilitate the practice of the present disclosure and are not to be construed as limiting in any way the remainder of the disclosure.

### Examples

The humanized anti-TrkA antibodies described herein represent a novel subgroup of anti-TrkA antibodies, which are highly effective at inhibiting the functional activation of TrkA. The anti-TrkA mouse monoclonal antibody known as MNAC13 disclosed in WO00/73344, and its humanized variants disclosed in WO09/098238 do not achieve the same level of inhibition, which is desired under certain circumstances, e.g. for the treatment of pain and associated cancer pain, where a high level of inhibition can lead to increased efficacy of treatment. Thus, it would be desirable to develop humanized anti-TrkA antibodies that would exhibit inhibition of TrkA to a high degree.

Methods to assess antibody mediated inhibition of the functional activation of TrkA are well known in the art and include TF-1 cell proliferation assays, wherein the ability of antibodies to block cell surface TrkA/beta-NGF mediated cell proliferation is assayed using the factor dependent human erythroleukemic cell line TF-1 (Kitamura T et al,. (1989) J. Cellular Physiology 140(2):323-34). Humanized antibodies disclosed in WO09/098238 have demonstrated inhibitory activity in TF-1 proliferation assays with the BXhVH5VL1 candidate having the highest degree of inhibition amongst humanized variants of the MNAC13 mouse antibody. In addition humanized antibodies disclosed in WO09/098238 have demonstrated direct binding to the extracellular region of TrkA using Surface Plasmon Resonance (SPR) technique. Hence, to design new humanized variants of the mouse MNAC 13 antibody with improved binding affinities to human TrkA and more importantly improved inhibition potencies in TF-1 cell proliferation assays over the BXhVH5VL1 humanized variant, a selected subset of the humanized antibodies disclosed in WO09/098238 was used as a starting point for engineering.

The letters following "BXh" are VH or VL or both. VH or VL indicates a heavy chain having IGHG1 isotype with a specific heavy chain variable domain denoted by a specific number e.g. BXhVH5, or a light chain having kappa isotype with a specific light chain variable domain denoted by a specific number e.g. BXhVL1, respectively. When both a VH with a specific number as well as a VL with a specific number are presented after "BXh" e.g. BXhVH5VL1, they indicate a specific antibody molecule consisting of a specific combination of heavy and light chains e.g. BXhVH5VL1 antibody refers to an antibody having BXhVH5 heavy chain and BXhVL1 light chain. Newly engineered humanized variants based on the selected subset of VH and VL variable domains described in WO09/098238 were termed "GBR" antibodies. Conversely, the same nomenclature system is in force with regard to the GBR antibodies. Specific substitutions made within variable heavy chain domains as used herein are further indicated in brackets. All GBR antibodies were of IGHG1 heavy chain isotype with kappa isotype light chains except as otherwise stated.

### Example 1: Antibody Engineering

Out of the forty antibodies disclosed in WO09/098238, five humanized antibodies were selected as inputs for engineering: BXhVHIVL1 having heavy chain variable domain VH1 (SEQ ID NO: 1) and light chain variable domain VL1 (SEQ ID NO: 6), BXhVH3VL1 having heavy chain variable domain VH3 (SEQ ID NO: 3) and light chain variable domain VL1, BXhVH3VL3 having heavy chain variable domain VH3 and light chain variable domain VL3 (SEQ ID NO: 8), BXhVH5VL1 having heavy chain variable domain VH5 (SEQ ID NO: 5) and light chain variable domain VL1, and BXhVH5VL3 having heavy chain variable domain VH5 and light chain variable domain VL1. MNAC13 mouse antibody as used herein has heavy chain variable domain MNAC13 VH with SEQ ID NO: 20, heavy chain with SEQ ID NO 21, and light chain with SEQ ID NO: 22.

Surprisingly, all engineered antibodies benefited from a simple change of valine to alanine (mouse back-mutation) in the heavy chain variable domain at position 37 (Kabat numbering (Kabat EA *et al, ibid.).* This substitution had the unique property of broadly enhancing the affinity for human TrkA amongst all engineered antibodies as measured by SPR. More importantly, the same substitution led to a potency increase in TF-1 cell proliferation assays for most variants, which unexpectedly when introduced in the VH5 domain paired with the VL1 domain (GBR VH5 (V37A)VL1 antibody) was a ten-fold increase in comparison to the BXhVH5VL1 humanized antibody. Notably, the same GBR VH5(V37A)VL1 antibody also exhibited a three-fold potency increase in TF-1 cell proliferation assays when compared to the MNAC13 mouse antibody.

Since an increase in the number of mouse-derived residues in humanized antibodies can increase their risk of immunogenicity (Harding FA et al., (2010) MAbs 2(3):256-65), further investigation was made to reduce the number of mouse residues in the engineered antibodies. The change of mouse residue lysine at position 3 for a glutamine in the GBR VH5(V37A)VL1 antibody resulted in the GBR VH5(K3Q,V37A)VL1 antibody having the same number of mouse residues compared to the BXhVH5VL1 humanized antibody, but with increased binding affinity, equivalent FAB thermo-stability to the parental mouse MNAC13 antibody, and increased inhibitory potency.

Finally, since antibody-mediated effector functions can be detrimental to therapies wherein only TrkA blockade is required, it is desirable to develop anti-TrkA antibodies that would inhibit TrkA without mediating the killing of TrkA expressing cells by immune cells. The human IGHG4 isotype does not carry effector functions such as ADCC or CDC, and as such is a particularly well suited antibody format when effector functions are not required or detrimental to therapy. However, one drawback of the naturally occurring human IGHG4 isotype is known as the "FAB exchange" phenomenon by which naturally occurring human IGHG4 antibodies are known to exchange heavy chains *in vivo* (Labrijn AF et al., (2009) Nat. Biotechnol. 27(8):767-71). Methods to block heavy chain exchange between recombinant and endogenous human IGHG4s are known in the art and the exchange is known to be efficiently blocked by substitution of the serine residue at position 228 (EU numbering, Edelman GM *et al*., *ibid.)* located in the hinge region for a proline residue, thereby mimicking the conformational hinge structure found in human IGHG1 isotype (Lewis KB et al., (2009) Mol. Immunol. 46(16):3488-94). GBR VH5(V37A)VL1 and GBR VH5(K3Q,V37A)VL1 were both formatted as IGHG4 S228P immunoglobulins for the aforementioned therapeutic purpose and showed equivalent potency to their IGHG1 isotype counterpart, thereby making both GBR VH5(V37A)VL1 IGHG4 S228P and GBR VH5(K3Q,V37A)VL1 IGHG4 S228P antibodies suitable for human therapies wherein only TrkA blockade is required.

### Methods

### Design of engineered variants

A 3D model for the VH5-VL1 pair of variable domains was calculated using the structure homology-modelling server SWISS-MODEL (Arnold K et al., (2006) Bioinformatics 22(2):195-201; http://swissmodel.expasy.org) set in automated mode. The retrieved model template was the experimentally solved MNAC13 FAB 3D structure (PDB code 1SEQ, www.pdb.org, Berman HM et al., (2000) Nucleic Acids Res. 28(1):235-42, Covaceuszach S et al., (2005) Proteins 58(3):717-27). Sequence alignment of MNAC13 VH, VH1, VH3, VH5 and germline VH3-023*01 (SEQ ID NO: 23) domains showed different amino acid content at positions: 3, 5, 19, 37, 40, 42, 44, 49, 50, 52A, 53, 60, 62, 74, 82A, 83, 84, 89, and 94 (Kabat numbering). Model analysis allowed the selection of a subset of positions based on their putative influence on CDR regions and/or heavy chain-light chain variable domain packing. This subset of positions consisted of: 3, 37, 40, 42, 44, 49, 50, 60, 62, 89, and 94 (Kabat numbering). Hence engineered antibodies had heavy chain variable domains encompassing substitutions at single or multiple positions in the aforementioned variable domain sequences (VH1, VH3, and VH5 with SEQ ID NO: 1, 3, 5, respectively) selected from the subset of positions described above. More specifically, engineered antibodies consisted of the substituted heavy chain variable domains as described above paired with one of the two previously mentioned light chain variable domains VL1 or VL3 (SEQ ID NO 6 and 8).

### Molecular Biology

Engineered heavy chain variable domain coding DNA sequences (cDNAs) were created by standard mutagenesis techniques using the vector DNAs for BXhVH1, BXhVH3, and BXhVH5 described in WO 09/098238 as PCR templates. Similarly, light chain variable domain cDNAs were directly amplified from the vector DNAs for BXhVL1 and BXhVL3 described in WO 09/098238. Variable domain cDNAs were further assembled upstream of their respective constant domain cDNA sequences using PCR assembly techniques. Finally, the complete heavy and light chain cDNAs were ligated in independent vectors that are based on a modified pcDNA3.1 vector (Invitrogen, CA, USA) carrying the CMV promoter and a Bovine Growth Hormone poly-adenylation signal. The light chain specific vector allowed expression of kappa isotype light chains by ligation of the light chain variable domain cDNA of interest in front of the kappa light chain constant domain cDNA using BamHI and BsiWI restriction enzyme sites; while the heavy chain specific vector was engineered to allow ligation of the heavy chain variable domain cDNA of interest in front of the cDNA sequence encoding the IGHG1 CH1, IGHG1 hinge region, IGHG1 CH2, and IGHG1 CH3 constant domains using BamHI and SalI restriction enzyme sites. In both heavy and light chain expression vectors, secretion was driven by the murine VJ2C leader peptide containing the BamHI site. The BsiWI restriction enzyme site is located in the kappa constant domain; whereas the SalI restriction enzyme site is found in the IGHG1 CH1 domain.

IGHG4 immunoglobulin formatting having substitution S228P was achieved by replacing the cDNA sequence encoding the IGHG1 CH1, IGHG1 hinge region, IGHG1 CH2, and IGHG1 CH3 constant domains for a cDNA sequence encoding the IGHG4 CH1, IGHG4 hinge region having S228P substitution, IGHG4 CH2, and IGHG4 CH3 constant domains in the heavy chain specific vector described above. Substitution S228P was introduced in a human IGHG4 heavy chain cDNA template by standard PCR mutagenesis techniques.

### Antibody Production

For transient expression of antibodies, equal quantities of heavy and light chains vectors were co-transfected into suspension-adapted HEK293-EBNA1 cells (ATCC® catalogue number: CRL-10852) using polyethylenimine (jetPEI®, Polyplus transfection, Illkirch Cedex, France). Typically, 100 ml of cells in suspension at a density of 0.8-1.2 million cells per ml is transfected with a DNA-jetPEI® mixture containing 50 µg of expression vector encoding the heavy chain and 50 µg of expression vector encoding the light chain. When recombinant expression vectors encoding antibody genes are introduced into the host cells, antibodies are produced by further culturing the cells for a period of 4 to 5 days to allow for secretion into the culture medium (EX-CELL 293, HEK293-serum-free medium; Sigma, Buchs, Switzerland), supplemented with 0.1% pluronic acid, 4 mM glutamine, and 0.25 µg/ml geneticin).

Antibodies were purified from cell-free supernatant using recombinant protein-A streamline media (GE Healthcare Europe GmbH, Glattbrugg, Switzerland), and buffered exchanged into phosphate buffer saline prior to assays.

### Stability testing using differential scanning calorimetry

Calorimetric measurements were carried out on a VP-DSC differential scanning microcalorimeter (GE Healthcare Europe GmbH, Glattbrugg, Switzerland). The cell volume was 0.128 ml, the heating rate was 200°C/h, and the excess pressure was kept at 65 p.s.i. All antibodies were used at a concentration of 1 mg/ml in PBS (pH 7.4). The molar heat capacity of each protein was estimated by comparison with duplicate samples containing identical buffer from which the protein had been omitted. The partial molar heat capacities and melting curves were analyzed using standard procedures. Thermograms were baseline-corrected and concentration-normalized before being further analyzed using a Non-Two State model in the Origin software (v7.0, GE Healthcare Europe GmbH, Glattbrugg, Switzerland).

### Affinity measurements by SPR

SPR analysis was used to measure the association and dissociation rate constants for the binding kinetics of the different antibodies (mouse and humanized antibodies). The binding kinetics of the mouse antibody and humanized variants were measured on a BIACORE 2000 instrument (GE Healthcare Europe GmbH, Glattbrugg, Switzerland) at room temperature, and analyzed with the BiaEvaluation software (v4.1, GE Healthcare Europe GmbH, Glattbrugg, Switzerland).

Since antibodies are bivalent molecules, it is best to immobilize antibodies onto the sensor chip. If antibodies are used as analytes, SPR measurements will bear a valency component in addition to affinity. Although the BIAcore evaluation software can model bivalency and extract affinity constants, it is preferable to circumvent any valency bias by working with a monovalent analyte whenever possible. To this aim, a monovalent form of the human TrkA extracellular region was produced by digestion of a recombinant human TrkA-Fc fusion protein (SEQ ID NO: 24) made in HEK293-EBNA1 cells. The fusion protein consisted of the human TrkA extracellular region (SEQ ID NO: 25) fused to an IGHG1 Fc region wherein a protease-specific cleavage sequence was included between the two regions (TEV cleavage protease amino acid sequence: ENLYFQS). Following protease cleavage, the monovalent form of the human TrkA extracellular region was further purified to homogeneity using standard chromatographic techniques which included a protein-A step to remove Fc fragments. Finally, the monovalent human TrkA extracellular region protein was buffer-exchanged into PBS before being diluted in Biacore running buffer for affinity measurements. Sample purity, homogeneity and molecular weight were confirmed by SDS-PAGE and size-exclusion analysis.

Antibodies were immobilized via capture of their Fc portion to allow for correct orientation on the sensor chip surface. A monoclonal mouse anti-human IgG (Fc) antibody sensor chip was used to capture all humanized antibodies regardless of their isotypes (Human Antibody Capture Kit, catalogue number BR-1008-39, GE Healthcare Europe GmbH), and a polyclonal rabbit anti-mouse immunoglobulin sensor chip was used to capture the MNAC13 mouse antibody (Mouse Antibody Capture Kit, catalogue number BR-1008-38, GE Healthcare Europe GmbH).

Data (sensorgram: fc2-fc1) were fitted with a 1:1 Langmuir model with mass transfer in spite of having little mass transfer limitation in mass transfer tests. To account for the experimental variations in captured antibody at the beginning of each measurement, the Rmax value was set to local in all fits. Dissociation times were of at least 350 seconds. Measurements were performed in triplicate, and included zero-concentration samples for referencing. Both Chi2 and residual values were used to evaluate the quality of a fit between the experimental data and individual binding models.

### Proliferation assay with TF-1 cells

Suspension adapted TF-1 cells (ATCC® number: CRL-2003) were grown in complete RPMI medium containing 10% FCS, and 5 ng/ml of rhuβNGF (recombinant human beta-NGF, R&D Systems Europe Ltd, Abingdon, UK). For the assay, TF-1 cells were incubated for 5h in complete RPMI without human beta-NGF. Following this starvation step, cells were centrifuged and seeded in flat-bottomed 96 well plates with various concentrations of each antibody and a fixed concentration of recombinant beta-NGF. TF-1 cells were seeded at a density of 7000 cells/well and the total antibody-cell mixture volume was 200 µl with 5 ng/ml of human beta-NGF (final concentration). This mixture was incubated for 4 days at 37°C with CO₂ supplementation. At day 3, the colorimetric dye Alamar blue (AbD Serotec, Morphosys AbD GmbH, Düsseldorf, Germany) was added to each well without any change to the incubation conditions. At day 4, fluorescence was read on a Bio-Tek Synergy™ 2 spectrophotometer/microplate reader (BioTek Instruments GmbH, Luzern, Switzerland) with a 530-to-560 nm excitation wavelength and a 590 nm emission wavelength. Experiments were performed at least twice and measurements for each antibody concentration were done in triplicates.

### Results

### Engineering new humanized anti-TrkA antibodies based on BXhVH5VL1

Based on a 3D model for the VH5-VL1 pair of variable domains, a subset of common 3D positions within the different heavy chain variable domains (VH1, VH3, and VH5) were selected for mouse-to-human as well as human-to-mouse mutations; this group consisted of positions: 3, 37, 40, 42, 44, 49, 50, 60, 62, 89, and 94 (Kabat numbering). The engineering strategy with regard to the combination of substitutions as used herein was based on the complementarity of the different substitutions in terms of on their putative influence on CDR regions and/or variable domain packing and/or immunogenicity.

In a first approach mouse-to-human and human-to-mouse mutations were engineered in the BXhVH5VL1 candidate in the context of an IGHG1 isotype format.
The mouse-to-human mutations undertaken included the following substitutions: K3Q, T40A, R44G, A49S, Y50A, P60A, T62S, and R94K. Production yields and affinities of some of the engineered anti-TrkA antibodies based on these single or combination of substitutions are shown in Table 1 and 2, respectively. Combining most or all mouse-to-human mutations led to poor production yields and complete loss of binding to TrkA. For example, mouse-to-human substitution A49S combined with Y50A induced a complete abrogation of binding, which was not rescued by the other mouse-to-human substitutions and mouse-to-human substitutions K3Q, T40A, R44G, and R94K did not lead to any improvement in affinity either alone or in combination with other mouse-to-human substitutions.

The human-to-mouse mutations undertaken included the following substitutions: V37A, G42E, and V89L. Human-to-mouse substitution V37A had the most positive impact and led to at least a two-fold increase in affinity (K_{D} is decreased by at least two-fold) (Table 2 and FIG. 1); a 2.5 fold increase in affinity was recorded for the GBR VH5(V37A)VL1 IGHG1 antibody . This increase in affinity was abrogated when combining human-to-mouse substitution V37A with mouse-to-human substitutions P60A and T62S, but maintained when making combinations with mouse-to-human substitutions K3Q and/or R44G.
Taken together these results identified the importance of positions 49 and 50 to allow binding and the unique property of the human-to-mouse substitution V37A in enhancing the affinity of BXhVH5VL1 by at least two-fold. Since antibody-mediated effector functions can be detrimental to therapies wherein only TrkA blockade is required, the GBR VH5(V37A)VL1 and the GBR VH5(K3Q,V37A)VL1 were both reformatted in the aforementioned IGHG4 isotype having the S228P substitution. Both engineered antibodies showed similar affinities to their IGHG1 isotype counterparts, indicating that the affinity improvement brought by the V37A substitution was not affected by the isotype switch.

### Engineering new humanized anti-TrkA antibodies based on BXhVH1VL1, BXhVH3VL1 BXhVH3VL1 and BXhVH5VL3

In a second approach, the V37A substitution was introduced in the other selected candidates originating from WO 09/098238, and the affinities of the engineered anti-TrkA antibodies benchmarked against BXhVH5VL1 and MNAC13 antibodies by SPR. Table 3 and 4 show, respectively, the production yields and affinities of the V37A substituted antibodies. Although engineered antibodies had differences in their production yields, all engineered antibodies exhibited an enhancement in affinity upon introduction of the V37A substitution. Affinities were improved by 8, 20, 30, and 55%, for BXhVH3VL1, BXhVH1VL1, BXhVH3VL3, and BXhVH5VL3, respectively. Thus, the human-to-mouse substitution V37A did not only lead to an increase in affinity when introduced in BXhVH5VL1 but, and most unexpectedly also led to an increase in affinity amongst all humanized variants. Notably, GBR VH5(V37A)VL1 or GBR VH5(K3Q,V37A)VL1 both as IGHG1 or IGHG4 S228P isotype had affinities matching the affinity measured for the MNAC13 mouse antibody (Tables 2 and 4); both being increased by at least two-fold when compared to the BXhVH5VL1 antibody (K_{D} is decrease by 2.5 to 2.7 fold, respectively).

Table 3 and FIG.2 includes the melting temperatures of the FAB portions measured within the engineered antibodies. Monoclonal antibodies melting profiles are characteristic of their isotypes but the mid-point melting temperature (Tm) of the FAB fragment can be easily identified even in the context of a full-length immunoglobulin (Garber E and Demarest SJ (2007) Biochem. Biophys. Res. Commun. 355(3):751-7). Such mid-point melting of the FAB portion was used to monitor the stability of the engineered candidates. GBR VH5(V37A)VL1 and GBR VH5(K3Q,V37A)VL1 FAB fragments each displayed a single transition at 73.6 and 73°C respectively, both having equivalent thermo-stability with a shape and an amplitude of the FAB transition consistent with a cooperative unfolding which is generally observed for a compactly folded FAB fragment indicating that the engineering process was successful at retaining FAB stability. This is further exemplified, when comparing the FAB Tm of GBR VH5(V37A)VL1 or GBR VH5(K3Q,V37A)VL1 with the MNAC13 FAB Tm (74°C), the differences being within 1°C or less.

### Functional testing of engineered anti-TrkA antibodies

Engineered anti-TrkA antibodies were assayed for their ability to inhibit the functional activation of TrkA in TF-1 cell proliferation assays (FIG. 3). The half maximal inhibitory concentration (IC50) which is a measure of the effectiveness of a compound in inhibiting biological function was calculated for each curve and the results are given in Table 5. Note that GBR VH1(V37A)VL1 was not assayed due to its lower affinity by SRP.

It was found that GBR VH5(V37A)VL1 was the best performer followed by GBR VH5(K3Q, V37A)VL1, GBR VH5(K3Q,V37A)VL1 IGHG4 S228P, and GBR VH3(V37A)VL1 which had identical IC50s. GBR VH3(V37A)VL3 and GBR VH5(V37A)VL3 antibodies had slightly higher IC50s but all tested engineered antibodies performed better than BXhVH5VL1, GBR VH5(V37A)VL1 being ten-fold better. Notably, the GBR VH5(V37A)VL1 antibody also exhibited a three-fold potency increase in TF-1 cell proliferation assays when compared to the MNAC13 mouse antibody. All V37A variants had equivalent or better IC50 when compared to the mouse parental antibody.

**Table 1: production yields from a selection of engineered anti-TrkA antibodies based on BXhVH5VL1; all antibodies are of the IGHG1 isotype except as otherwise stated. (*) IGHG4 isotype with S228P substitution. ⁺ Includes mouse-to-human and human-to-mouse mutations as described in Example 1.**

| **Humanized anti-TrkA antibodies** | **SEQ ID NOs** | **Number of mutations⁺** | **Transient expression (mg/l)** |
|---|---|---|---|
| BXhVH5VL1 | 28,29 | 8 | 10 |
| GBR VH5(K3Q)VL1 | 50,29 | 7 | 16 |
| GBR VH5(V37A)VL1 | 51,29 | 9 | 19 |
| GBR VH5(V37A)VL1 (*) | 52,29 | 9 | 11 |
| GBR VH5(G42E)VL1 | 53,29 | 9 | 19 |
| GBR VH5(V89L)VL1 | 54,29 | 9 | 17 |
| GBR VH5(R94K)VL1 | 55,29 | 7 | 15 |
| GBR VH5(K3Q,V37A)VL1 | 56,29 | 8 | 52 |
| GBR VH5(K3Q,V37A)VL1 (*) | 57,29 | 8 | 11 |
| GBR VH5(K3Q,T40A)VL1 | 58,29 | 6 | 3 |
| GBR VH5(P60A,T62S)VL1 | 59,29 | 6 | 7 |
| GBR VH5(K3Q,V37A,R44G)VL1 | 60,29 | 7 | 24 |
| GBR VH5(K3Q,A49S,Y50A)VL1 | 61,29 | 5 | 15 |
| GBR VH5(K3Q,P60A,T62S)VL1 | 62,29 | 5 | 7 |
| GBR VH5(K3Q,T40A,P60A,T62S)VL1 | 63,29 | 4 | 8 |
| GBR VH5(K3Q,V37A,T40A,P60A,T62S)VL1 | 64,29 | 5 | 12 |
| GBR VH5(K3Q,T40A,R44G,A49S,Y50A)VL1 | 65,29 | 3 | 22 |
| GBR VH5(K3Q,A49S,Y50A,P60A,T62S)VL1 | 66,29 | 3 | 16 |
| GBR VH5(K3Q,T40A,R44G,A49S,Y50A,P60A,T62S)VL1 | 67,29 | 1 | 3 |
| GBR VH5(K3Q,T40A,R44G,A49S,Y50A,P60A,T62S,R94K)VL1 | 68,29 | 0 | 3 |

**Table 2: affinities from a selection of engineered anti-TrkA antibodies based on BXhVH5VL1; all antibodies are of the IGHG1 isotype except as otherwise stated. (*) IGHG4 isotype with S228P substitution. N.B. indicates no binding.**

| **Humanized anti**-**TrkA antibodies** | **K_{D} (nM)** |
|---|---|
| BXhVH5VL1 | 40.9 |
| GBR VH5(V37A)VL1 | 16 |
| GBR VH5(V37A)VL1 (*) | 15 |
| GBR VH5(R94K)VL1 | 52 |
| GBR VH5(K3Q,V37A)VL1 | 15.4 |
| GBR VH5(K3Q,V37A)VL1 (*) | 15 |
| GBR VH5(K3Q,V37A,R44G)VL1 | 20 |
| GBR VH5(K3Q,A49S,Y50A)VL1 | N.B. |
| GBR VH5(K3Q,V37A,T40A,P60A,T62S)VL1 | 48 |
| GBR VH5(K3Q,T40A,R44G,A49S,Y50A)VL1 | N.B. |
| GBR VH5(K3Q,A49S,Y50A,P60A,T62S)VL1 | N.B. |
| GBR VH5(K3Q,T40A,R44G,A49S,Y50A,P60A,T62S)VL1 | N.B. |
| GBR VH5(K3Q,T40A,R44G,A49S,Y50A,P60A,T62S,R94K)VL1 | N.B. |

**Table 3: production yields and FAB thermo-stability data from a selection of engineered anti-TrkA antibodies; all antibodies are of the IGHG1 isotype.**

| **Anti-TrkA antibodies** | **SEQ ID NOs** | **Expression (mg/l)** | **Tm Fab (°C)** |
|---|---|---|---|
| MNAC13 | 21,22 | 5 | 74 |
| BXhVH1VL1 | 26,29 | 10 | 64.7 |
| GBR VH1(V37A)VL1 | 69,29 | 16 | 65.3 |
| BXhVH3VL1 | 27,29 | 9 | 71.3 |
| GBR VH3(V37A)VL1 | 70,29 | 1 | 70.2 |
| BXhVH3VL3 | 27,30 | 6 | 71 |
| GBR VH3(V37A)VL3 | 70,30 | 9 | 69.3 |
| BXhVH5VL1 | 28,29 | 16 | 76.5 |
| GBR VH5(V37A)VL1 | 51,29 | 19 | 73.6 |
| GBR VH5(K3Q,V37A)VL1 | 56,29 | 52 | 73 |
| BXhVH5VL3 | 28,30 | 16 | 76.4 |
| GBR VH5(V37A)VL3 | 51,30 | 19 | 73.3 |

**Table 4: affinities of V37A engineered anti-TrkA antibodies; all antibodies are of the IGHG1 isotype.**

| **Anti**-**TrkA antibodies** | **kₒₙ (1/Ms)** | **k_{off} (1/s)** | **K_{D} (nM)** |
|---|---|---|---|
| MNAC13 | 6.02x10⁴ | 1.09x10⁻³ | 18 |
| BXhVH1VL1 | 3.13x10⁴ | 1.33x10⁻² | 426 |
| GBR VH1(V37A)VL1 | 1.96x10⁴ | 6.63x10⁻³ | 338 |
| BXhVH3VL1 | 4.65x10⁴ | 6.19x10⁻³ | 133 |
| GBR VH3(V37A)VL1 | 3.82x10⁴ | 4.71x10⁻³ | 123 |
| BXhVH3VL3 | 3.14x10⁴ | 4.59x10⁻³ | 134 |
| GBR VH3(V37A)VL3 | 3.1x10⁴ | 2.95x10⁻³ | 95 |
| BXhVH5VL1 | 1.04x10⁵ | 4.26x10⁻³ | 40.9 |
| GBR VH5(V37A)VL1 | 1.4x10⁵ | 2.25x10⁻³ | 16 |
| GBR VH5(K3Q,V37A)VL1 | 1.7x10⁵ | 2.62x10⁻³ | 15.4 |
| GBR VH5VL3 | 6.23x10⁴ | 3.95x10⁻³ | 63 |
| GBR VH5(V37A)VL3 | 7.45x10⁴ | 2.05x10⁻³ | 28 |

**Table 5: TF-1 cell proliferation assay IC50s for V37A engineered anti-TrkA antibodies; all antibodies are of the IGHG1 isotype except as otherwise stated. (*) IGHG4 isotype with S228P substitution. N.D. indicates not determined.**

| **Anti-TrkA antibodies** | **IC50 (µg/ml)** |
|---|---|
| MNAC13 | 0.23 |
| BXhVH5VL1 | 0.73 |
| GBR VH1(V37A)VL1 | N.D. |
| GBR VH3(V37A)VL1 | 0.15 |
| GBR VH3(V37A)VL3 | 0.29 |
| GBR VH5(V37A)VL1 | 0.075 |
| GBR VH5(K3Q,V37A)VL1 | 0.15 |
| GBR VH5(K3Q,V37A)VL1(*) | 0.15 |
| GBR VH5(V37A)VL3 | 0.22 |

### Example 2: Humanized anti-TrkA antibody reverses acute inflammatory hyperalgesia of the paw induced by intraplantar injection of CFA.

Intraplantar injection of Complete Freunds adjuvant (CFA) into one of the hind paws of mice causes acute inflammatory hyperalgesia that can be assessed using the weight bearing method. Naive mice normally distribute their body weight equally between their two hind paws. However, when the CFA injected hind paw is inflamed and painful, the weight is re-distributed to lessen that placed on the injected paw (ipsilateral) and increase that on the non-injected (contralateral) hind paw.

### Methods

Weight bearing through each hind limb was measured using an incapacitance tester (Linton Instruments, UK). Mice were placed in the incapacitance tester with the hind paws on separate sensors and the average force exerted by both hind limbs was recorded over 2 seconds. Naive AMB1 mice were acclimatized to the procedure room in their home cages, with food and water available *ad libitum.* The AMB1 mice were generated by replacement of exon 1 of mouse TrkA with that of its human counterpart and as such they exclusively express the human TrkA protein. Habituation to the incapacitance tester was performed over several days. Baseline weight bearing recordings were taken prior to induction of insult. Inflammatory hyperalgesia was induced by intraplantar injection of CFA (20ul of 1.5mg/ml solution) into the left hind paw. A pre-treatment weight bearing reading was taken to assess hyperalgesia 23 hours post-CFA. Animals were then ranked and randomised according to CFA window in a Latin square design. 24 hours post-CFA, animals were treated with a single i.p. injection of 0.1mg/kg isotype control i.p. or 0.0001, 0.001, 0.01 and 0.1mg/kg of the antibody GBR VH5(K3Q,V37A)VL1 IGHG4 S228P i.p. (10ml/kg dose volume). The non-selective NSAID indomethacin was used as a positive control at 10mg/kg p.o. (5ml/kg dose volume). Weight bearing readings were taken at 4, 8, 24, 48, 72, 96 and 120 hours post-antibody/drug treatment.

Behavioural assessments were performed blind. Weight bearing (g) readings were taken for both ipsilateral and contralateral hind paws and expressed as % weight bearing difference (% ipsi/contra). Data were analysed by comparing treatment groups to isotype control group at each time point. Statistical analysis was performed as a repeated measures ANOVA followed by Planned comparison test using InVivoStat, (p<0.05 considered significant).

### Results

Intraplantar injection of CFA induced marked hyperalgesia as detected 23 hrs post-CFA by a shift in weight bearing from the ipsilateral to the contralateral hind paw resulting in a drop in the % ipsi/contra ratio (Fig. 4). The isotype control treatment initiated at 24 hrs post-CFA showed no effect on the hyperalgesia. A significant reversal of the hyperalgesia was observed with 0.01 and 0.1mg/kg of the antibody GBR VH5(K3Q,V37A)VL1 IGHG4 S228P from 4-48 hours post-dose when compared to the isotype control. Doses of 0.001mg/kg of the antibody GBR VH5(K3Q,V37A)VL1 IGHG4 S228P or below were ineffective at reversing the hyperalgesia. Indomethacin (10mg/kg) showed a significant reversal of hyperalgesia at all time points tested. In conclusion, the antibody GBR VH5(K3Q,V37A)VL1 IGHG4 S228P gave a significant reversal of acute inflammatory hyperalgesia of the paw similar to indomethacin. Furthermore, the effective doses of GBR VH5(K3Q,V37A)VL1 IGHG4 S228P administered (0.01 and 0.1 mg/kg) are many fold less than the effective dose of indomethacin (10mg/kg), demonstrating that the anti-TrkA antibody can be administered at a much lower dose than the standard treatment for this condition.

### Example 3: Humanized anti-TrkA antibody reverses chronic inflammatory hyperalgesia of the knee joint induced by intra-articular injection of CFA

Intra-articular injection of CFA into one of the knee joints of the hind limbs of mice induces chronic inflammatory hyperalgesia that can be assessed using the weight bearing method. When the CFA injected joint is inflamed and painful, the weight is re-distributed to lessen that placed on the limb with the injected (ipsilateral) joint and increase that on the limb with the non-injected (contralateral) joint. The development of these signs in this animal model are believed to be clinically relevant as they reflect symptoms displayed by patients presenting chronic inflammatory joint pain associated with underlying conditions such as osteoarthritis and rheumatoid arthritis.

### Methods

Naive AMB1 mice were acclimatised to the procedure room in their home cages, with food and water available *ad libitum.* The AMB1 mice were generated by replacement of exon 1 of mouse TrkA with that of its human counterpart and as such they exclusively express the human TrkA protein. Habituation to the incapacitance tester was performed. Baseline weight bearing readings were taken immediately prior to CFA injection. Animals were anaesthetised using isoflurane and oxygen mixed 3:1 in sterile conditions. The knee area was shaved and cleaned with a dilute hibiscrub solution. The left knee was injected with 10µl of 10mg/ml CFA. Animals were allowed to recover in a warmed environment before returning to their home cage. Animals were assessed for development of inflammatory hyperalgesia using the weight bearing method at days 4, 7 and 10 post-CFA. Based on the day 10 post-CFA measurement, animals were ranked and randomised to treatment groups according to their CFA window. At 13 days post-CFA when the hyperalgesia was well-established, animals were treated with a single injection of either isotype control antibody at 10mg/kg i.p. or the antibody GBR VH5(K3Q,V37A)VL1 IGHG4 S228P at 0.01, 1 and 10mg/kg i.p. The COX-2 selective NSAID celecoxib was used as a positive control at 60mg/kg p.o. twice daily. For the antibody treated groups, weight bearing was measured at 4, 8, 24 and 96 hrs post-dosing. For the celecoxib treated group, weight bearing was measured at 1 and 8 hrs post-dosing on day 13 post-CFA and then at 1 hr post-dosing on days 14-17 post-CFA. Behavioural assessments were performed blind. Weight bearing (g) readings were taken for both ipsilateral and contralateral hind limbs and expressed as % weight bearing difference (% ipsi/contra). Data were analysed by comparing treatment groups to isotype control group at each time point. Statistical analysis was performed as a repeated measures ANOVA followed by Planned comparison test using InVivoStat, (p<0.05 considered significant).

### Results

Injection of CFA into the knee joint caused marked and long-lasting hyperalgesia from day 3 onwards as detected by a shift in weight bearing from the ipsilateral to the contralateral hind limb resulting in a drop in the % ipsi/contra ratio (Fig. 5). The isotype control antibody treatment had no effect on the hyperalgesia. A significant reversal of hyperalgesia was seen with celecoxib (60mg/kg) 1 hour post dose during the course of the treatment. A significant reversal of the hyperalgesia was observed with a single injection of 0.01, 1 and 10mg/kg of the antibody GBR VH5(K3Q,V37A)VL1 IGHG4 S228P from 4-72 hours post-dose, with only the highest two doses being significant at 96 hours when compared to the isotype control. In conclusion, a single dose of 0.01 mg/kg the antibody GBR VH5(K3Q,V37A)VL1 IGHG4 S228P or above gave a significant and sustained reversal of chronic inflammatory hyperalgesia of the knee joint similar to multiple dosings of celecoxib. Furthermore, only a single dose of 0.01 mg/kg of the antibody GBR VH5(K3Q,V37A)VL1 IGHG4 S228P was required to achieve an effect compared with multiple doses of celecoxib at 60 mg/kg indicating that the antibody GBR VH5(K3Q,V37A)VL1 IGHG4 S228P can be given at a much lower dose and less frequently than the NSAID celecoxib, which is the current standard of care.

### Example 4: Humanized anti-TrkA antibody reverses chronic osteoarthritic hyperalgesia of the knee joint induced by intra-articular injection of MIA

Intra-articular injection of monosodium iodoacetate (MIA) into the knee joint of mice leads to the development of chronic hyperalgesia associated with local joint inflammation coupled with cartilage degradation. As such, the hyperalgesia measured in the MIA model closely resembles osteoarthritic-associated pain. Furthermore, the cartilage degradation leads to chronic neuronal damage with neuropathic-like characteristics. Since pregabalin, an anticonvuldant drug is the recommended first-line drug in the treatment of neuropathic pain it is used here as a comparator compound. Tramadol, a weak µ-opioid receptor agonist, is increasingly being used for the treatment of OA because, in contrast to NSAIDs, tramadol does not produce gastrointestinal bleeding or renal problems nor does it affect articular cartilage. For this reason tramadol was used alongside pregabalin as a comparator to the antibody GBR VH5(K3Q,V37A)VL1 IGHG4 S228P.

### Methods

Naïve AMB1 mice were acclimatised to the procedure room in their home cages, with food and water available *ad libitum.* The AMB1 mice were generated by replacement of exon 1 of mouse TrkA with that of its human counterpart and as such they exclusively express the human TrkA protein. Habituation to the incapacitance tester was performed over several days. Base line weight bearing readings measurements were taken. Animals were anaesthetised using isoflurane and oxygen mixed 3:1 in sterile conditions. The knee area was shaved and cleaned with a dilute hibiscrub solution. MIA, 5µl of 100mg/ml (500µg) or saline (sham) was injected into the knee joint of the left hind leg. Animals were allowed to recover in a warmed environment, before returning to their home cage. On days 3 - 23 post-MIA, animals were assessed at regular intervals using weight bearing for development of knee joint hyperalgesia. On day 14 post-MIA, weight bearing measurements were taken and animals were ranked and randomised to treatment groups according to their MIA response window followed by treatment with a single i.p. injection of 1, 10, 100 µg/kg antibody GBR VH5(K3Q,V37A)VL1 IGHG4 S228P or 100 µg/kg isotype control antibody (5ml/kg dose volume). As a comparator control, animals were treated on day 14 post-MIA with tramadol at 10mg/kg or pregabalin at 30mg/kg p.o. (5ml/kg dose volume) followed by tramadol at 30mg/kg or pregabalin at 100mg/kg every other day on days 16-22 post-MIA (5ml/kg dose volume). All animals were assessed using weight bearing at 4, 8, and 24 hours post-dosing on day 14 post-MIA followed by every 24 hours for antibody treated groups and 1 and 24 hours post-dose for tramadol and pregabalin treated groups. Behavioural assessments were performed blind. Weight bearing (g) readings were taken for both ipsilateral and contralateral hind limbs and expressed as % weight bearing difference (% ipsi/contra). Data were analysed by comparing treatment groups to sham group at each time point (day 3-14 post-MIA), for effects of MIA on hyperalgesia. Post-dosing data were analysed by comparing treatment groups to the isotype control group at each time point. Statistical analysis was performed as a repeated measures ANOVA followed by Planned comparison test using InVivoStat, (p<0.05 considered significant).

### Results

Injection of MIA at 500µg into the knee joint caused a marked hyperalgesia from day 3 onwards as detected by a shift in weight bearing from the ipsilateral to the contralateral hind limb resulting in a drop in the % ipsi/contra ratio. A significant reversal of hyperalgesia was seen with a single injection of 10 and 100µg/kg of the antibody GBR VH5(K3Q,V37A)VL1 IGHG4 S228P from 4hrs - 7 days post-dose when compared to isotype control at each time point (Fig. 6A). The antibody GBR VH5(K3Q,V37A)VL1 IGHG4 S228P at 1µg/kg had no effect on the hyperalgesia. Tramadol (10mg/kg) and pregabalin (30mg/kg) had no effect at 1 hour post-dosing on day 14 post-MIA, but showed significant reversal of hyperalgesia by 4 hours post-dose (Fig. 6B). Due to lack of effect at 1 hour post-dosing on day 14 post-MIA, tramadol was increased to 30mg/kg and pregabalin was increased to 100mg/kg, which both showed significant reversal of hyperalgesia at 1 hour post-dose on day 16, 18 and 20 post-MIA. In conclusion, a single dose of 10µg/kg of the antibody GBR VH5(K3Q,V37A)VL1 IGHG4 S228P or above gave a significant and sustained reversal of chronic osteoarthritic hyperalgesia of the knee joint similar to multiple dosings of tramadol and pregabalin. Furthermore, only a single dose of the antibody GBR VH5(K3Q,V37A)VL1 IGHG4 S228P at 10µg/kg was required to achieve an effect compared with multiple doses of two drugs, tramadol and pregabalin, used at far higher doses (30mg/kg and 100mg/kg, respectively). Therefore the anti-TrkA antibody can be administered at a much lower dose and with a lower dosing frequency than pregabalin and tramadol.

### Example 5: Humanized anti-TrkA antibody reverses peripheral neuropathic hyperalgesia and allodynia induced by chronic constriction injury of the sciatic nerve

Peripheral neuropathic pain is a chronic form of pain arising from injury, dysfunction or disease of the peripheral nervous system. It typically comprises hyperalgesia (heightened response to painful stimuli) as well as allodynia (painful response to non-painful stimuli). Neuropathic pain can be induced in animals by experimental injury of peripheral nerves and this is typically achieved by chronic constriction injury (CCI) of the sciatic nerve via loose ligation (Bennett & Xie (1998) Pain, 33: 87- 107). Since pregabalin is the recommended first-line drug in the treatment of neuropathic pain it is used here as a comparator compound.

### Methods

CCI surgery was performed in male and female AMB1 mice under ketamine xylazine (100 and 10 mg/kg/10ml, i.p.) anaesthesia. AMB1 mice were generated by replacement of exon 1 of mouse TrkA with that of its human counterpart and as such they exclusively express the human TrkA protein. Following hair clipping, the left sciatic nerve was exposed at mid-thigh level, aseptically. Three loose ligature (using 4-0 black silk) were place around the sciatic nerve at 1-2 mm before the branching of nerve. The surgical site was treated with betadine solution following skin suture. Animals were allowed to recover for the next seven days. One day before the experiment, animals were acclimatized to the measuring device (cold plate) and the pre-dose paw withdrawal thresholds were recorded (0hr). Animals were then regrouped into six groups. The following day, animals were treated with a single i.p. injection of isotype control antibody (1000 µg/kg/10ml) or different doses of the antibody GBR VH5(K3Q,V37A)VL1 IGHG4 S228P (10, 100 & 1000 µg/kg/10ml). Pregabalin (30 mg/kg/10ml, p.o.) or saline were administered once daily over a seven day post-dose period. Post-dose readouts were recorded at 4h, 24h and then every other day until the 7th day post-dose. Post-dose readouts were recorded 1h after pregabalin or saline dosing on all days. Post-dose readouts were recorded first for mechanical hyperalgesia and 5 min later for cold allodynia. Mechanical hyperalgesia was measured as the threshold force (g) required to elicit paw withdrawal using a dynamic plantar aesthesiometer (Plantar Von Frey instrument; Ugo Basile Srl, Italy). Cold allodynia was measured as time taken for paw withdrawal from a cold plate in seconds (IITC Life Science Inc., USA).

### Results

Seven days after CCI surgery, mice exhibited marked mechanical hyperalgesia (Fig. 7A) and cold allodynia (Fig. 7B) as seen by a sharp reduction in their paw withdrawal threshold and paw withdrawal latency time, respectively. All doses of the antibody GBR VH5 (K3Q,V37A)VL1 IGHG4 S228P gave a reversal of mechanical hyperalgesia, as seen by an increase in the paw withdrawal threshold (Fig. 7A) and cold allodynia (Fig. 7B), as seen by an increase in the paw withdrawal latency. The extent and duration of this reversal was clearly dose dependent and was comparable to pregabalin at the highest dose of 1 mg/kg (1000 µg/kg) for both readouts. This dose of the antibody GBR VH5 (K3Q,V37A)VL1 IGHG4 S228P of 1 mg/kg was effective when administered as a single dose, compared to multiple doses of pregabalin of 30 mg/kg, again showing that the anti-TrkA antibody can be administered at a lower dose and lower dosing frequency compared to the standard treatment of pregabalin.

## Claims

1. A humanized anti-TrkA antibody or fragment thereof that binds to human TrkA comprising one of the following combination of heavy and light chain variable domain comprising the sequences selected from the group consisting of SEQ ID NO: 1 and SEQ ID NO: 6, SEQ ID NO: 3 and SEQ ID NO: 6, SEQ ID NO: 3 and SEQ ID NO: 8, SEQ ID NO: 5 and SEQ ID NO: 6, and SEQ ID NO: 5 and SEQ ID NO: 8 wherein the non-CDR region of the heavy chain variable domain comprise an amino acid substitution at an amino acid position selected from the group consisting of V37A or K3Q and V37A, wherein the amino acid position of each group member is indicated utilizing the numbering system set forth in Kabat.

2. The humanized anti-TrkA antibody or fragment thereof of claim 1, wherein the amino acid substitution of the non-CDR region of the heavy chain variable domain of the antibody or the fragment thereof comprises V37A or K3Q and V37A, wherein the amino acid position is indicated utilizing the numbering system set forth in Kabat.

3. A humanized anti-TrkA antibody or fragment thereof of claim 1 or 2 comprising:
a) a heavy chain variable domain comprising a sequence selected from the group consisting of SEQ ID NOs: 31-34, 36, 48-49, and
b) a light chain variable domain comprising a sequence selected from the group consisting of SEQ ID NOs: 6 and 8.

4. The humanized anti-TrkA antibody or fragment thereof of any one of claims 1 to 3, further comprising heavy and/or light constant regions and a hinge region, wherein the heavy constant region and the hinge region are of human IGHG1 isotype or are of human IGHG4 isotype, in particular heavy constant region and the hinge region are of human IGHG4 isotype wherein the hinge region comprises amino acid substitution S228P, wherein the amino acid position is indicated utilizing the EU numbering system.

5. The humanized anti-TrkA antibody or fragment thereof of any one of claim 1 to 4 comprising:
a) a heavy chain comprising a sequence selected from the group consisting of SEQ ID NOs: 50-54, 56, 57, 69 and 70 and
b) a light chain comprising a sequence selected from the group consisting of SEQ ID NOs: 29 and 30.

6. The humanized anti-TrkA antibody or fragment thereof of any one of claims 1 to 5, wherein the antibody is a full length antibody or wherein
the antibody is an antibody fragment selected from the group consisting of Fab, Fab', Fab'-SH, Fd, Fv, dAb, F(ab')₂, scFv, bispecific single chain Fv dimers, diabodies, triabodies and scFv genetically fused to the same or a different antibody.

7. The humanized anti-TrkA antibody or fragment thereof of any one of claims 1 to 6, wherein the humanized anti-TrkA antibody or fragment thereof is capable of inhibiting the functional activation of TrkA.

8. The humanized anti-TrkA antibody or fragment thereof of any one of claims 1 to 7, wherein the humanized anti-TrkA antibody or fragment thereof binds to human TrkA with an affinity (KD) of 500 nM or less.

9. The humanized anti-TrkA antibody or fragment thereof of any one of claims 1 to 8, wherein the humanized anti-TrkA antibody or fragment thereof has at least equivalent or lower IC50 in a TF-1 cell proliferation assay than the corresponding parental murine antibody.

10. The humanized anti-TrkA antibody or fragment thereof of any one of claims 1 to 9, wherein the humanized anti-TrkA antibody has a FAB fragment thermostability temperature greater than 65°C which is equivalent to the FAB fragment thermostability temperature of the parental murine antibody.

11. A composition comprising the humanized anti-TrkA antibody or fragment thereof of any one of claims 1 to 10 and a pharmaceutically acceptable carrier.

12. The composition of claim 11 further comprising another pharmaceutically active agent wherein the said another pharmaceutically active agent is one or more of:
a) an analgesic agent
b) another anti-TrkA antibody
c) NGF
d) an anti-cancer agent
e) an anti-NGF antibody.

13. The humanized anti-TrkA antibody or fragment thereof of any one of claims 1 to 10, the composition of claim 11 or 12, for use in the treatment of pain.

14. The humanized anti-TrkA antibody or fragment thereof of any one of claims 1 to 10, the composition of claim 11 or 12, for use in the treatment of pain associated with one or more of the following: inflammatory pain, post-surgical pain, post-operative pain (including dental pain), neuropathic pain, peripheral neuropathy, diabetic neuropathy, diabetic nephropathy, fracture pain, gout joint pain, post-herpetic neuralgia, cancer pain, osteoarthritis or rheumatoid arthritis pain, sciatica, pains associated with sickle cell crises, headaches (e.g., migraines, tension headache, cluster headache), dysmenorrhea, endometriosis, uterine fibroids, musculoskeletal pain, chronic low back pain, fibromyalgia, sprains, visceral pain, ovarian cysts, prostatitis, chronic pelvic pain syndrome, cystitis, interstitial cystitis, painful bladder syndrome and/or bladder pain syndrome, pain associated with chronic abacterial prostatitis, incisional pain, migraine, trigeminal neuralgia, pain from bums and/or wounds, pain associated with trauma, pain associated with musculoskeletal diseases, ankylosing spondilitis, periarticular pathologies, pain from bone metastases, pain from HIV, erythromelalgia or pain caused by pancreatitis or kidney stones, malignant melanoma, Sjogren's syndrome, asthma, (e.g., uncontrolled asthma with severe airway hyper-responsiveness), intractable cough, demyelinating diseases, chronic alcoholism, stroke, thalamic pain syndrome, pain from toxins, pain from chemotherapy, inflammatory bowel disorders, irritable bowel syndrome, inflammatory eye disorders, inflammatory or unstable bladder disorders, psoriasis, skin complaints with inflammatory components, sunburn, carditis, dermatitis, myositis, neuritis, collagen vascular diseases, chronic inflammatory conditions, inflammatory pain and associated hyperalgesia and allodynia, neuropathic pain and associated hyperalgesia or allodynia, diabetic neuropathy pain, causalgia, sympathetically maintained pain, deafferentation syndromes, epithelial tissue damage or dysfunction, disturbances of visceral motility at respiratory, genitourinary, gastrointestinal or vascular regions, allergic skin reactions, pruritis, vitiligo, general gastrointestinal disorders, colitis, gastric ulceration, duodenal ulcers, vasomotor or allergic rhinitis, bronchial disorders, dyspepsia, gastroesophageal reflux, pancreatitis, visceralgia fibrous dysplasia of bone (FD), oncological pain, HIV infection Crohn's disease and post surgical adhesions, gall bladder stones

15. An article of manufacture comprising the humanized anti-TrkA antibody or fragment thereof of any one of claims 1 to 10 or the composition of claim 11 and 12.

## Patentansprüche

1. Humanisierter anti-TrkA-Antikörper oder ein Fragment davon, das an Human-TrkA bindet, umfassend eine der nachstehenden Kombination einer variablen Domäne einer schweren und leichten Kette, umfassend die Sequenzen, ausgewählt aus der Gruppe, bestehend aus SEQ ID NR.: 1 und SEQ ID NR.: 6, SEQ ID NR.: 3 und SEQ ID NR.: 6, SEQ ID NR.: 3 und SEQ ID NR.: 8, SEQ ID NR.: 5 und SEQ ID NR.: 6 und SEQ ID NR.: 5 und SEQ ID NR.: 8,
wobei die Nicht-CDR-Region der variablen Domäne von der schweren Kette eine Aminosäure-Substitution bei einer Aminosäure-Position umfasst, ausgewählt aus der Gruppe, bestehend aus V37A oder K3Q und V37A, wobei die Aminosäure-Position von jedem Gruppenelement unter Anwenden des Nummerierungssystems nach Kabat angezeigt wird.

2. Humanisierter anti-TrkA-Antikörper oder ein Fragment davon nach Anspruch 1, wobei die Aminosäure-Substitution der Nicht-CDR-Region der variablen Domäne einer schweren Kette des Antikörpers oder des Fragments davon V37A oder K3Q und V37A umfasst, wobei die Aminosäure-Position unter Anwenden des Nummerierungssystems nach Kabat angezeigt wird.

3. Humanisierter anti-TrkA-Antikörper oder ein Fragment davon nach Anspruch 1 oder 2, umfassend:
a) eine variable Domäne einer schweren Kette, umfassend eine Sequenz, ausgewählt aus der Gruppe, bestehend aus SEQ ID NRn.: 31-34, 36, 48-49, und
b) eine variable Domäne einer leichten Kette, umfassend eine Sequenz, ausgewählt aus der Gruppe, bestehend aus SEQ ID NRn.: 6 und 8.

4. Humanisierter anti-TrkA-Antikörper oder ein Fragment davon nach einem der Ansprüche 1 bis 3, weiter umfassend schwere und/oder leichte konstante Regionen und eine Gelenkregion, wobei die schwere konstante Region und die Gelenkregion vom Human-IGHG1-Isotyp sind oder vom Human-IGHG4-Isotyp sind, insbesondere schwere konstante Region und die Gelenkregion vom Human-IGHG4-Isotyp sind, wobei die Gelenkregion Aminosäure-Substitution S228P umfasst, wobei die Aminosäure-Position unter Anwenden des EU-Nummerierungssystems angezeigt wird.

5. Humanisierter anti-TrkA-Antikörper oder ein Fragment davon nach einem von Anspruch 1 bis 4, umfassend:
a) eine schwere Kette, umfassend eine Sequenz, ausgewählt aus der Gruppe, bestehend aus SEQ ID NRn.: 50-54, 56, 57, 69 und 70 und
b) eine leichte Kette, umfassend eine Sequenz, ausgewählt aus der Gruppe, bestehend aus SEQ ID NRn.: 29 und 30.

6. Humanisierter anti-TrkA-Antikörper oder ein Fragment davon nach einem der Ansprüche 1 bis 5, wobei der Antikörper ein Volllängen-Antikörper ist oder wobei der Antikörper ein Antikörper-Fragment ist, ausgewählt aus der Gruppe, bestehend aus Fab, Fab', Fab'-SH, Fd, Fv, dAb, F(ab')₂, scFv, bispezifischen Einzelketten-Fv-Dimeren, Diakörpern, Triakörpern und scFv, genetisch fusioniert an den gleichen oder einen anderen Antikörper.

7. Humanisierter anti-TrkA-Antikörper oder ein Fragment davon nach einem der Ansprüche 1 bis 6, wobei der humanisierte anti-TrkA-Antikörper oder ein Fragment davon in der Lage ist, die funktionelle Aktivierung von TrkA zu hemmen.

8. Humanisierter anti-TrkA-Antikörper oder ein Fragment davon nach einem der Ansprüche 1 bis 7, wobei der humanisierte anti-TrkA-Antikörper oder ein Fragment davon an Human-TrkA mit einer Affinität (KD) von 500 nM oder weniger bindet.

9. Humanisierter anti-TrkA-Antikörper oder ein Fragment davon nach einem der Ansprüche 1 bis 8, wobei der humanisierte anti-TrkA-Antikörper oder ein Fragment davon einen IC50 in einem TF-1-Zellen-Proliferationsassay mindestens äquivalent oder geringer als der entsprechende parentale murine Antikörper aufweist.

10. Humanisierter anti-TrkA-Antikörper oder ein Fragment davon nach einem der Ansprüche 1 bis 9, wobei der humanisierte anti-TrkA-Antikörper eine FAB-Fragment-Thermostabilitätstemperatur größer als 65 °C aufweist, die äquivalent zu der FAB-Fragment-Thermostabilitätstemperatur des parentalen murinen Antikörpers ist.

11. Zusammensetzung, umfassend humanisierten anti-TrkA-Antikörper oder ein Fragment davon nach einem der Ansprüche 1 bis 10 und einen pharmazeutisch verträglichen Träger.

12. Zusammensetzung nach Anspruch 11, weiter umfassend einen weiteren pharmazeutischen Wirkstoff, wobei der weitere pharmazeutische Wirkstoff einer oder mehrere ist von:
a) einem analgetischen Mittel
b) einem weiteren anti-TrkA-Antikörper
c) NGF
d) einem Anti-Krebsmittel
e) einem anti-NGF-Antikörper.

13. Humanisierter anti-TrkA-Antikörper oder ein Fragment davon nach einem der Ansprüche 1 bis 10, Zusammensetzung nach Anspruch 11 oder 12, zur Verwendung bei der Behandlung von Schmerz.

14. Humanisierter anti-TrkA-Antikörper oder ein Fragment davon nach einem der Ansprüche 1 bis 10, Zusammensetzung nach Anspruch 11 oder 12, zur Verwendung bei der Behandlung von Schmerz, verbunden mit einem oder mehreren der nachstehenden: Entzündungsschmerz, post-chirurgischem Schmerz, post-operativem Schmerz (einschließlich dentalen Schmerzes), neuropathischem Schmerz, peripherer Neuropathie, diabetischer Neuropathie, diabetischer Nephropathie, Frakturschmerz, Gichtgelenksschmerz, postherpetischer Neuralgie, Krebsschmerz, Osteoarthritis- oder rheumatischem Arthritisschmerz, Ischias, Schmerzen, verbunden mit Sichelzellkrise, Kopfschmerzen (z.B. Migränen, Spannungskopfschmerz, Clusterkopfschmerz), Dysmenorrhö, Endometriose, Gebärmutterfibromen, Schmerz des Bewegungsapparats, chronischer Lumbalgie, Fibromyalgie, Verstauchungen, viszeralem Schmerz, Eierstockzysten, Prostataentzündung, chronischem Beckenentzündungsschmerzsyndrom, Zystitis, interstitieller Zystitis, schmerzvollem Blasensyndrom und/oder Blasenschmerzsyndrom, Schmerz, verbunden mit chronischer abakterieller Prostataentzündung, Narbenschmerz, Migräne, Trigeminusneuralgie, Schmerz von Verbrennungen und/oder Wunden, Schmerz, verbunden mit Trauma, Schmerz, verbunden mit Erkrankungen des Bewegungsapparats, Morbus Bechterew, periartikulären Pathologien, Schmerz von Knochenmetastasen, Schmerz von HIV, Erythromelalgie oder Schmerz, verursacht durch Pankreatitis oder Nierensteine, malignen Melanomen, Sjögren-Syndrom, Asthma, (z.B. unkontrolliertes Asthma mit schwerer Luftwegshyperreaktion), hartnäckigem Husten, demyelinisierenden Krankheiten, chronischem Alkoholismus, Schlaganfall, thalamischem Schmerzsyndrom, Schmerz von Toxinen, Schmerz von Chemotherapie, entzündlichen Darmstörungen, reizbarem Darmsyndrom, entzündlichen Augenstörungen, entzündlichen oder instabilen Blasenstörungen, Psoriasis, Hautbeschwerden mit entzündlichen Komponenten, Sonnenbrand, Karditis, Dermatitis, Myositis, Neuritis, kollagen-vaskulären Erkrankungen, chronischen entzündlichen Zuständen, Entzündungsschmerz und zugehöriger Hyperalgesie und Allodynie, neuropathischem Schmerz und verbundener Hyperalgesie oder Allodynie, diabetischem Neuropathieschmerz, Kausalgie, sympathetisch unterhaltenem Schmerz, Deafferentationssyndromen, epithelialer Gewebeschädigung oder Dysfunktion, Störungen der viszeralen Motilität beim Atmen, urogenitaler, gastrointestinaler oder vaskulärer Regionen, allergischen Hautreaktionen, Pruritus, Vitiligo, allgemeinen gastrointestinalen Störungen, Colitis, gastrischer Ulzeration, duodenalen Geschwüren, vasomotorischem oder allergischem Schnupfen, bronchialen Störungen, Dyspepsie, gastroösophagealem Reflux, Pankreatitis, Viszeralgie-fibröser Dysplasie des Knochens (FD), onkologischem Schmerz, HIV-Infektion Crohnscher Krankheit und post-chirurgischen Verwachsungen, Gallenblasensteinen.

15. Herstellungsgegenstand, umfassend den humanisierten anti-TrkA-Antikörper oder ein Fragment davon nach einem der Ansprüche 1 bis 10 oder die Zusammensetzung nach Anspruch 11 und 12.

## Revendications

1. Anticorps anti-TrkA humanisé ou fragment de celui-ci qui se lie au TrkA humain comprenant une des combinaisons suivantes de domaine variable de chaîne lourde et légère comprenant les séquences sélectionnées dans le groupe constitué de SEQ ID NO : 1 et de SEQ ID NO : 6, de SEQ ID NO : 3 et de SEQ ID NO : 6, de SEQ ID NO : 3 et de SEQ ID NO : 8, de SEQ ID NO : 5 et de SEQ ID NO : 6, et de SEQ ID NO : 5 et de SEQ ID NO : 8
dans lequel la région non-CDR du domaine variable de chaîne lourde comprend une substitution d'acide aminé à une position des acides aminés sélectionnée dans le groupe constitué de V37A ou K3Q et V37A,
dans lequel la position des acides aminés de chaque membre du groupe est indiquée en utilisant le système de numérotation de Kabat.

2. Anticorps anti-TrkA humanisé ou fragment de celui-ci selon la revendication 1, dans lequel la substitution d'acides aminés de la région non-CDR du domaine variable de chaîne lourde de l'anticorps ou du fragment de celui-ci comprend V37A ou K3Q et V37A, dans lequel la position des acides aminés est indiquée en utilisant le système de numérotation de Kabat.

3. Anticorps anti-TrkA humanisé ou fragment de celui-ci selon la revendication 1 ou 2, comprenant :
a) un domaine variable de chaîne lourde comprenant une séquence sélectionnée dans le groupe constitué de SEQ ID NO : 31 à 34, 36, 48 à 49, et
b) un domaine variable de chaîne légère comprenant une séquence sélectionnée dans le groupe constitué de SEQ ID NO : 6 et 8.

4. Anticorps anti-TrkA humanisé ou fragment de celui-ci selon l'une quelconque des revendications 1 à 3, comprenant en outre des régions constantes lourdes et/ou légères et une région charnière, dans lequel la région constante lourde et la région charnière sont d'un isotype IGHG1 humain ou sont d'un isotype IGHG4 humain, en particulier une région constante lourde et la région charnière sont d'un isotype IGHG4 humain dans lequel la région charnière comprend une substitution d'acide aminé S228P, dans lequel la position des acides aminés est indiquée en utilisant le système de numérotation EU.

5. Anticorps anti-TrkA humanisé ou fragment de celui-ci selon l'une quelconque des revendications 1 à 4 comprenant :
a) une chaîne lourde comprenant une séquence sélectionnée dans le groupe constitué de SEQ ID NO : 50 à 54, 56, 57, 69 et 70 et
b) une chaîne légère comprenant une séquence sélectionnée dans le groupe constitué de SEQ ID NO : 29 et 30.

6. Anticorps anti-TrkA humanisé ou fragment de celui-ci selon l'une quelconque des revendications 1 à 5, dans lequel l'anticorps est un anticorps complet ou dans lequel l'anticorps est un fragment d'anticorps sélectionné dans le groupe constitué de Fab, de Fab', de Fab'-SH, de Fd, de Fv, de dAb, de F(ab')₂, de scFv, de dimères Fv à chaîne unique bispécifiques, de dimères d'anticorps, de trimères d'anticorps et de scFv génétiquement fusionnés au même anticorps ou à un anticorps différent.

7. Anticorps anti-TrkA humanisé ou fragment de celui-ci selon l'une quelconque des revendications 1 à 6, dans lequel l'anticorps anti-TrkA humanisé ou un fragment de celui-ci est capable d'inhiber l'activation fonctionnelle de TrkA.

8. Anticorps anti-TrkA humanisé ou fragment de celui-ci selon l'une quelconque des revendications 1 à 7, dans lequel l'anticorps anti-TrkA ou un fragment de celui-ci se lie au TrkA humain avec une affinité (KD) de 500 nM ou moins.

9. Anticorps anti-TrkA humanisé ou fragment de celui-ci selon l'une quelconque des revendications 1 à 8, dans lequel l'anticorps anti-TrkA humanisé ou un fragment de celui-ci a au moins un IC50 équivalent ou inférieur dans un essai de prolifération de cellules TF-1 à celui de l'anticorps murin parental correspondant.

10. Anticorps anti-TrkA humanisé ou fragment de celui-ci selon l'une quelconque des revendications 1 à 9, dans lequel l'anticorps anti-TrkA humanisé a une température de thermostabilité du fragment FAB supérieure à 65 °C qui est équivalente à la température de thermostabilité du fragment FAB de l'anticorps murin parental.

11. Composition comprenant l'anticorps anti-TrkA humanisé ou un fragment de celui-ci selon l'une quelconque des revendications 1 à 10 et un véhicule pharmaceutiquement acceptable.

12. Composition selon la revendication 11 comprenant en outre un autre agent pharmaceutiquement actif dans laquelle ledit autre agent pharmaceutiquement actif est un ou plusieurs de :
a) un agent analgésique
b) un autre anticorps anti-TrkA
c) NGF
d) un agent anti-cancéreux
e) un anticorps anti-NGF.

13. Anticorps anti-TrkA ou fragment de celui-ci selon l'une quelconque des revendications 1 à 10, la composition selon la revendication 11 ou 12, pour une utilisation dans le traitement de la douleur.

14. Anticorps anti-TrkA humanisé ou fragment de celui-ci selon l'une quelconque des revendications 1 à 10, la composition selon la revendication 11 ou 12, pour une utilisation dans le traitement de la douleur associée avec une ou plusieurs des suivantes : douleur inflammatoire, douleur post-chirurgicale, douleur postopératoire (incluant la douleur dentaire), douleur neuropathique, neuropathie périphérique, neuropathie diabétique, néphropathie diabétique, douleur de fracture, douleur articulaire de la goutte, névralgie post-herpétique, douleur cancéreuse, douleur d'ostéoarthrite ou d'arthrite rhumatoïde, sciatique, douleurs associées à des crises de drépanocytose, maux de tête (par exemple, migraines, céphalée de tension, céphalée en grappes), dysménorrhée, endométriose, fibromes utérins, douleur musculo-squelettique, douleur chronique du bas du dos, fibromyalgie, entorses, douleur viscérale, kystes ovariens, prostatite, syndrome de la douleur pelvienne chronique, cystite, cystite interstitielle, syndrome de la vessie douloureuse et/ou syndrome de douleurs de la vessie, douleur associée à la prostatite chronique non-bactérienne, douleur incisionnelle, migraine, névralgie trigéminale, douleur de brûlures et/ou blessures, douleur associée à un traumatisme, douleur associée à des maladies musculo-squelettiques, spondylite ankylosante, pathologies périarticulaires, douleur liée aux métastases osseuses, douleur liée au VIH, érythromélalgie ou douleur causée par une pancréatite ou par des calculs rénaux, mélanome malin, syndrome de Sjogren, asthme, (par exemple, asthme non maîtrisé avec hyperréactivité des voies aériennes sévère), toux intraitable, maladies démyélinisantes, alcoolisme chronique, syndrome de la douleur thalamique, douleur liée aux toxines, douleur liée à la chimiothérapie, troubles inflammatoires de l'intestin, syndrome du côlon irritable, troubles inflammatoires de l'oeil, troubles inflammatoires ou instables de la vessie, psoriasis, affections de la peau avec des composants inflammatoires, coups de soleil, cardite, dermatite, myosite, névrite, maladies vasculaires du collagène, maladies inflammatoires chroniques, douleur inflammatoire et hyperalgésie ou allodynie associées, douleur neuropathique et hyperalgésie ou allodynie associées, douleur neuropathique diabétique, causalgie, douleurs transmises de manière persistante par le système sympathique, syndromes de désafférentation, lésion ou dysfonction du tissu épithélial, troubles de la motilité viscérale aux régions respiratoire, génito-urinaire, gastrointestinale ou vasculaire, réactions allergiques de la peau, prurit, vitiligo, troubles gastro-intestinaux généraux, colite, ulcération gastrique, ulcération duodénale, rhinite vasomotrice ou allergique, troubles bronchiques, dyspepsie, reflux gastrooesophagien, pancréatite, viscéralgie dysplasie fibreuse des os (DF), douleur oncologique, infection par le VIH, la maladie de Crohn et adhérences post-chirurgicales, calculs de la vésicule biliaire

15. Article manufacturé comprenant l'anticorps anti-TrkA humanisé ou un fragment de celui-ci selon l'une quelconque des revendications 1 à 10 ou la composition selon la revendication 11 ou 12.
